# EUROPEAN PATENT APPLICATION

(11) **EP 3 831 939 A1**
(43) Date of publication of application: **09.06.2021**
(21) Application number: 19214152.1
(22) Date of filing: 06.12.2019
(51) Int. Cl.: C12N 9/12, C12N 9/22, C12N 15/10

(54) **FUSION OF SITE-SPECIFIC RECOMBINASES FOR EFFICIENT AND SPECIFIC GENOME EDITING**

(71) Applicant: Technische Universität Dresden, 01069 Dresden (DE)
(72) Inventor: BUCHHOLZ, Frank, 01307 Dresden (DE); LANSING, Felix, 01099 Dresden (DE); KARPINSKI, Janet, 01279 Dresden (DE); ROJO ROMANOS, Teresa, 01097 Dresden (DE); SONNTAG, Jan, 01307 Dresden (DE); MUKHAMETZYANOVA, Liliya, 01099 Dresden (DE)
(74) Representative: Maikowski & Ninnemann Patentanwälte Partnerschaft mbB

(57) **Abstract**

The invention relates generally to the field of genome editing and provides DNA recombinases, which efficiently and specifically recombine genomic target sequences via the fusion of recombinase monomers. More specifically, the invention provides a fusion protein for efficient and specific genome editing, comprising a complex of recombinases comprising at least a first recombinase enzyme, a second recombinase enzyme and at least one linker, wherein said first recombinase enzyme and said second recombinase enzyme specifically recognize a first half-site and a second half-site of an upstream target site or a downstream target target site of a recombinase; wherein said first recombinase enzyme and said second recombinase enzyme are interconnected via a linker; and wherein said linker comprises or consists of an oligopeptide comprising 4 to 50 amino acids. The invention also discloses designer-recombinases, which catalyze the inversion of a DNA sequence present in the intlh regions on the human X chromosome. The invention further relates to nucleic acid molecules encoding said DNA recombinases and fusion proteins, as well as a pharmaceutical composition comprising said fusion proteins, DNA recombinases and nucleic acid molecules.

## Description

### Field of the invention

The invention relates generally to the field of genome editing and provides DNA recombinases, which efficiently and specifically recombine genomic target sequences via the fusion of recombinase monomers. More specifically, the invention provides a fusion protein for efficient and specific genome editing, comprising a complex of recombinases comprising at least a first recombinase enzyme, a second recombinase enzyme and at least one linker, wherein said first recombinase enzyme and said second recombinase enzyme specifically recognize a first half-site and a second half-site of an upstream target site and/or a downstream target site of a recombinase; wherein said first recombinase enzyme and said second recombinase enzyme are interconnected via a linker; and wherein said linker comprises or consists of an oligopeptide comprising 4 to 50 amino acids. The invention also discloses designer-recombinases, which catalyze the inversion of a DNA sequence present in the intlh regions on the human X chromosome. The invention further relates to nucleic acid molecules encoding said DNA recombinases and fusion proteins, as well as to the use of said fusion proteins, DNA recombinases and nucleic acid molecules in a pharmaceutical composition.

### Background of the invention

Genome engineering is becoming an increasingly important technology in biomedical research. The main approach in the field of gene editing nowadays is the nuclease-mediated introduction of double-strand breaks (DSB) at the locus of interest that are subsequently corrected by the cellular repair pathways. There are four types of programmable nucleases that can be divided in two groups based on their mode of target DNA sequence recognition. Meganucleases, zing finger nucleases (ZFNs) and transcription activator-like nucleases (TALENs) guide the nuclease to the specific locus using protein-DNA interactions, while clustered, regularly interspaced, short-palindromic repeat-associated (CRISPR) endonucleases direct it using RNA-DNA interactions (Caroll, 2014; Wang *et al.,* 2017). Programmable nucleases are candidates for therapeutic application, and several of them are already in clinical trials (Tebas *et al.,* 2014; Qasim *et al.,* 2015; Cyranoski, 2016).

Yet, one of the main challenges of programmable nucleases is the risk of unpredictable sequence rearrangements. The introduced DSBs are repaired by the cells primarily using non-homologous end-joining (NHEJ) or homology-directed repair (HDR). The repair by HDR is precise and maintains genomic stability, as the sequence is copied from the second allele or a donor sequence that matches the target. However, HDR is mainly active during DNA replication, and in most cells NHEJ events outnumber HDR. NHEJ is an error-prone repair mechanism, which leads to insertions and deletions (indels) in the repaired DNA fragment. This may result in adverse events due to alteration of gene sequence. (Caroll, 2014; Cox *et al.,* 2015; Kosicki *et al.,* 2018)

Alternative tools that are widely used for genome engineering include site-specific recombinases (SSRs) from the tyrosine recombinase family. Tyrosine SSRs have considerable advantages over programmable nucleases, as they are not dependent on the cellular DNA repair pathways because they perform the full recombination reaction without any accessory factors (Meinke *et al.,* 2016). This leads to highly specific, predictable and precise genome editing events, which makes them attractive for therapeutic applications.

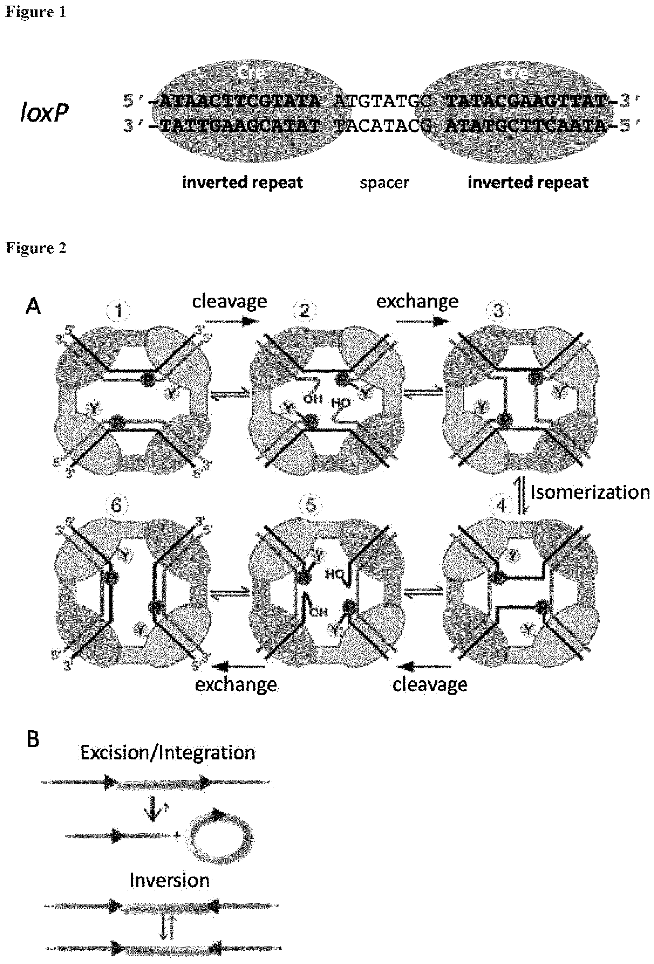

One of the most commonly used SSRs is Cre - a tyrosine recombinase from the *E. coli* bacteriophage P1 that recognizes 34 bp symmetric *loxP* sites, consisting of two 13 bp palindromic sequences flanking an 8 bp spacer (Figure 1).

The recombination reaction carried out by SSRs is a multistep process. First, four single recombinase molecules bind to each palindromic half-site of *loxP,* forming an active recombination synapsis (Figure 2A). At this stage, one of the recombinases of each of the dimers is activated, and performs the cleavage of the DNA. The cleavage occurs due to nucleophilic attack of tyrosine at position 324 of Cre to the scissile phosphate in the spacer of the *loxP* site. This leads to the formation of a 3'-phospho-tyrosine intermediate and release of a 5'-hydroxyl group. This step is followed by the strand exchange caused by the attack of the other 3'-phospho-tyrosine linkage by the released 5'-hydroxyl group, and the formation of a Holiday Junction intermediate. Next, the Holiday Junction is isomerized, and the structure of the molecules change, in that the active pair of Cre molecules become inactive and the previously inactive Cre molecules become activated. The same process of cleavage and exchange is then repeated with the second set of strands (Meinke *et al.,* 2016).

The result of recombination depends on the orientation of the spacer. If the spacer sequences of two *loxP* sites are oriented in the same direction, the recombination leads to excision or integration of the DNA fragment (Figure 2B). If the spacers are in the inverted orientation, the recombination results in inversion of the flanked DNA sequence (Meinke *et al.,* 2016).

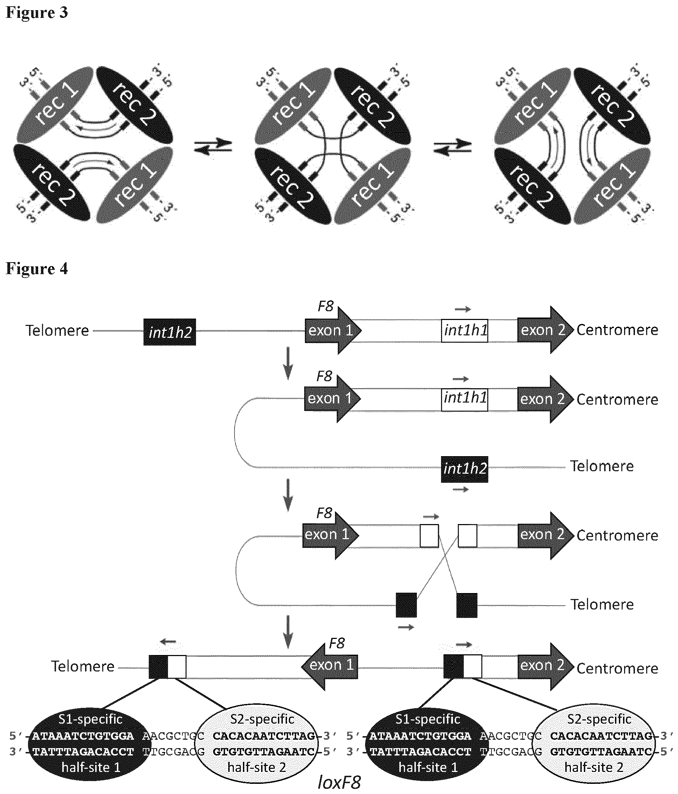

In order to use the Cre recombinase system, the *loxP* sites are typically introduced via genome engineering at the desired genomic locus. While this approach has been successfully used for conditional mutagenesis in animal models for investigation of gene function and modeling of genetic events underlying human diseases (Albanese *et al.,* 2002; Justice *et al.,* 2011), it greatly limits its application in humans. To overcome this restriction, techniques to alter the recombinase enzyme to make it recombine an artificial DNA sequence have been developed, for example, to excise the HIV provirus from infected cells (Buchholz and Stewart, 2001; Santoro and Schultz, 2002; Sarkar *et al.,*2007; Karpinski *et al.,* 2016). The palindromic nature of the target site considerably confines the number of possible target sites in genomes that can be recombined. Nevertheless, previous work has shown that single SSRs can be generated that specifically recombine asymmetric target sites (Sarkar *et al.,*2007; Karpinski *et al.,* 2016). Another possibility to recombine asymmetric target sites is to build heterospecific recombinase systems, where two different recombinase monomers bind to the respective half-site and both enzymes cooperatively recombine the final asymmetric target site (Figure 3) (Saraf-Levy *et al.,* 2006). That such systems can indeed be developed for advanced genome editing in human cells has recently been demonstrated (Lansing *et al.,* 2019). Therefore, these systems could significantly expand the utility of SSR systems.

However, the single recombinases of the heterodimer could also form homodimers with activity on symmetric sites. Therefore, using two recombinases instead of one bears the potential risk of having more off-target events since each recombinase of a heterodimer has its own symmetric off-target sites in addition to the putative asymmetric heterodimer off-targets. In order to use a heterodimer for therapeutic application, high efficacy and specificity of the system should be ensured. One of the options would be modification of the heterodimer that prevents the individual recombinases from performing recombination as homodimers. In this scenario, the activity on the symmetric sites could potentially be eliminated or restricted. Therefore, it is of high interest to generate systems that make recombinase heterodimers obligate.

Consequently, several approaches have been developed to increase Cre specificity, including the generation of obligate heterodimers with "hydrophobic size switch" (Gelato *et al.,* 2008), an obligate heterotetrameric complex with selective destabilization (Zhang *et al.,* 2015) or destabilizing binding cooperativity (Eroshenko and Church, 2013). However, these alterations all lead to marked reduction in recombination activity that are likely to be incompatible with efficient genome alterations for therapeutic applications.

One of the human diseases that can possibly be corrected by SSRs is Hemophilia A (HA). HA is an X-linked monogenetic disease caused by defects in the F8 gene that encodes the blood clotting factor VIII (FVIII). The F8 gene is located on the long arm of the X chromosome (Xq28). The gene spans 186 kb, and consists of 27 exons.

FVIII is mainly synthesized in Liver Sinusoidal Endothelial Cells (LSECs) and other human endothelial cells (Shahani *et al.,* 2014; Turner and Moake, 2015). FVIII is an important component of the blood coagulation cascade. FVIII circulates in the blood bound to von Willebrand factor, and dissociates from it after the activation by thrombin. Activated FVIII (FVIIIa) binds FIXa and this complex subsequently activates FX, leading to a chain of reactions forming a blood clot (Dahlback, 2000).

HA affects 1 in about 5,000 males (Graw *et al.,* 2005). Clinical severity of HA depends on the residual activity level of the factor VIII in the blood and is classified into three groups: mild (5-40% of normal level), moderate (1-5%), severe (<1%) (White *et al.,* 2001). Patients with mild HA experience bleeding episodes only in cases of significant traumas or surgeries. Moderately affected individuals have spontaneous bleedings after trivial trauma. Severe HA is characterized by frequent spontaneous bleedings into internal organs, muscles and joints. Repeated hemarthroses often lead to the disabling condition called hemophilic arthropathy. This complication is characterized by chronic pain, joint impairment and dramatic decrease of patients' lives quality (Pandey and Mittal, 2001; Fischer *et al.,* 2005; Melchiorre *et al.,* 2017).

More than half of the HA patients have the severe form of the disease, and in most of the cases it is caused by a genomic inversion due to intrachromosomal recombination with homologous regions outside of the F8 gene (Graw *et al.,* 2005).

The second most common genetic alteration in patients with severe HA (∼1-5%) is the inversion of exon 1. It happens due to homologous recombination between two 1 kb sequences (intlh), in intron 1 and in the region positioned telomeric to the F8 gene (Figure 4). The sequences are located in opposite orientation and in a distance of approximately 140 kb. Homologous recombination leads to the inversion and translocation of exon 1, thus, resulting in the disruption and dysfunction of the F8 gene (Castaldo *et al.,* 2007; Tizzano *et al.,* 2003).

The standard treatment for severe HA is a substitution therapy - administration of plasma-derived or recombinant concentrate of blood coagulation factor VIII. However, due to their short half-lives (8-12 hours), very frequent administration (two to three times weekly or even every other day) is necessary. This procedure has a great impact on patients' quality of life (Peyvandi *et al.,* 2016). Several modifications on the protein have been developed to increase the half-lives of the recombinant FVIII, such as PEGylation and fusion with the fragment of an IgG, that allowed to obtain the therapy with comparable efficacy but with the half-life 1.5-fold longer than that of the standard FVIII infusion (Mahlangu *et al.,* 2014; Konkle *et al.,* 2015; Peyvandi *et al.,* 2016). The extension of the half-lives leads to a small reduction of administration frequency resulting from two injections per week to one injection every 5 days (Carcao, 2014).

In addition to the frequent intravenous injections, a great challenge of the current treatment is that some of the patients (around 30%) develop inhibitory antibodies against the administrated FVIII, thereby compromising the treatment (Gouw *et al.,* 2013). Moreover, the economic burden of HA should be taken into consideration. The average annual direct cost of the standard treatment is around EUR 200,000 in Europe, while in Germany it is more than EUR 300,000 per patient (O'Hara *et al.,* 2017). Thus, only the prophylaxis of HA patients without complications is extraordinary expensive. Therefore, it is important to develop other treatment options or even a curative therapy.

Alternative therapies that inhibit the anticoagulant pathways in order to achieve hemostasis were developed and some of them, such as siRNA degrading antithrombin III mRNA and monoclonal antibodies inhibiting Tissue Factor Pathway Inhibitor or Protein C, are currently in stage I-III of clinical trials. The advantage of these approaches is the possibility of being used by all the patients, with and without FVIII inhibitors, as well as the lower treatment frequency (once per week) and subcutaneous mode of administration. However, clinical studies show that the dose of the treatment has to be carefully adjusted, since overdosing increases the risk of thrombotic effects. (Peters and Harris, 2018)

Another approach is to use the FVIIIa mimetic bispecific antibody Emicizumab. This antibody bridges FIXa to FX. Therefore, it basically mimics the function of FVIII. However, this approach lacks the regulation by the thrombin cleavage. Thus, it cannot be inactivated and relies on other steps of the coagulation cascade, also having a high risk of thrombotic effects (Lenting *et al.,* 2017).

The monogenic nature of the disease makes HA an attractive target for gene therapy. Four vectors based on the Adeno-Associated Virus (AAV) serotype that transduce hepatocytes are currently in clinical trials. This treatment has shown improvements for some patients. However, the safety of the approach still has to be investigated, since one of the main side effects is elevation of liver enzymes (Pasi *et al.,* 2017; George *et al.,* 2017; Peters and Harris, 2018). Furthermore, the non-integrative nature of AAV limits the application of the therapy in children, due to dilution and loss of the vector expression as a result of cell proliferation during organ growth (Vandamme *et al.,* 2017).

The therapies described above compensate the defective FVIII gene function, but do not correct the causing mutation. Having the gene inverted back to the normal orientation would allow for stable expression of FVIII under physiological conditions. Importantly, increase of the FVIII levels to 1-5% of normal would significantly improve lives of patients with severe HA, because it would greatly lower the risk of internal spontaneous bleedings and the administration of the FVIII would be needed only in case of traumas and surgeries.

The group of Jin-Soo Kim has used programmable nucleases to correct two inversions causing severe HA. They succeeded in inverting exon 1 for 0.2-0.4% using ZFNs in HEK 293 cells (Lee *et al.,* 2012) and for 1.4% using TALENs (Park *et al.,* 2014) in human iPSCs. The most efficient correction (reversion) of 6.7% for exon 1 and 3.7% for the exons from 1 to 22 in the F8 gene in HA patient-derived iPSCs was achieved using CRISPR-Cas9 technology (Park et al., 2015). While impressive, the obtained results with nucleases have limited therapeutic utility. Programmable nucleases perform the correction by the introduction of two double strand breaks in the homologous regions, resulting in either inversion or deletion of the flanked DNA fragment made by the cellular repair pathways. In addition, indels at the cutting sites are also likely to occur. Therefore, the inversion performed by ZFN, TALEN or CRISPR-Cas9 is more a stochastic than a controlled event.

To solve this shortcoming, SSRs could be used to correct genetic inversions causing diseases. SSRs are highly specific, and can carry out inversions of DNA sequences in a genomic context, without dependence on any accessory factors (Yu and Bradley, 2002) when target sites for the recombinase are present in the appropriate orientation. The objective therefore was to develop an SSR system that can correct the intlh inversion in human cells at high efficacy and specificity.

### Summary of the invention

The problem of the present invention is therefore to provide DNA recombinases, which efficiently and specifically recombine genomic target sequences. In particular, the problem has to be solved that each individual recombinase monomer has to find and recognize its binding site efficiently and specifically.

With this invention it is shown that recombinase monomers can be tethered together into a single polypeptide without loosing the ability to recombine the respective target site. This is unexpected, given the known molecular details of the recombination reaction (stepwise cleavage, Holiday structure formation and isomerization). The current dogma was that the 4 recombinase molecules necessary to carry out the recombination reaction have to be flexible monomers to be able to function as an enzymatic complex. It is shown herein that fusion of SSRs via specific linkers can be applied to designer-recombinases, evolved to recombine a sequence present in inverted repeats, causally involved in a genetic alteration of the FVIII gene causing HA in humans. Unexpectedly, fusion of two heterospecific recombinases with specific linkers prevent the molecule from recombining the symmetric target sites, making the system obligate. Furthermore, the fusion protein has high activity and shows greatly improved specificity in comparison to the non-linked enzymes. Directed molecular evolution lead to an improved linker sequence with desirable features.

The invention also discloses a target sequence in the intlh sequences (*loxF8*) implicated in the FVIII inversion, suitable for the generation of designer-recombinases.

The problem of the invention is in particular solved by providing a fusion protein for efficient and specific genome editing, comprising a complex of recombinases comprising at least a first recombinase enzyme, a second recombinase enzyme and at least one linker, wherein said first recombinase enzyme and said second recombinase enzyme specifically recognize a first half-site and a second half-site of an upstream target site and/or a downstream target site of a recombinase; wherein said first recombinase enzyme and said second recombinase enzyme are interconnected via a linker; and wherein said linker comprises or consists of an oligopeptide comprising 4 to 50 amino acids.

The invention further relates to DNA recombinases, which specifically recognize upstream and downstream target sequences of the *loxF8* recombinase target sites, and which catalyze the inversion of a gene sequence between these upstream and downstream target sequences of the *loxF8* recombinase target sites.

The invention relates further to nucleic acid molecules encoding a fusion protein or a DNA recombinase according to the invention.

In a further embodiment, the invention provides a mammalian, insect or bacterial host cell comprising said nucleic acid molecule encoding a fusion protein or DNA recombinase according to the invention.

A fusion protein or a DNA recombinase or a nucleic acid molecule according to the invention can be used as a medicament and can therefore be comprised in a pharmaceutical composition, optionally in combination with one or more therapeutically acceptable diluents or carriers.

The fusion protein or DNA recombinase or the pharmaceutical composition according to the invention are suitable for the treatment of a disease that can be cured by genomic editing, in particular the treatment of hemophilia A.

In a further embodiment, a method for determining recombination on genomic level in a host cell culture or patient, comprising a fusion protein or DNA recombinase for efficient and specific genome editing according to the invention, is provided.

### Brief description of the drawings

**Figure 1** is a scheme of Cre molecules binding its target *loxP* site. The *loxP* site consists of two inverted repeats (13 bp) flanking a non-palindromic spacer (8 bp). Two Cre molecules bind to the *loxP* site, each to one of the half-sites.
**Figure 2** represents a scheme of the tyrosine SSR recombination reaction. (**A**) Schematic of the stepwise recombination mechanism. Four recombinase molecules bind two DNA substrates, forming a tetrameric complex (1). Recombinases in the "cleaving competent" conformation are shown in light grey, the once in the "noncleaving" conformation are shown in dark grey. The nucleophilic tyrosine is indicated as a Y in a light grey circle and shown only for the active monomers. The activated nucleophilic tyrosine attacks the scissile phosphate (indicated as a P), forms a 3'-phosphotyrosine linkage and leads to release of free 5'OH (2). The released 5'OH attacks the neighboring phosphotyrosine, forming a Holiday Junction Intermediate and leading to strand exchange (3). The complex is isomerized and the active monomers change the conformation to inactive and vice versa (4). The cleavage and strand exchange steps are repeated (5,6). (**B**) Possible outcomes of the recombination reaction. Recombination reactions can lead to excision/integration or inversion of the DNA fragment, depending on the orientation of target sites (spacer sequence). The target sites are indicated as black triangles, and their directionality indicates the orientation of the target sites. (Meinke et al., 2016).
**Figure 3****:** shows a schematic model of recombination by a heterotetrameric Cre complex. Black-shaded ellipses represent wild type Cre monomers, grey-shaped ellipses represent mutated Cre monomers, each bound to different half-sites of *loxP* sites (black-shaded strands represent the original *loxP* site and the grey-shaded strand represent the half-site recognized by the mutated Cre) (Saraf-Levy et al., 2006).
**Figure 4****:** shows a schematic of a F8 gene inversion causing severe forms of HA. F8 gene or its part is depicted, exons are shown in as arrows, introns are shown in white boxes, the line represents DNA outside the F8 gene. A. Inversion of exon 1 is caused by recombination between the homologous regions in intron 1 (int1h1) and on the telomeric side outside of the F8 gene (intlh2). The crossover is represented by splitting the int1h1 and intlh2 boxes. The inverted situation is shown on the bottom with the two chosen target sequences (*loxF8,* SEQ ID NO: 17) bound by designer-recombinases (S1-specific, black and S2-specific, white). Note the inverted orientation of the spacer sequence.

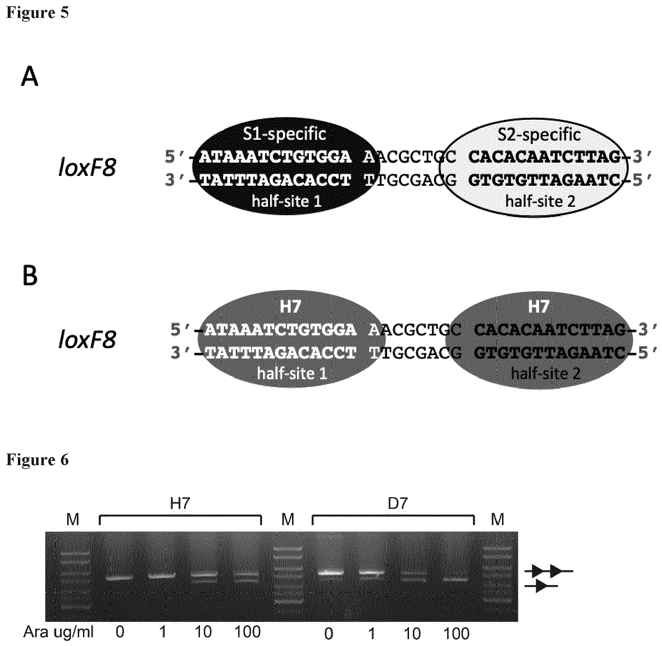

**Figure 5****:** shows a scheme of the F8 heterodimer (A) and the single monomer (B) recognizing the *loxF8* target site. The single monomer recombinase (H7, SEQ ID NO: 66, grey) recognizes both half-sites, whereas the S1-specific recombinase recognizes the left half-site 1 (SEQ ID NO: 32, black) and the S2-specific recombinase recognizes the right half-site 2 (SEQ ID NO: 33, white).
**Figure 6****:** shows the recombination activity comparison of the single F8-recombinase (H7, SEQ ID NO: 66) with the dual F8 recombinase (D7, SEQ ID NOs: 32 and 33) in *E.coli.* Digested plasmid DNA isolated from cells harboring the pEVO-loxF8 plasmid expressing the indicated recombinases at indicated concentrations of Arabinose (Ara) are shown. The line with two arrows marks the non-recombined bands. The line with one arrow marks the recombined bands. Note the favorable recombination activity of the D7 heterodimer recombinase at all tested conditions. M, marker.

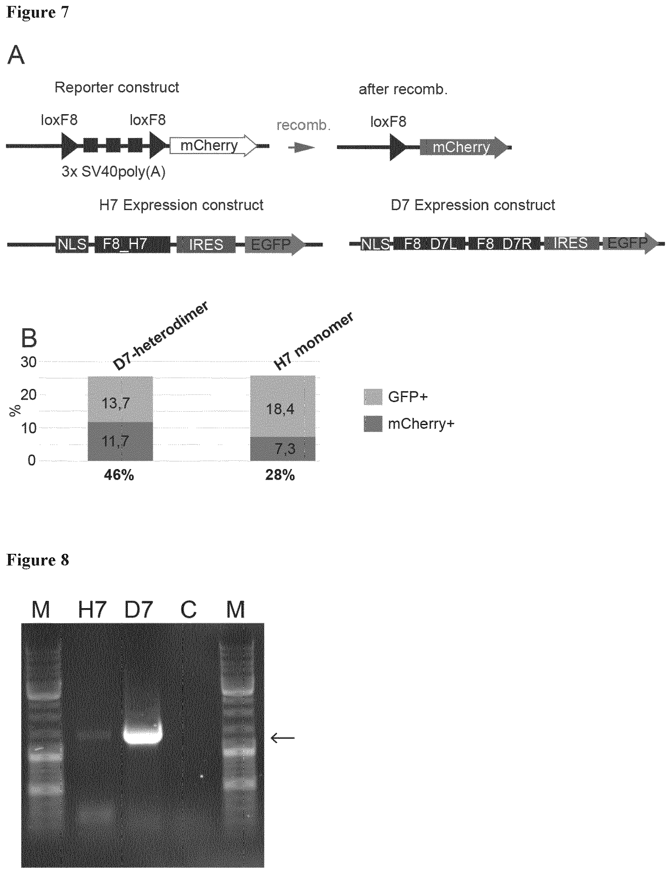

**Figure 7****:** shows the recombination activity comparison of the single F8-recombinase (H7, SEQ ID NO: 66) with the dual F8 recombinase (D7, SEQ ID NO: 32 and 33) for transient transfections in mammalian cells. (A) Illustration of the employed plasmids. (B) Results determined by FACS analyses. The overall recombination efficiencies are indicated below the bars in % where the left column shows D7 heterodimer and the right column H7 monomer.
**Figure 8****:** shows that the F8 recombinases invert the genomic F8 locus in human tissue culture cells. A PCR assay detecting the genomic inversion in genomic DNA isolated from HEK293 cells transfected with indicated recombinase expression constructs is shown. The arrow marks the specific inversion band. Note the prominent band obtained for cells transfected with the D7 heterodimer construct, indicating efficient inversion. In contrast, the H7 monomer transfected cells only display a weak band, signifying that the inversion was less efficient. C, control (non-transfected cells); M, marker.

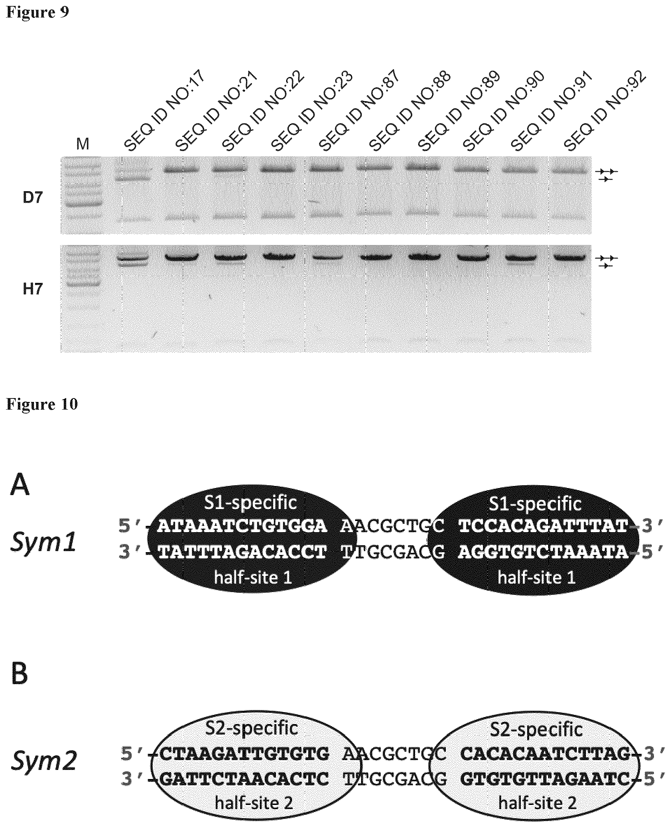

**Figure 9****:** shows the off-target analyses of F8 recombinases. Digested plasmid DNA isolated from cells harboring the pEVO-loxHS1-9 plasmids expressing the indicated recombinases (H7 monomer, D7 heterodimer). The lines with two arrows mark the non-recombined bands. The lines with one arrow mark the recombined bands. Note that the H7 monomer shows the recombination specific bands in several lanes. M, marker.
**Figure 10****:** shows the symmetric target sites deduced from the two loxF8 half-sites. (A) The sequence of the symmetric *Sym1* site is shown bound by two monomers of an S1-specific recombinase (black). (B) The sequence of the symmetric *Sym2* site is shown bound by two monomers of an S2-specific recombinase (white).

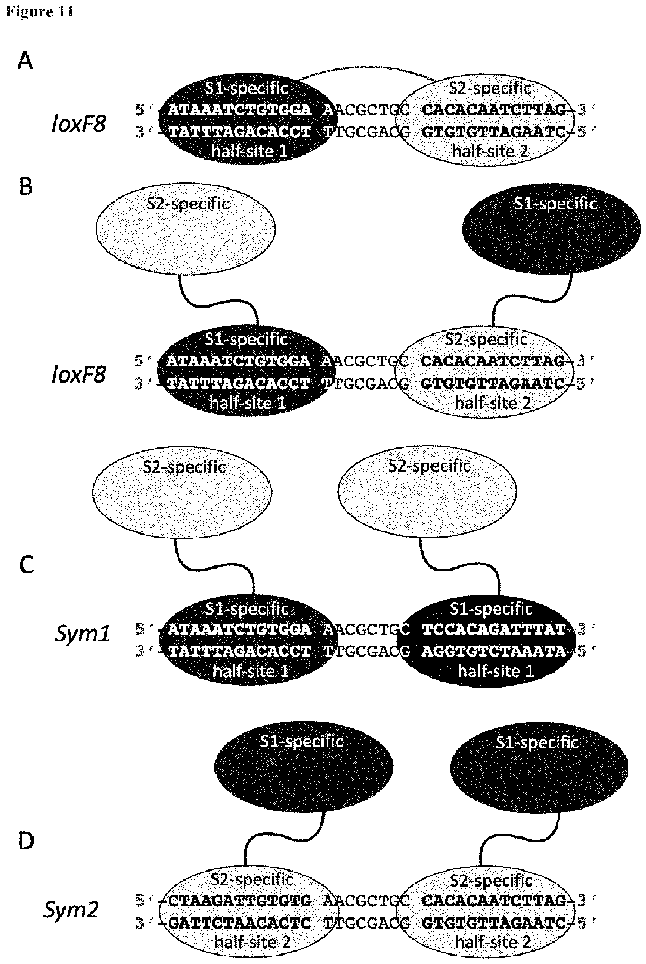

**Figure 11****:** shows possible binding scenarios of fused heterospecific recombinases to symmetric and asymmetric *loxF8* target sites.

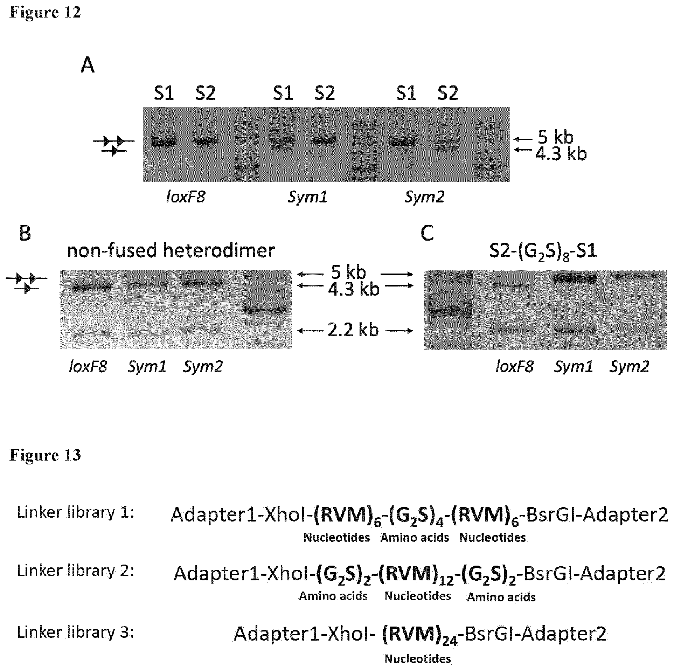

**Figure 12****:** shows the recombination efficacy of the recombinase monomers, non-linked heterodimer and S2-(G₂S)₈-S1 on the *loxF8, Sym1* and *Sym2* target sites. (A) Recombination activity of the S1-specific (S1) and S2-specific (S2) recombinase monomers. Note the absence on recombination of the individual enzymes on the asymmetric *loxF8* site. In contrast, the S 1 monomer shows activity only on the symmetric *Sym1* target site, while S2 shows activity only on the symmetric *Sym2* target site. (B) Recombination activity of the non-fused recombinase heterodimer. Note that all target sites are recombined when both recombinases are expressed together in the same cell. (C) Recombination activity of the fused recombinase heterodimer S2-(G₂S)₈-S1. Note that only the asymmetric *loxF8* site is recombined, while both, *Sym1* and *Sym2,* are not recombined by the fusion recombinase. 200 µg/ml L-arabinose was used for induction for all experiments. Recombination of the plasmid DNA was assayed by restriction enzyme digestion (SacI-HF + SbfI-HF), resulting in a smaller fragment for recombined (one triangle) and a bigger fragment for unrecombined plasmids (two triangles).
**Figure 13****:** shows a scheme of the linker libraries design. Three oligonucleotides to be used for PCR. Each of them contained two adapters from each side for primer annealing, XhoI and BsrGI-HF restriction sites, and linker sequence. Part or the whole amino acid sequence of the (G₂S)₈ sequence was changed to degenerate codons RVM. Nucleotide and amino acid sequences are indicated where appropriate.

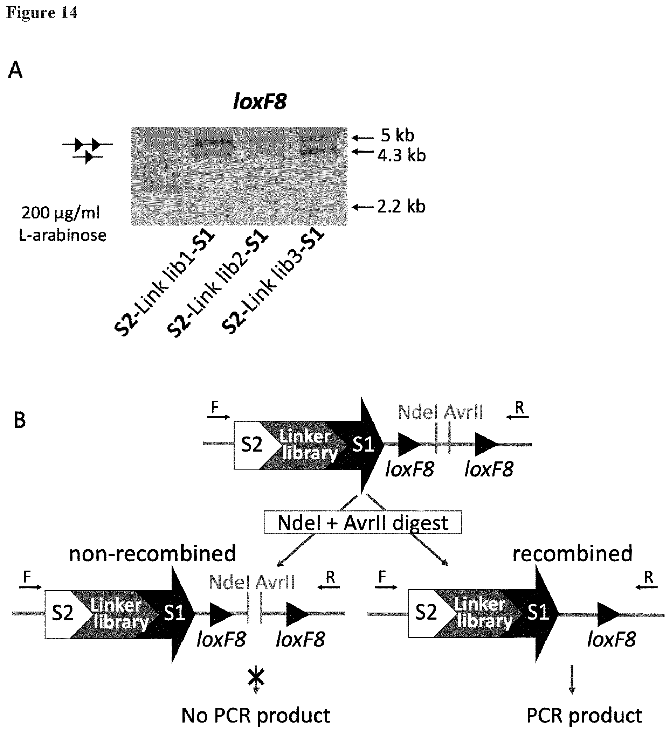

**Figure 14****:** shows the linker selection on the asymmetric *loxF8* site. (**A**) Initial recombination activity of the heterodimer fused with three different linker libraries (lib1-3) on the *loxF8* site. 200 µg/ml L-arabinose was used for induction. Recombination of the plasmid DNA was assayed by restriction enzyme digestion (SacI-HF + SbfI-HF), resulting in a smaller fragment for recombined (one triangle) and a bigger fragment for unrecombined plasmids (two triangles). (**B**) Scheme of the linker selection on the *loxF8* site. Digestion with NdeI and AvrII restriction enzymes takes place only on the non-recombined plasmids, therefore the PCR product with indicated primers (F and R) cannot be obtained from these plasmids. Only the linked heterodimers that have recombined the *loxF8* site are amplified by PCR, allowing to select only active fusion proteins.

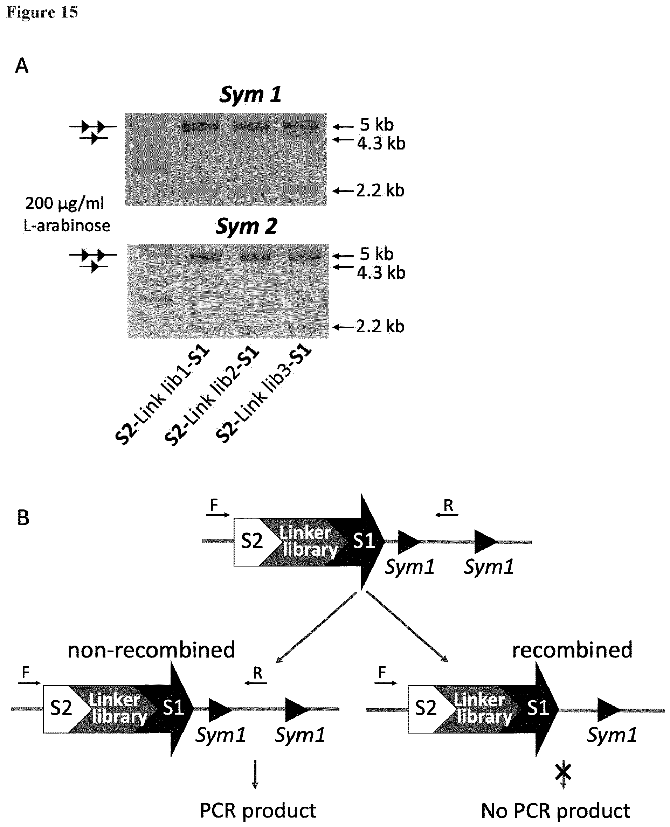

**Figure 15****:** shows the linker counter-selection on the symmetric target sites. (A) Recombination activity of the heterodimer fused with indicated linker libraries (lib1-3) on the *Sym1* and *Sym2* sites. 200 µg/ml L-arabinose was used for induction. Recombination of the plasmid DNA was assayed by restriction enzyme digestion (SacI-HF + SbfI-HF), resulting in a smaller fragment for recombined (one triangle) and a bigger fragment for unrecombined plasmids (two triangles). (**B**) Scheme of the linker counter-selection on the symmetric target sites, shown on the example of *Sym1.* The reverse primer (R) anneals between two symmetric sites, therefore the PCR product cannot be obtained from the recombined plasmids, as this DNA fragment is excised during the recombination. Only the linked heterodimers that have not recombined the symmetric site are amplified by PCR, that allows to select only fusion proteins that are not active on the symmetric sites.

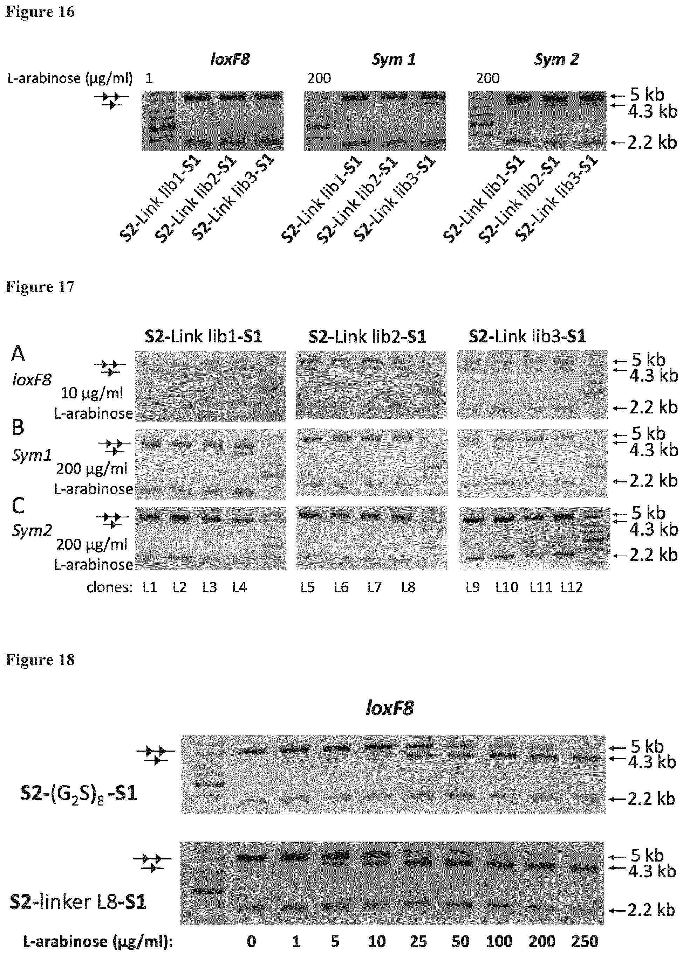

**Figure 16****:** shows the final recombination activity of the evolved libraries on the *loxF8, Sym1* and *Sym2* sites. 1 µg/ml and 200 µg/ml L-arabinose were used for induction on the asymmetric *loxF8* and symmetric *Sym1* and *Sym2* sites, respectively. Recombination of the plasmid DNA was assayed by restriction enzyme digestion (SacI-HF + SbfI-HF), resulting in a smaller fragment for recombined (one triangle) and a bigger fragment for unrecombined plasmids (two triangles).
**Figure 17** shows clonal analyses of fused D7 heterodimers on the *loxF8, Sym1,* and *Sym2* sites. Recombination activity of 4 clones from each library (L1, L2, L3, L4 from S2-link lib1-S1, L5, L6, L7, L8 from S2-link lib2-S1, L9, L10, L11, L12 from S2-link lib3-S1) was analyzed on the *loxF8* (A), *Sym1* (B), and *Sym2* (C) target sites. 10 µg/ml and 200 µg/ml L-arabinose were used for induction on the asymmetric *loxF8* and symmetric *Sym1* and *Sym2* sites, respectively. Recombination of the plasmid DNA was assayed by restriction enzyme digestion (SacI-HF + SbfI-HF), resulting in a smaller fragment for recombined (one triangle) and a bigger fragment for unrecombined plasmids (two triangles). Clone L8 was chosen for further analyses based on its high recombination activity on *loxF8* and its lack of recombination on *Sym1* and *Sym2.*
**Figure 18****:** shows the comparison of the recombination efficacy of the D7 heterodimer fused with (G₂S)₈ and L8 linkers on the *loxF8* target site. Recombination activity of the recombinase heterodimer fused with (G₂S)₈ or the L8 linker on the *loxF8* site. L-arabinose concentrations from 0 to 250 µg/ml were used for recombinase induction as indicated. Recombination of the plasmid DNA was assayed by restriction enzyme digestion (SacI-HF + SbfI-HF), resulting in a smaller fragment for recombined (one triangle) and a bigger fragment for unrecombined plasmids (two triangles). Note the increased activity of the L8 linked recombinases.

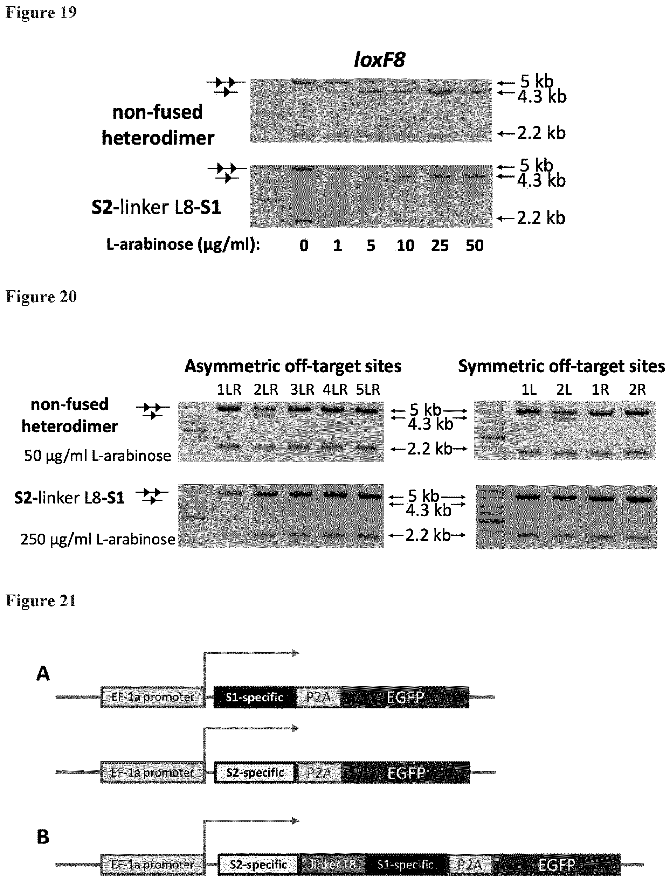

**Figure 19****:** shows the recombination activity of the non-linked D7 heterodimer and the S2-linker L8-S1 recombinases on the asymmetric loxF8 target site. Recombinases were assayed in the pEVO-SDO vector. L-arabinose concentrations from 0 to 50 µg/ml were used for recombinase induction as indicated. Recombination of the plasmid DNA was assayed by restriction enzyme digestion (SacI-HF + SbfI-HF), resulting in a smaller fragment for recombined (one triangle) and a bigger fragment for unrecombined plasmids (two triangles). Note the increased activity of the L8 linked recombinases. SDO; shine dalgano optimized.
**Figure 20****:** shows the recombination activity of the non-linked D7 heterodimer and the S2-linker L8-S 1 recombinases on predicted human genome off-target sites. Recombination activity of the non-linked D7 heterodimer and the D7 heterodimer fused with the L8 linker on asymmetric (1LR, 2LR, 3LR, 4LR, 5LR) and symmetric (1L, 2L, 1R, 2R) off-target sites are shown. 50 µg/ml and 250 µg/ml L-arabinose were used for induction of the non-linked heterodimer and S2-linker L8-S1, respectively. Recombination of the plasmid DNA was assayed by restriction enzyme digestion (SacI-HF + SbfI-HF), resulting in a smaller fragment for recombined (one triangle) and a bigger fragment for unrecombined plasmids (two triangles). Note that the L8-fused D7 heterodimer does not show marked off-target recombination, whereas off-target recombination can be observed for the non-fused version on target sites 2LR and 2L.
**Figure 21****:** shows a scheme of the recombinase expression plasmids used in human cells. (A) Illustration of the plasmids for expression of single recombinases (S1- or S2-specific). (B) Illustration of the expression plasmid for the fused D7 heterodimer (S2-linker L8-S1). For all expression plasmids EGFP expression is coupled to recombinase expression through a self-cleaving 2A peptide from porcine teschovirus-1 polyprotein (P2A). The arrows mark transcription start sites from the EF-1a promoter.

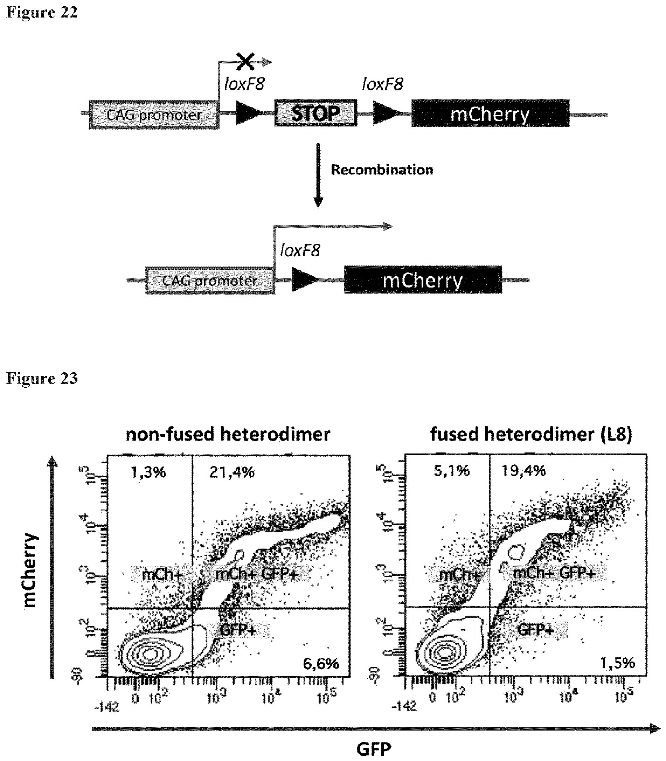

**Figure 22****:** shows a scheme of the *mCherry* gene-based reporter assay. The reporter plasmid contains stop codons between the two *loxF8* target sites that prevents the expression of *mCherry.* Upon recombination, the stop codon is removed, resulting in constitutive expression of *mCherry* conferring red color to cells. The arrows mark transcription start sites from the chicken beta-actin (CAG) promoter.
**Figure 23****:** shows flow cytometry analyses of the reporter assay in human cells. Plots of the flow cytometry analyses of HEK 293T cells transfected with the non-fused and fused heterodimer (L8) expressing plasmids and mCherry reporter plasmid are shown. Cells expressing the D7 heterodimers are identified by expression of the green fluorescent protein (GFP+). Cells that have recombined the reporter plasmid are identified by the expression of red fluorescent protein (mCh+).

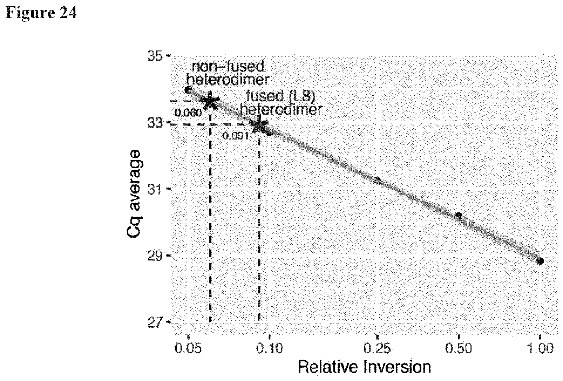

**Figure 24****:** shows the inversion efficacy performed by the heterodimers in mammalian cells. The line chart represents the results of qPCR performed on genomic DNA extracted 48 hours after transfection with D7 recombinase heterodimer expression vectors. The line presents the linear regression model of the standard curve with R²=0,9918. Relative inversion of the non-fused and fused (L8) recombinase heterodimers is shown. Note the increased inversion frequency of the fused L8 heterodimer.

### General Definitions

As used herein, the expressions "cell", "cell line," and "cell culture" are used interchangeably and all such designations include progeny. Thus, the words "transformants" and "transformed cells" include the primary subject cell and culture derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, this will be clear from the context.

The terms "polypeptide", "peptide", and "protein", as used herein, are interchangeable and are defined to mean a biomolecule composed of amino acids linked by a peptide bond.

If peptide or amino acid sequences are mentioned herein, each amino acid residue is represented by a one-letter or a three-letter designation, corresponding to the trivial name of the amino acid, in accordance with the following conventional list:

| Amino Acid | One-Letter Symbol | Three-Letter Symbol |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

The terms "a", "an" and "the" as used herein are defined to mean "one or more" and include the plural unless the context is inappropriate.

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system, animal or human being sought by a researcher, veterinarian, medical doctor or other clinician, which includes alleviation of the symptoms of the disease or disorder being treated. As used herein, the term "pharmaceutically acceptable" embraces both human and veterinary use: For example, the term "pharmaceutically acceptable" embraces a veterinarily acceptable compound or a compound acceptable in human medicine and health care.

Naturally occurring DNA recombining enzymes, in particular site-specific recombinase (SSR) systems (such as tyrosine-type SSRs), generally consist of four identical monomers. In general, they recognize two identical and symmetric, palindromic target sites, which consist each of two about 13 nucleotide long half-sites separated by an asymmetric frequently 8 nucleotide long spacer (Fig. 1). Depending on the number and relative orientation of the target sites and their spacers, the DNA recombining enzyme either performs an excision, an integration, an inversion or a replacement of genetic content (Fig. 2; reviewed in Meinke et al., 2016).

As used herein, "upstream" means the 5' target site of a recombinase in a DNA, comprising a first half-site, such as a left half-site, and a second half-site, such as a right half-site, wherein said first half-site and said second half-site are separated by a spacer sequence.

As used herein, "downstream" means the 3' target site of a recombinase in DNA, a first half-site, such as a left half-site, and a second half-site, such as a right half-site, wherein said first half-site and said second half-site are separated by a spacer sequence.

### Detailed description of the invention

The invention provides a fusion protein for efficient and specific genome editing, comprising a complex of recombinases, wherein said complex of recombinases comprises at least a first recombinase enzyme, a second recombinase enzyme and at least one linker, wherein said first recombinase enzyme and said second recombinase enzyme specifically recognize a first half-site and a second half-site of a recombinase target site; wherein said first recombinase enzyme and said second recombinase enzyme are interconnected via a linker; and wherein said linker comprises or consists of an oligopeptide comprising 4 to 50 amino acids.

The recombinases comprised in the complex are preferably DNA recombinases and may be naturally occurring recombinases (i.e. recombinases isolated from any type of biologicals samples) or designer recombinases, such as recombinases evolved by directed molecular evolution or rational design, or any combinations thereof. Methods to create designer recombinases are known in the art. WO 2018/229226 A1 for example teaches vectors and methods to generate designer DNA recombining enzymes by directed molecular evolution. WO2008083931A1 discloses the directed molecular evolution of tailored recombinases (Tre 1.0) that uses sequences in the long terminal repeat (LTR) of HIV as recognition sites (loxLTR Tre 1.0). Further developments of this approach using asymmetric target sites were described in WO2011147590 A2 (Tre 3.0) and WO2016034553 A1 (Tre 3.1 and Tre/Brec1) as well as the publication Karpinski J et al 2016 (Brec1). Methods to engineer naturally occurring or designer-recombinases by rational design are also known to the art (e.g Abi-Ghanem *et al.,* 2013; Karimova *et al.,* 2016).

Within the scope of the invention are compositions of the recombinase complex, wherein e.g.
- all four recombinase monomers are different;
- three recombinase monomers are identical and one monomer is different;
- two recombinase monomers are identical and the other two monomers are different;
- the complex comprises two different homodimers,;
- the complex? comprises two different heterodimers;
- the complex comprises two identical heterodimers;
- all four recombinase monomers are identical;
- the complex comprises two different monomers; or
- the complex comprises two identical monomers.

"Different" in this context means that the monomers are not identical in their primary structure, i.e. show differences in their amino acid sequences; and/or show a high specificity towards one of the four half-sites of an upstream target site and a downstream target site of a recombinase, which leads advantageously to a surprisingly increased specificity of the fusion proteins of the invention.

In a preferred embodiment, the recombinase complex is a dimer, more preferably a heterodimer for recognition of a first target sequence and a second target sequence of an upstream or downstream recombinase target site in a DNA, wherein the monomers of said heterodimer are fused via a linker.

In a further preferred embodiment, the recombinase complex of the invention is a tetramer, more preferably a heterotetramer for the recognition of an upstream target site and a downstream target site of a recombinase in a DNA, wherein at least two monomers of said heterotetramer are fused via a linker.

In a more preferred embodiment, the complex consists of two heterodimers, most preferably two identical heterodimers, wherein the monomers of each heterodimer are interconnected via a linker.

Further preferably, the monomers of said heterodimer have been evolved by directed evolution or rational design to specifically recognize a first half-site or second half-site of a recombinase target site. Accordingly, a first heterodimer in said complex of two heterodimers, specifically recognizes the first half-site and the second half-site of an upstream recombinase target site in a DNA, wherein the second heterodimer specifically recognizes the first half-site and the second half-site of a downstream recombinase target site.

More preferably, the monomers of said heterodimer are tyrosine site-specific recombinases.

Most preferably, a first recombinase monomer of said heterodimer has an amino acid sequence characterized by the following specific amino acids: Q in position 5, A in position 40, T in position 44, V in position 80, R in position 90, L in position 94, R at position 132, G at position 150, R in position 219, V in position 272, S in position 323 and L in position 325.

Further most preferably, a second recombinase monomer of said heterodimer has an amino acid sequence characterized by the following specific amino acids: Q in position 5, T in position 80, H in position 90, S in position 94, K in position 249, G in position 266, V in position 272 and K in position 282.

These amino acid positions refer to the numbering of the sequence of wild type recombinase Cre of SEQ ID NO: 68.

For the avoidance of doubt, when the recombinase complex is represented by a dimer, particularly a homodimer or preferably a heterodimer, one linker for the interconnection of the two monomers of the dimer is present in the fusion protein of the invention. When the recombinase complex is represented by a tetramer, one, two three or four linkers for the interconnection of the four monomers of the tetramer are present in the fusion protein of the invention.

Each of the linkers comprised in the fusion protein of the invention is suitably a peptide, preferably an oligopeptide. In a preferred embodiment, the linker consists of 4-50 amino acids, more preferably 5-40 amino acids, most preferably 6-30 amino acids.

Based on the general knowledge of the mechanism of action of recombinases and the steric conditions of the recombinase mechanism of action, it would have been surprising for the person skilled in the art that the fusion of recombinases would not inhibit recombination. Surprisingly, it was experimentally shown herein that a recombination event could be observed on a recombinase target site that was induced by binding of a fusion protein consisting of a heterodimer and a linker, which indicates an obligate fashion of the recombination event.

Accordingly, each linker comprised in a fusion protein of the invention is in one embodiment an oligopeptide comprising repeats of (G₂S) selected from the group consisting of

| | |
|---|---|
| GGSGGS | (SEQ ID NO: 1); |
| GGSGGSGGSGGS | (SEQ ID NO: 2); |
| GGSGGSGGSGGSGGSGGS | (SEQ ID NO: 3); |
| GGSGGSGGSGGSGGSGGSGGSGGS | (SEQ ID NO: 4); |
| GGSGGSGGSGGSGGSGGSGGSGGSGGSGGS | (SEQ ID NO: 5); |
| GGSGGSGGSGGSGGSGGSGGSGGSGGSGGSGGSGGS | (SEQ ID NO: 6); |
| and | |
| GGSGGSGGSGGSGGSGGSGGSGGSGGSGGSGGSGGSGGSGGS | (SEQ ID NO: 7). |

However, fusion of the recombinases with a linker comprising (G2S) repeats resulted in some cases in a decrease of recombination activity compared to the non-linked recombinases. Further experiments have shown that the length of the linker plays a significant role on the activity of the recombinases. Good results were achieved with a linker comprising eight (G₂S) repeats. A further increase of the linker length did not improve recombination efficacy, however, decreased the specificity as the obtained fusion proteins showed activity on the symmetric sites of the recombinase target sites, which is not desired. Accordingly, in a preferred embodiment, the linker comprised in the fusion protein of the invention is most preferably an oligopeptide consisting of 24 amino acids.

It has been further investigated whether the amino acid composition of the linker influences recombination activity on the final target site. Three linker libraries were designed. The purpose of the libraries was to find a linker with the same high specificity on the symmetric sites (no recombination) and improved activity on the final *loxF8* recombinase target site. Part or the whole linker sequence comprising eight (G₂S) repeats was changed to the degenerate codon RVM that codes for nine of the amino acids commonly used in natural linkers (Chen, et al., 2013), namely Ala, Arg, Asn, Asp, Glu, Gly, Lys, Ser, Thr amino acids. Thereby, a large number of linker variants were created.

In a further preferred embodiment, the fusion protein of the invention therefore comprises a linker, which comprises or consists of an oligopeptide with an amino acid sequence selected from formula 1, formula 2 and formula 3 as set out below, which represent the tree linker libraries mentioned above:

X₁-X₂-X₃-X₄-X₅-X₆-(G₂S)₄-X₇-X₈-X₉-X₁₀-X₁₁-X₁₂ (formula 1);

(G₂S)₂-X₁-X₂-X₃-X₄-X₅-X₆-X₇-X₈-X₉-X₁₀-X₁₁-X₁₂-(G₂S)₂ (formula 2);

and 
X1– X2– X3– X4– X5–X6– X7– X8– X9– X10– X11–X12- X13– X14– X15– X16– X17–X18– X19–
X20– X21– X22– X23–X24 (formula 3);
 wherein
G is glycine;
S is serine; and
each of X₁ to X₂₄ is independently selected from the group consisting of alanine, arginine, asparagine, aspartic acid, glutamine, glycine, lysine, serine and threonine.

With regard to the recombinases used in the recombinase complex, the fusion protein according to the invention preferably comprises complexes of heterodimers, wherein each recombinase enzyme of said heterodimers is a tyrosine site-specific recombinase, which have been evolved by directed evolution or generated by other means to independently recognize the half-sites of an upstream target site and the half-sites of a downstream target site of tyrosine site-specific recombinases.

Suitably, said tyrosine site-specific recombinases are selected from the group consisting of Cre, Dre-, VCre-, SCre-, Vika-, lambda-Int-, Flp-, R-, Kw-, Kd-, B2-, B3-, Nigri- and Panto-recombinase. The recognition target sites of these bacterial and yeast T-SSR systems have been discussed in Meinke *et al.,* 2016 and in Karimova *et al.,* 2016, and are shown in Table 1 below:

**Table 1. Recognition Target Sites of Bacterial and Yeast T-SSR Systems**

| **T-SSR/Site** | **DNA target sequence** |
|---|---|
| | **(Natural host)** |
| *CrelloxP* | 5'-ATAACTTCGTATA**A\|TGTATG C**TATACGAAGTTAT-3' (SEQ ID NO: 73) (Bacteriophage PI) |
| Dre/*rox* | 5'-TAACTTTAAATA**ATGCCAAT**TATTTAAAGTTA-3' (SEQ ID NO: 74) (Bacteriophage P6) |
| VCre/*Vloxp* | 5'-TCAATTTCTGAGA**ATGACAGT**TCTCGGAAATTGA-3' _(SEQ ID NO: 75) (P0908 of *Vibrio* sp.) |
| SCre/*SloxP* | 5'-CTCGTGTCCGATA**ACTGTAAT**TATCGGACATGAT-3' (SEQ ID NO: 76) (PI of *Shewanella* sp.) |
| Vika/*vox* | 5'-AATAGGTCTGAGA**ATGGGCGT**TCTCAGACGTATT-3' (SEQ ID NO: 77) (*Vibrio coralliilyticus*) |
| Lambda-Int/*attP* | 5'-CAGCTTT**TTTATAC**TAAGTTG-3' (SEQ ID NO: 78) (Bacteriophage lambda) |
| FLP/*FRT* | 5'-GAAGTTCCTATAC\|**TTTCTAGA**GAATAGGAACTTC-3' (SEQ ID NO: 79) (*Saccharomyces cerevisiae*) |
| R/*RRT* | 5'-TTGATGAAAGAA**TAACGTA**TTCTTTCATCAA-3' (SEQ ID NO: 80) (*Zygosaccharomyces rouxii*) |
| *Kw*/*KwRT* | 5'-ACGAAAAATGGTAAGGAA**TAGACCA**TTCCTTACCATTTTTCGT-3' (SEQ ID NO: 81) (*Kluyveromyces waltii*) |
| Kd/*KdRT* | 5'-AAACGATATCAGACATTTGTCTGATAATGCTTCATTATCAGACAAAT GTCTGATATCGTTT-3' (SEQ ID NO: 82) (Kluyveromyces drosophilarum) |
| B2/*B2RT* | 5'-GAGTTTCATTAAGGAA**TAACTA**ATTCCCTAATGAAACTC-3' (SEQ ID NO: 83) (*Zygosaccharomyces bailii*) |
| B3/*B3RT* | 5'-GGTTGCTTAAGAA**TAAGTAA**TTCTTAAGCAACC-3' (SEQ ID NO: 84) (*Zygosaccharomyces bisporus*) |
| Nigri/*nox* | 5'-TGAATGTCCTATA**ATTACACT**TATAGGACATTCA-3' (SEQ ID NO: 85) (*Vibrio nigripulchritudo*) |
| Panto/*pox* | 5'-GAAACTTTAAATA**ATAAGTCT**TATTTAAAGTTTC-3' (SEQ ID NO: 86) (*Pantoea sp. aB*) |

- Solid underlined:: left half -site
- Dashed-underlined:: right half-site
- Bold:: Spacer sequence

As described above, a majority of severe cases of hemophilia A in patients are caused by the inversion due to intrachromosomal recombination with homologous regions outside of the F8 gene (Graw *et al.,* 2005), such as the inversion of exon 1 due to homologous recombination between two 1 kb sequences, in intron 1 and in the region positioned telomeric to the F8 gene (Figure 4).

It was a further problem of the present invention to provide a fusion protein, which specifically corrects the DNA inversions, which are causing severe HA, preferably the inversion of exon 1. In order to solve this problem, two *loxF8* target sites were identified in intron 1 and the homologous region positioned telomeric to the gene F8. Afterwards, two SSRs recognizing different half-sites of *loxF8* were evolved by directed molecular evolution (Figure 5A). Each of the so evolved recombinases recognizes one half-site of a final target site, and cooperatively perform the recombination.

By altering the DNA-binding properties of naturally occurring SSRs, these systems can be repurposed to recombine DNA sequences with therapeutic value. Methods to create designer recombinases are known in the art. WO 2018/229226 A1 for example teaches vectors and methods to generate designer DNA recombining enzymes by directed molecular evolution. WO2008083931A1 discloses the directed molecular evolution of tailored recombinases (Tre 1.0) that uses sequences in the long terminal repeat (LTR) of HIV as recognition sites (loxLTR Tre 1.0). Further developments of this approach using asymmetric target sites were described in WO2011147590 A2 (Tre 3.0) and WO2016034553 A1 (Tre 3.1 and Tre/Brec1) as well as the publication Karpinski J et al 2016 (Brec1). Methods to engineer naturally occurring or designer-recombinases by rational design are also known to the art (e.g. Abi-Ghanem *et al.,* 2013; Karimova *et al.,* 2016)

As an alternative to generating a single designer-recombinase that targets an asymmetric target site, dual designer-recombinases that recombine the target site together as a heterodimer can also be generated (Lansing *et al.,* 2019). However, a comparative study for a single designer-recombinase and a dual designer-recombinase system (heterodimer), targeting the same DNA target has not been performed, yet. To perform such a comparative study, recombinases of the two classes utilizing SLiDE technology (Buchholz and Stewart, 2001; Lansing *et al.,* 2019) were generated that were targeted to recombine a conserved sequence in the int1h1 and intlh2 sequences (*loxF8,* Fig. 4, Fig. 5).

To obtain a single recombinase recognizing the target sequence as a monomer, SLiDE was performed on the asymmetric *loxF8* site, equivalent to the directed evolution of Tre and Brec1 on the asymmetric *loxLTR* and *loxBRT* sequences, respectively (Sarkar *et al.,* 2007; Hauber *et al.,* 2013). After 168 rounds of directed evolution, individual recombinases were tested in *E.coli* in the pEVO vector using different L-arabinose concentrations. The best clone identified (H7) recombined the *loxF8* sequence when recombinase expression was induced with low concentrations of L-arabinose (Fig. 6).

To generate a recombinase system recognizing the same target sequence as a heterodimer, SLiDE was first performed on the symmetric *loxF8* half-sites, equivalent to the directed evolution of the Hex recombinases, recombining a sequence on human chromosome 7 (Lansing *et al.,* 2019). Recombinase libraries with activity on the symmetric *loxF8* half-sites (after 88 and 89 rounds of directed evolution, respectively) were then coexpressed in *E.coli* from a pEVO-plasmid harboring two full, asymmetric *loxF8* target sites. After 3 additional rounds of directed evolution, dual recombinase heterodimers were tested in *E.coli* in the pEVO vector using different L-arabinose concentrations. The best heterodimer clone identified (D7) recombined the *loxF8* sequence more efficiently than the single H7 clone (Fig. 6), indicating that the generation of dual recombinase heterodimers is more effective than the generation of a single recombinase targeting an asymmetric site.

To investigate the recombination activities of the monomer (H7) and heterodimer (D7) F8-recombinases in mammalian cells, HeLa cells were co-transfected with recombination reporter and recombinase expression plasmids (Fig. 7A). As in the *E.coli* assays, the H7 recombinase was less efficient than the heterodimer D7 recombinase (Fig. 7B; 28% recombination efficiency versus 46% recombination efficiency).

To compare the abilities of the single F8-recombinase (H7) and the heterodimer F8 recombinase (D7) to induce the F8 inversion in human cells, the recombinase expression plasmids were transfected into HeLa cells. 48 hours post transfection genomic DNA was isolated. A PCR reaction was performed to detect the genomic inversion. Again, the monomer H7 recombinase did not perform as well as the heterodimer D7 recombinase in this assay (Fig. 8).

To compare the specificity of the H7 monomer with the D7 heterodimer, the recombinases were tested on nine human sequences that display the highest similarity to the *loxF8* sequence (SEQ ID NOs: 21 to 23 and 87 to 92, Table 2). The nine off-target sites and the *loxF8* on-target site were cloned as excision substrates into the pEVO vectors harboring the respective recombinases and the plasmids were grown for 24 hours in the presence of 10 µg/ml L-arabinose in *E.coli.* DNA extracted from these cultures revealed that the H7 monomer recombined the *loxF8* site (SEQ ID NO: 17) in addition to the off-target sites with the SEQ ID NOs: 21, 22, 87 and 91. In contrast, the D7 heterodimer was more efficient in recombining the *loxF8* sequence and also much more specific and only slightly recombined the off-target sites with the SEQ ID NO: 22 (Fig. 9). Based on these cumulative results, the heterodimer D7 was selected for further experiments.

While the D7 heterodimer showed better characteristics than the H7 monomer, it still showed some off-target recombination. For therapeutic applications it is desirable to employ genome editing tools that are highly specific and do not edit the genome at any other site then the intended one. As previous attempts have only reported limited success in improving the applied properties of heterospecific recombinases, possibly ways to improve the behavior of dual recombinases were explored. One possibility was to physically connect the two D7 heterodimers by fusing them with a peptide linker. State of the art literature suggested that this would most likely not work, as the individual enzymes need to move considerably during the recombination reaction. However, if it worked, this approach might provide a straightforward method for generating highly efficient and specific obligate recombination systems.

In order to obtain the fused heterodimers with the best linker properties, they were selected for the activity on the *loxF8* site and counter-selected for activity on both of the symmetric sites (Fig. 10). Linked recombinases can theoretically form different protein complexes with activity on the symmetric or asymmetric target sites (Fig. 11). All three initial libraries showed activity on the symmetric sites, indicating that the linker composition has an important influence on the specificity. Fusion proteins with linkers having an amino acid sequence selected from SEQ ID NOs. 8 to 16 showed a satisfying activity on the *loxF8* site.

The invention therefore provides in a further embodiment a fusion protein, wherein said linker comprises or consists of an oligopeptide selected from the group consisting of

| | |
|---|---|
| AEATSEGGSGGSGGSGGSNGARRT | (SEQ ID NO: 8); |
| AGTTARGGSGGSGGSGGSGRRGAK | (SEQ ID NO: 9); |
| KNGRGRGGSGGSGGSGGSRTKRET | (SEQ ID NO: 10); |
| GGSGGSTAAKEGAASSASGGSGGS | (SEQ ID NO: 11); |
| GGSGGSNSRSNTENSDKGGGSGGS | (SEQ ID NO: 12); |
| GGSGGSNGEEGTERGKATGGSGGS | (SEQ ID NO: 13); |
| GGSGGSTTKANRAKGGRGGGSGGS | (SEQ ID NO: 14); |
| GANEDTNTEAAGSEGNEKTGTNSA | (SEQ ID NO: 15); and |
| GESRAEDGAKGNGRGKGEATAGAA | (SEQ ID NO: 16). |

Further important criteria for the linker selection were the activity on the *loxF8* site at low induction level and the absence of activity on the symmetric sites of *loxF8* at high induction level (Fig. 16, Fig. 17). Best results in this regard were achieved with a fusion protein comprising a linker with the amino acid sequence
GGSGGSTTKANRAKGGRGGGSGGS (SEQ ID NO: 14).

Accordingly, in the preferred embodiment, the invention provides a fusion protein, wherein said linker comprises or consists of an oligopeptide, which has an amino acid sequence of SEQ ID NO: 14.

More preferably, the fusion protein of the invention specifically recognizes the upstream recombinase target sequence of the *loxF8* target site, which has the nucleic acid sequence
ATAAATCTGTGGAAACGCTGCCACACAATCTTAG (SEQ ID NO: 17);
and that recognizes the downstream recombinase target sequence of the loxF8 target site, which has the nucleic acid sequence
CTAAGATTGTGTGGCAGCGTTTCCACAGATTTAT (SEQ ID NO: 18)

Most preferably, the fusion protein of the invention shows high specificity on the *loxF8* target site with the target sequences of SEQ ID NOs: 17 and 18 and does not show activity on off-target sites at a high induction level (Fig. 20). Off-target sites, which are preferably not recognized by the fusion protein of the invention, are selected from the group consisting of SEQ ID NOs: 19 to 29 and 87 to 92 as shown in Table 2.

**Table 2: Nucleic acid sequences of off-target sites**

| **Target site** | **Sequence** | **SED ID NO:** |
|---|---|---|
| loxF8 | ATAAATCTGTGGA AACGCTGC CACACAATCTTAG | 17 |
| Sym1 | ATAAATCTGTGGA AACGCTGC TCCACAGATTTAT | 19 |
| Sym2 | CTAAGATTGTGTG AACGCTGC CACACAATCTTAG | 20 |
| 1LR | ATAAATTTGTGGA AATTAAAC **A**ACACACTCTTA**A** | 21 |
| 2LR | A**C**AAA**AT**TGTGGA AATTAAAC **A**ACACAATCTTA**A** | 22 |
| 3LR | A**C**AAAT**A**TGTGGA AATTAAAC **A**ACACA**C**TCTT**GA** | 23 |
| 4LR | ATAAAT**T**TGTGGA AATTAAAC **A**ACACACTCTTA**A** | 24 |
| 5LR | **T**TAA**GAG**TGTG**TT** TTTTAATT **TC**CACA**TAT**TT**GT** | 25 |
| 1L | ATAAAT**A**TGTG**A**A TATACATA **TT**CACA**T**ATTTAT | 26 |
| 2L | ATA**T**ATCT**A**T**A**GA TATAGATA TCCACAGAT**A**TAT | 27 |
| 1R | CTA**T**G**T**TT**T**TG**A**G GTCTTATT CACA**A**AATCTT**TT** | 28 |
| 2R | CTATTATTGTGTA ACAAATTA CCCCCAAACTTAG | 29 |
| HS4 | ATAAAT**A**TGTG**TG** TATATATA CACACAA**A**C**A**TA**T** | 87 |
| HS5 | ATA**T**ATCTGTG**T**A TATATATA CACACA**CA**C**A**TA**T** | 88 |
| HS6 | ATA**T**AT**A**TGTG**T**A TATATATA CACACA**TA**C**A**TA**T** | 89 |
| HS7 | ATA**T**AT**G**TGTG**T**A TATATATA CACACA**CA**C**A**TA**C** | 90 |
| HS8 | ATAAAT**A**TGTG**T**A AACTAAAC **A**ACACA**C**TCTTA**A** | 91 |
| HS9 | A**C**AAAT**A**TGTGGA AACTAAAC **A**ACACA**T**TCTT**GA** | 92 |

| | | |
|---|---|---|
| Bold: Sequences that are non-homologous to the target *loxF8* sequence (SEQ ID NO: 17) Underlined: Spacer sequences | | |

The upstream (5') recombinase target sequence of the *loxF8* target site, which has the nucleic acid sequence of SEQ ID NO: 17; and the downstream (3') recombinase target sequence of the *loxF8* target site, which has the nucleic acid sequence SEQ ID NO: 18, have been identified as part of the invention. Accordingly, the invention relates in further preferred embodiment to a *loxF8* recombinase target site comprising a 5' target sequence of SEQ ID NO: 17 and a 3' target sequence of SEQ ID NO: 17.

To generate DNA recombinases that recombine the upstream and downstream target sequences of the *loxF8* target site, the substrate linked directed evolution approach was employed (Buchholz and Stewart 2001).

Said DNA recombinase according to the invention is an enzyme, which recombines a nucleic acid, in particular DNA, sequences by recognizing two target sites (recognition sites, i.e. one upstream and one downstream recognition site or sequence) and causing a deletion, an insertion, an inversion or a replacement of a DNA sequence. Advantageously, the DNA recombinase according to the invention recognizes the asymmetric recognition sites of the *loxF8* sequence according to SEQ ID No. 17 (upstream) and SEQ ID NO: 18 (downstream). These recognition sites do not occur anywhere else in the human genome and therefore can be used for specific DNA recombination. The DNA recombinase according to the invention advantageously does not need target sites that are artificially introduced in the genome. Further advantageously and most preferably, the DNA recombinase according to the invention causes an inversion of a DNA sequence. A further advantage is that the DNA recombinase according to the invention allows precise genome editing without triggering endogenous DNA repair pathways.

Through several cycles of evolution, enzymes with activity on target sequences of SEQ ID NO: 17 and SEQ ID NO: 18 were generated.

The problem has firstly been solved by a recombinase monomer which has an amino acid sequence with at least 70 %, preferably 80 %, more preferably 90 %, sequence identity with a sequence according to SEQ ID NO: 66. In a preferred embodiment, the invention therefore provides a recombinase monomer which has an amino acid sequence with at least 70 %, preferably 80 %, more preferably 90 %, sequence identity with a sequence according to SEQ ID NO: 66.

In a preferred embodiment, the DNA recombinase of SEQ ID NO: 66 comprises one or more mutations compared to the Cre recombinase protein of SEQ ID NO: 68. Exemplary mutations include L5Q, V7L, P12S, P15L, V16A, V23T, M30V, F31L, R34S, H40Q, M44S, S47F, K57E, K62E, Y77H, Q90N, Q94S, S108G, T140A, D143S, Q144R, S147A, C155P, I166V, A175S, A231V, K244R, N245G, A249K, R259C, E262Q, E266A, T268A, I272L, S305P, P307V, E308Q, N317T, N319E and I320S. More preferably, the recombinase of SEQ ID NO: 66 has, compared to the Cre recombinase protein of SEQ ID NO: 68, one or more, preferably two, three, for, five, six, eight, nine, ten or more mutations selected from the group consisting of include L5Q, V7L, P12S, P15L, V16A, V23T, M30V, F31L, R34S, H40Q, M44S, S47F, K57E, K62E, Y77H, Q90N, Q94S, S108G, T140A, D143S, Q144R, S147A, C155P, I166V, A175S, A231V, K244R, N245G, A249K, R259C, E262Q, E266A, T268A, I272L, S305P, P307V, E308Q, N317T, N319E and I320S. In a most preferred embodiment, the recombinase of SEQ ID NO: 66 has all of the aforementioned mutations.

For the avoidance of doubt, the amino acid which is indicated by the one letter code before the position number represents the amino acid in the wildtype sequence (for example SEQ ID NO: 68 of Cre recombinase); and the amino acid which is indicated by the one letter code after the position number represents the amino acid in the mutated, i.e. the evolved recombinase of the present invention.

Further most preferably, the sequence with at least 70 %, preferably 80 %, more preferably 90 %, sequence identity with a recombinase of SEQ ID NO: 66 is characterized by the following specific amino acids: Q at position 5, Q at position 40, S at position 44, N at position 90, S at position 94, S at position 143, R at position 144, P at position 155, V at position 231, K at position 249, and L at position 272. These amino acid residues are predicted critical residues for target site recognition based on deep sequencing and are not found in the sequence of wildtype Cre-recombinase of SEQ ID NO: 68.

Because the recombination efficiency and specificity of this recombinase monomer was not satisfactory, DNA recombinase heterodimers were developed, also through several cycles of evolution.

As a result, the problem of the invention has been further solved by a DNA recombinase, which consists of a heterodimer, wherein said heterodimer comprises or consists of a first recombinase enzyme and a second recombinase enzyme, wherein said first recombinase enzyme is a polypetide, which has an amino acid sequence with at least 70 %, preferably 80 %, more preferably 90 %, sequence identity with a sequence according to SEQ ID NO: 30 and wherein said second recombinase enzyme is a polypeptide, which has an amino acid sequence with at least 70 %, preferably 80 %, more preferably 90 %, sequence identity with a sequence according to SEQ ID NO: 31.

Preferably, the DNA recombinase of SEQ ID NO: 30 comprises one or more mutations compared to the Cre recombinase protein of SEQ ID NO: 68. Exemplary mutations include N3D, L5Q, V7L, P12S, P15L, V23A, M30V, H40A, K43N, M44T, L58S, K62E, Y77H, A80V, K86N, Q90R, G93A, Q94L, S108G, A131V, K132R, E150G, N151S, C155R, Q156N, I166V, A175S, V182I, I195V, K219R, D232G, T253S, S257T, R259D, A260V, E262R, E266V, T268A, I272V, Y273H, K276R, A285R, P307A, N317T, N319E, I320S, N323S and I325L. More preferably, the recombinase of SEQ ID NO: 30 has, compared to the Cre recombinase protein of SEQ ID NO: 68, one or more, preferably two, three, for, five, six, eight, nine, ten or more mutations selected from the group consisting of N3D, L5Q, V7L, P12S, P15L, V23A, M30V, H40A, K43N, M44T, L58S, K62E, Y77H, A80V, K86N, Q90R, G93A, Q94L, S108G, A131V, K132R, E150G, N151S, C155R, Q156N, I166V, A175S, V182I, I195V, K219R, D232G, T253S, S257T, R259D, A260V, E262R, E266V, T268A, I272V, Y273H, K276R, A285R, P307A, N317T, N319E, I320S, N323S and I325LIn a most preferred embodiment, the recombinase of SEQ ID NO: 30 has all of the aforementioned mutations.

Further most preferably, the recombinase of SEQ ID NO: 30 exhibits a sequence characterized by the following specific amino acids: Q at position 5, A at position 40, T at position 44, V at position 80, R at position 90, L at position 94, R at position 155, R at position 219, V at position 266, V at position 272, S at position 323 and L at position 325. These amino acid residues are predicted critical residues for target site recognition based on deep sequencing and are not found in the sequence of wildtype Cre-recombinase of SEQ ID NO: 68.

Preferably, the DNA recombinase of SEQ ID NO: 31 comprises one or more mutations compared to the Cre recombinase protein of SEQ ID NO: 68. Exemplary mutations include L5Q, T6A, V7L, L11P, P12S, A13T, P15L, V16A, S20C, R34S, M44A, L46Q, A53V, N60S, K62R, Y77H, A80T, Q90H, Q94S, R101Q, S108G, K122R, T140A, F142L, S147A, I166V, A175G, K183R, N235D, K244R, N245Y, A249K, R259Y, E262Q, E266G, T268A, I272V, R282K, I306L, N317T, I320S and G342D. More preferably, the recombinase of SEQ ID NO: 31 has, compared to the Cre recombinase protein of SEQ ID NO: 68, one or more, preferably two, three, for, five, six, eight, nine, ten or more mutations selected from the group consisting of L5Q, T6A, V7L, L11P, P12S, A13T, P15L, V16A, S20C, R34S, M44A, L46Q, A53V, N60S, K62R, Y77H, A80T, Q90H, Q94S, R101Q, S108G, K122R, T140A, F142L, S147A, I166V, A175G, K183R, N235D, K244R, N245Y, A249K, R259Y, E262Q, E266G, T268A, I272V, R282K, I306L, N317T, I320S, G342D. In a most preferred embodiment, the recombinase of SEQ ID NO: 31 has all of the aforementioned mutations.

Further most preferably, the sequence with at least 70 %, preferably 80 %, more preferably 90 %, sequence identity with a recombinase of SEQ ID NO: 31 is characterized by the following specific amino acids: Q at position 5, A at position 44, T at position 80, H at position 90, S at position 94, K at position 249, G at position 266, V at position 272 and K at position 282. These amino acid residues are predicted critical residues for target site recognition based on deep sequencing and are not found in the sequence of wildtype Cre-recombinase of SEQ ID NO: 68.

Recombinant protein expression using bacterial and other host organisms is a fundamental technology for protein production. A key step in recombinant protein expression is codon optimization where a coding sequence for a protein of interest is designed by synonymous substitution aiming to increase its expression level. In conventional approach, rare codons are substituted by frequent codons according to the genomic codon usage in a host organism. The basis of this approach is that endogenous genes whose coding sequences consist of frequent codons have high protein expression levels, and thus recombinant protein expression is also considered to be improved by increasing the codon frequency. Another approach is to introduce synonymous substitution computationally predicted to destabilize mRNA secondary structures. Since stable mRNA secondary structures may inhibit translation, this approach is considered to improve recombinant protein expression by enhancing translational efficiency. The association between these sequence features and protein expression levels has been indicated by omics analyses of endogenous genes. The direct evidence of their influences in recombinant protein expression has been shown in the art using a relatively small number of genes.

After performing further cycles of molecular directed evolution, a DNA recombinase has been developed, which shows improved enzyme activity.

Accordingly, the invention provides in a further embodiment a DNA recombinase, which is a heterodimer, wherein the first monomer is recombinase enzyme which has a sequence with at least 70 %, preferably 80 %, more preferably 90 % sequence identity with a sequence according to SEQ ID NO. 32 and wherein the second monomer is a recombinase enzyme which has a sequence with at least 70 %, preferably 80 %, more preferably 90 %, sequence identity with a sequence according to SEQ ID NO. 33..

Preferably, the DNA recombinase of SEQ ID NO: 32 comprises one or more mutations compared to the Cre recombinase protein of SEQ ID NO: 68. Exemplary mutations include L5Q, V7L, P12S, P15L, V16A, D17N, V23A, M30V, Q35R, H40A, M44T, S51T, Y77H, A80V, K86N, Q90R, G93A, Q94L, S108G, N111S, A131V, K132R, Q144K, E150G, I166V, A175S, V182I, K219R, E222G, D232G, R259D, A260V, E262R, I264V, E266A, T268A, I272V, A275T, R282G, A285T, P307A, N317T, N319E, I320S, N323S and I325L. In a more preferred embodiment, the recombinase of SEQ ID NO: 32 has, compared to the Cre recombinase protein of SEQ ID NO: 68, one or more, preferably two, three, for, five, six, eight, nine, ten or more mutations selected from the group consisting of L5Q, V7L, P12S, P15L, V16A, D17N, V23A, M30V, Q35R, H40A, M44T, S51T, Y77H, A80V, K86N, Q90R, G93A, Q94L, S108G, N111S, A131V, K132R, Q144K, E150G, I166V, A175S, V182I, K219R, E222G, D232G, R259D, A260V, E262R, I264V, E266A, T268A, I272V, A275T, R282G, A285T, P307A, N317T, N319E, I320S, N323S and I325L. In a most preferred embodiment, the recombinase of SEQ ID NO: 32 has all of the aforementioned mutations.

Further most preferably, the sequence with at least 70 %, preferably 80 %, more preferably 90 %, sequence identity with a recombinase of SEQ ID NO: 32 is characterized by the following specific amino acids: Q at position 5, A at position 40, T at position 44, V at position 80, R at position 90, L at position 94, K at position 144, R at position 219, V at position 272, G at position 282, S at position 323 and L at position 325. These amino acid residues are predicted critical residues for target site recognition based on deep sequencing and are not found in the sequence of wildtype Cre-recombinase of SEQ ID NO: 68.

Preferably, the DNA recombinase of SEQ ID NO: 33 comprises one or more mutations compared to the Cre recombinase protein of SEQ ID NO: 68. Exemplary mutations include L4I, L5Q, V7P, N10S, P12S, P15L, V16T, E22V, V23T, M28A, R34S, K57E, F64L, A66V, Y77H, A80T, Q90H, Q94S, S102A, S108G, K122R, K132Q, M149V, I166V, A175S, K244R, N245Y, A249K, R259Y, E262Q, E266G, T268A, I272V, D277G, R282K, S305P, N317T, I320S and G342S. In a more preferred embodiment, the recombinase of SEQ ID NO: 33 has, compared to the Cre recombinase protein of SEQ ID NO: 68, one or more, preferably two, three, for, five, six, eight, nine, ten or more mutations selected from the group consisting of L4I, L5Q, V7P, N10S, P12S, P15L, V16T, E22V, V23T, M28A, R34S, K57E, F64L, A66V, Y77H, A80T, Q90H, Q94S, S102A, S108G, K122R, K132Q, M149V, I166V, A175S, K244R, N245Y, A249K, R259Y, E262Q, E266G, T268A, I272V, D277G, R282K, S305P, N317T, I320S and G342S. In a most preferred embodiment, the recombinase of SEQ ID NO: 31 has all of the aforementioned mutations.

Further most preferably, the sequence with at least 70 %, preferably 80 %, more preferably 90 %, sequence identity with a recombinase of SEQ ID NO: 33 is characterized by the following specific amino acids: Q at position 5, T at position 80, H at position 90, S at position 94, K at position 249, G at position 266, V at position 272 and K at position 282. These amino acid residues are predicted critical residues for target site recognition based on deep sequencing and are not found in the sequence of wildtype Cre-recombinase of SEQ ID NO: 68.

Preferably, the DNA recombinase monomer of SEQ ID NO: 32 exhibits a sequence, wherein the amino acid residue at position 5 is glutamine, the amino acid residue at position 17 is asparagine, the amino acid residue at position 35 is arginine, the amino acid residue at position 40 is alanine, the amino acid residue at position 44 is threonine, the amino acid residue at position 80 is valine, the amino acid residue at position 90 is arginine, the amino acid residue at position 94 is leucine, the amino acid residue at position 111 is serine, the amino acid residue at position 132 is arginine, the amino acid residue at position 144 is lysine, the amino acid residue at position 150 is glycine, the amino acid residue at position 219 is arginine, the amino acid residue at position 222 is glycine, the amino acid residue at position 264 is valine, the amino acid residue at position 272 is valine, the amino acid residue at position 275 is threonine, the amino acid residue at position 282 is glycine, the amino acid residue at position 323 is serine and/or the amino acid residue at position 325 is leucine.

These amino acid residues are specific for the DNA recombinase according to the invention and have not been found in the Cre recombinase (SEQ ID NO. 68).

Preferably, the DNA recombinase monomer of SEQ ID NO: 33 exhibits a sequence, wherein the amino acid residue at position 4 is isoleucine, the amino acid residue at position 5 is glutamine, the amino acid residue at position 7 is proline, the amino acid residue at position 16 is threonine, the amino acid residue at position 22 is valine, the amino acid residue at position 23 is threonine, the amino acid residue at position 28 is alanine, the amino acid residue at position 64 is leucine, the amino acid residue at position 66 is valine, the amino acid residue at position 80 is threonine, the amino acid residue at position 90 is histidine, the amino acid residue at position 94 is serine, the amino acid residue at position 102 is alanine, the amino acid residue at position 132 is glutamine, the amino acid residue at position 149 is valine, the amino acid residue at position 249 is Lysine, the amino acid residue at position 266 is glycine, the amino acid residue at position 272 is valine, the amino acid residue at position 277 is glycine, the amino acid residue at position 282 is lysine, the amino acid residue at position 305 is proline and/or the amino acid residue at position 342 is serine.

These amino acid residues are specific for the DNA recombinase according to the invention and have not been found in the Cre recombinase (SEQ ID NO. 68).

The DNA recombinases according to the invention are site-specific recombinases that target a sequence present in the Intlh repeat sequence of the factor 8 gene, which is more preferably inverted by the activity of the DNA recombinases according to the invention.

The DNA recombinases of the present invention include the polypeptides of SEQ ID NOs.: 30 to 33, as well as polypeptides which have at least 75 % similarity (e.g. preferably at least 50 %; and more preferably at least 70 % identity) to a DNA recombinase of SEQ ID NOs.: 30 to 33, more preferably at least 85 % similarity (e.g. preferably at least 70 % identity) to a DNA recombinase of SEQ ID NOs.: 30 to 33, and most preferably at least 95 % similarity (e.g. preferably at least 90 % identity) to a DNA recombinase of SEQ ID NOs.: 30 to 33. Moreover, they should preferably include exact portions of such DNA recombinases containing a sequence of at least 30 amino acids, and more preferably at least 50 amino acids. In a preferred embodiment, as used herein, the term "identity" refers to the amount of amino acids in accordance with the amino acid sequences SEQ ID NO.: 30, SEQ ID NO.: 31; SEQ ID NO.: 32 or SEQ ID NO. 33 with regard to the total number of amino acids.

Fragments or portions of the polypeptides of the present invention may be employed as intermediates for producing the corresponding full-length polypeptides by peptide synthesis. Fragments or portions of the polynucleotides of the present invention may also be used to synthesize full-length polynucleotides of the present invention.

In accordance with the present invention, activity and specificity of the DNA recombinases have been further increased by incorporating them in a fusion protein, wherein the monomers of said DNA recombinases are interconnected by a linker as described above.

Accordingly, the invention provides in a preferred embodiment a fusion protein, comprising a heterodimer of a DNA recombinase, wherein said heterodimer comprises a first recombinase enzyme, which has an amino acid sequence with at least 70 %, preferably 80 %, more preferably 90 %, sequence identity with a sequence selected from SEQ ID NO: 30 or 32; and a second recombinase enzyme, which has the amino acid sequence with at least 70 %, preferably 80 %, more preferably 90 %, sequence identity with a sequence selected from SEQ ID NO: 31 or 33 for the recognition of an upstream target sequence and a downstream target sequence of a recombinase target site.

In a more preferred embodiment, the invention provides a fusion protein, comprising a heterodimer of a DNA recombinase, wherein said heterodimer comprises a first recombinase enzyme, which has an amino acid sequence with at least 70 %, preferably 80 %, more preferably 90 %, sequence identity with a sequence according to SEQ ID NO: 30 and a second recombinase enzyme, which has the amino acid sequence with at least 70 %, preferably 80 %, more preferably 90 %, sequence identity with a sequence according to SEQ ID NO: 31 for the recognition of an upstream target sequence and a downstream target sequence of a recombinase target site.

In a most preferred embodiment, the invention provides a fusion protein, comprising a heterodimer of a DNA recombinase, wherein said heterodimer comprises a first recombinase enzyme, which has an amino acid sequence with at least 70 %, preferably 80 %, more preferably 90 %, sequence identity with a sequence according to SEQ ID NO: 32 and a second recombinase enzyme, which has the amino acid sequence with at least 70 %, preferably 80 %, more preferably 90 %, sequence identity with a sequence according to SEQ ID NO: 33 for the recognition of an upstream target sequence and a downstream target sequence of a recombinase target site.

As experimentally shown, the activity of the heterodimer - fusion protein is further influenced by the orientation, in which the recombinase monomers and the linker are connected to each other. Best activity of the heterodimer on the *loxF8* site has been shown, when the first recombinase enzyme, the second recombinase enzyme and the linker are interconnected such that
i. the C-terminus of the recombinase enzyme with the amino acid sequence of SEQ ID NO: 31, which specifically recognizes a second half-site, such as the right half-site of a recombinase target site, is fused with the linker to the N-terminus of the recombinase enzyme with the amino acid sequence of SEQ ID NO: 30, which specifically recognizes a first half-site, such as the left half-site of a recombinase target site; and more preferably
ii. the C-terminus of the recombinase enzyme with the amino acid sequence of SEQ ID NO: 33, which specifically recognizes a second half-site, such as the right half-site of a recombinase target site, is fused with the linker to the N-terminus of the recombinase enzyme with the amino acid sequence of SEQ ID NO: 32, which specifically recognizes a first half-site, such as the left half-site of a recombinase target site.

Accordingly, the complete fusion protein of the invention has in a preferred embodiment an amino acid sequence with at least 70 %, preferably 80 %, more preferably 90 %, sequence identity with a sequence
i. according to SEQ ID NO: 71; or most preferably
ii. according to SEQ ID NO: 72.

The invention further relates to a nucleic acid molecule, such as a polynucleotide or nucleic acid encoding a DNA recombinase or monomers thereof or a fusion protein according to the invention.

The "polynucleotides" or "nucleic acids" of the present invention may be in the form of RNA or in the form of DNA; DNA should be understood to include cDNA, genomic DNA, recombinant DNA and synthetic DNA. The DNA may be double-stranded or single-stranded and, if single stranded, may be the coding strand or non-coding (antisense) strand. The coding sequence, which encodes the polypeptide may be identical to the coding sequence for the polypeptides shown in SEQ ID NOs: 30 to 33, preferably of SEQ ID NOs: 32 and 33, or it may be a different coding sequence encoding the same polypeptide, as a result of the redundancy or degeneracy of the genetic code or a single nucleotide polymorphism. For example, it may also be an RNA transcript which includes the entire length of coding sequence for a polypeptide of any one of SEQ ID NOs 30 to 33. In a preferred embodiment, the "polynucleotide" according to the invention is one of SEQ ID NOs 34 to 37 and 67, wherein the polynucleotide of SEQ ID NO: 34 encodes a DNA recombinase monomer of SEQ ID NO: 30; the polynucleotide of SEQ ID NO: 35 encodes a DNA recombinase monomer of SEQ ID NO: 31, the polynucleotide of SEQ ID NO: 36 encodes a DNA recombinase monomer of SEQ ID NO: 32, the polynucleotide of SEQ ID NO: 37 encodes a DNA recombinase monomer of SEQ ID NO: 33; and the polynucleotide of SEQ ID NO: 67 encodes a DNA recombinase monomer of SEQ ID NO: 66.

The nucleic acids which encode the polypeptides of SEQ ID NOs: 30 to 33 and 66, preferably of SEQ ID NOs: 32 and 33 may include but are not limited to the coding sequence for the polypeptide alone; the coding sequence for the polypeptide plus additional coding sequence, such as a leader or secretory sequence or a proprotein sequence; and the coding sequence for the polypeptide (and optionally additional coding sequence) plus non-coding sequence, such as introns or a non-coding sequence 5' and/or 3' of the coding sequence for the polypeptide. The nucleic acids which encode the polypeptides of SEQ ID NOs: 30 to 33 and 66, preferably of SEQ ID NOs: 32 and 33 include nucleic acids, which have been codon-optimized for expression in human cells. They may further contain a nuclear localization sequence.

Thus, the term "polynucleotide encoding a polypeptide" or the term "nucleic acid encoding a polypeptide" should be understood to encompass a polynucleotide or nucleic acid which includes only a coding sequence for a DNA recombinase enzyme of the invention, e.g. a polypeptide selected from SEQ ID NOs: 30 to 33 and 66, preferably of SEQ ID NOs: 32 and 33 as well as one which includes additional coding and/or non-coding sequence. The terms polynucleotides and nucleic acid are used interchangeably.

The present invention also includes polynucleotides where the coding sequence for the polypeptide may be fused in the same reading frame to a polynucleotide sequence which aids in expression and secretion of a polypeptide from a host cell; for example, a leader sequence which functions as a secretory sequence for controlling transport of a polypeptide from the cell may be so fused. The polypeptide having such a leader sequence is termed a preprotein or a preproprotein and may have the leader sequence cleaved, by the host cell to form the mature form of the protein. These polynucleotides may have a 5' extended region so that it encodes a proprotein, which is the mature protein plus additional amino acid residues at the N-terminus. The expression product having such a prosequence is termed a proprotein, which is an inactive form of the mature protein; however, once the prosequence is cleaved, an active mature protein remains. The additional sequence may also be attached to the protein and be part of the mature protein. Thus, for example, the polynucleotides of the present invention may encode polypeptides, or proteins having a prosequence, or proteins having both a prosequence and a presequence (such as a leader sequence).

The polynucleotides of the present invention may also have the coding sequence fused in frame to a marker sequence, which allows for purification of the polypeptides of the present invention. The marker sequence may be an affinity tag or an epitope tag such as a polyhistidine tag, a streptavidin tag, a Xpress tag, a FLAG tag, a cellulose or chitin binding tag, a glutathione-S transferase tag (GST), a hemagglutinin (HA) tag, a c-myc tag or a V5 tag.

The HA tag would correspond to an epitope obtained from the influenza hemagglutinin protein (Wilson et al., 1984), and the c-myc tag may be an epitope from human Myc protein (Evans et al., 1985).

If the nucleic acid of the invention is a mRNA, in particular for use as a medicament, the delivery of mRNA therapeutics has been facilitated by significant progress in maximizing the translation and stability of mRNA, preventing its immune-stimulatory activity and the development of in vivo delivery technologies. The 5' cap and 3' poly(A) tail are the main contributors to efficient translation and prolonged half-life of mature eukaryotic mRNAs. Incorporation of cap analogs such as ARCA (anti-reverse cap analogs) and poly(A) tail of 120-150 bp into in vitro transcribed (IVT) mRNAs has markedly improved expression of the encoded proteins and mRNA stability. New types of cap analogs, such as 1,2-dithiodiphosphate-modified caps, with resistance against RNA decapping complex, can further improve the efficiency of RNA translation. Replacing rare codons within mRNA protein-coding sequences with synonymous frequently occurring codons, so-called codon optimization, also facilitates better efficacy of protein synthesis and limits mRNA destabilization by rare codons, thus preventing accelerated degradation of the transcript. Similarly, engineering 3' and 5' untranslated regions (UTRs), which contain sequences responsible for recruiting RNA-binding proteins (RBPs) and miRNAs, can enhance the level of protein product. Interestingly, UTRs can be deliberately modified to encode regulatory elements (e.g., K-turn motifs and miRNA binding sites), providing a means to control RNA expression in a cell-specific manner. Some RNA base modifications such as N1-methyl-pseudouridine have not only been instrumental in masking mRNA immune-stimulatory activity but have also been shown to increase mRNA translation by enhancing translation initiation. In addition to their observed effects on protein translation, base modifications and codon optimization affect the secondary structure of mRNA, which in turn influences its translation. Respective modifications of the nucelic acid molecules of the invention are also contemplated by the invention.

The present invention is considered to further provide polynucleotides which hybridize to the hereinabove-described sequences wherein there is at least 70 %, preferably at least 90 %, and more preferably at least 95 % identity or similarity between the sequences, and thus encode proteins having similar biological activity. Moreover, as known in the art, there is "similarity" between two polypeptides when the amino acid sequences contain the same or conserved amino acid substitutes for each individual residue in the sequence. Identity and similarity may be measured using sequence analysis software (e.g., ClustalW at PBIL (Pôle Bioinformatique Lyonnais) http://npsa-pbil.ibcp.fr). The present invention particularly provides such polynucleotides, which hybridize under stringent conditions to the hereinabove-described polynucleotides.

Suitably stringent conditions can be defined by, e.g., the concentration of salt or formamide in the prehybridization and hybridization solution, or by the hybridization temperature, and are well known in the art. In particular, stringency can be increased by reducing the concentration of salt, by increasing the concentration of formamide, and/or by raising the hybridization temperature.

For example, hybridization under high stringency conditions may employ about 50 % formamide at about 37 °C to 42 °C, whereas hybridization under reduced stringency conditions might employ about 35 % to 25 % formamide at about 30 °C to 35 °C. One particular set of conditions for hybridization under high stringency conditions employs 42 °C, 50 % formamide, 5x SSPE, 0.3 % SDS, and 200 µg/ml sheared and denatured salmon sperm DNA. For hybridization under reduced stringency, similar conditions as described above may be used in 35 % formamide at a reduced temperature of 35 °C. The temperature range corresponding to a particular level of stringency can be further narrowed by calculating the purine to pyrimidine ratio of the nucleic acid of interest and adjusting the temperature accordingly. Variations on the above ranges and conditions are well known in the art. Preferably, hybridization should occur only if there is at least 95 %, and more preferably at least 97 %, identity between the sequences. The polynucleotides which hybridize to the hereinabove described polynucleotides in a preferred embodiment encode polypeptides which exhibit substantially the same biological function or activity as the mature protein of SEQ ID NOs: 30 to 33 and 66, preferably of SEQ ID NOs: 32 and 33.

As mentioned, a suitable polynucleotide probe may have at least 14 bases, preferably 30 bases, and more preferably at least 50 bases, and will hybridize to a polynucleotide of the present invention, which has an identity thereto, as hereinabove described. For example, such polynucleotides may be employed as a probe for hybridizing to the polynucleotides encoding the polypeptides of SEQ ID NOs: 30 to 33 and 66, such as to the polynucleotides of SEQ ID NOs: 34 to 37 and 67, respectively, for example, for recovery of such a polynucleotide, or as a diagnostic probe, or as a PCR primer. Thus, the present invention includes polynucleotides having at least a 70 % identity, preferably at least a 90 % identity, and more preferably at least a 95 % identity to a polynucleotide of SEQ ID NOs: 34 to 37 and 67, which encodes a polypeptide of SEQ ID NOs: 30 to 33 and 66, as well as fragments thereof, which fragments preferably have at least 30 bases and more preferably at least 50 bases.

The terms "homology" or "identity," as used interchangeably herein, refer to sequence similarity between two polynucleotide sequences or between two polypeptide sequences, with identity being a more strict comparison. The phrases "percent identity or homology" and "identity or homology" refer to the percentage of sequence similarity found in a comparison of two or more polynucleotide sequences or two or more polypeptide sequences. "Sequence similarity" refers to the percent similarity in base pair sequence (as determined by any suitable method) between two or more polynucleotide sequences. Two or more sequences can be anywhere from 0-100 % similar, or any integer value there between. Identity or similarity can be determined by comparing a position in each sequence that can be aligned for purposes of comparison. When a position in the compared sequence is occupied by the same nucleotide base or amino acid, then the molecules are identical at that position. A degree of similarity or identity between polynucleotide sequences is a function of the number of identical or matching nucleotides at positions shared by the polynucleotide sequences.

A degree of identity of polypeptide sequences is a function of the number of identical amino acids at positions shared by the polypeptide sequences. A degree of homology or similarity of polypeptide sequences is a function of the number of amino acids at positions shared by the polypeptide sequences. The term "substantially identical," as used herein, refers to an identity or homology of at least 70 %, 75 %, at least 80 %, at least 85 %, at least 90 %, 91 %, 92 %, 93 %, 94 %, 95 %, 96 %, 97 %, 98 %, 99 %, or more.

The degree of sequence identity is determined by choosing one sequence as the query sequence and aligning it with the internet-based tool ClustalW with homologous sequences taken from GenBank using the blastp algorithm (NCBI).

As it is well known in the art, the genetic code is redundant in that certain amino acids are coded for by more than one nucleotide triplet (codon), and the invention includes those polynucleotide sequences which encode the same amino acids using a different codon from that specifically exemplified in the sequences herein. Such a polynucleotide sequence is referred to herein as an "equivalent" polynucleotide sequence. The present invention further includes variants of the hereinabove described polynucleotides which encode for fragments, such as part or all of the protein, analogs and derivatives of a polypeptide of SEQ ID NOs 30 to 33 and 66. The variant forms of the polynucleotide may be a naturally occurring allelic variant of the polynucleotide or a non-naturally occurring variant of the polynucleotide. For example, the variant in the nucleic acid may simply be a difference in codon sequence for the amino acid resulting from the degeneracy of the genetic code, or there may be deletion variants, substitution variants and addition or insertion variants. As known in the art, an allelic variant is an alternative form of a polynucleotide sequence, which may have a substitution, deletion or addition of one or more nucleotides that does not substantially alter the biological function of the encoded polypeptide.

In are more preferred embodiment, the polynucleotide of the invention encodes a complete fusion protein of the invention, more preferably encodes a heterodimer of a DNA recombinase, wherein said heterodimer comprises a first recombinase enzyme, which has an amino acid sequence with at least 70 %, preferably 80 %, more preferably 90 %, sequence identity with a sequence according to SEQ ID NO: 30 or 32 and a second recombinase enzyme, which has the amino acid sequence with at least 70 %, preferably 80 %, more preferably 90 %, sequence identity with a sequence according to SEQ ID NO: 31 or 33 for the recognition of an upstream target sequence and a downstream target sequence of a recombinase target site; wherein said polynucleotide further encodes a linker as described herein.

In one embodiment, the polynucleotide of the invention comprises a nucleic acid of SEQ ID NO 34 and a nucleic acid of SEQ ID NO 35, and a nucleic acid encoding a linker oligopeptide.

In a further embodiment, the polynucleotide of the invention comprises a nucleic acid of SEQ ID NO 36 and a nucleic acid of SEQ ID NO 37, and a nucleic acid encoding a linker oligopeptide.

The nucleic acid encoding the linker oligopeptide is preferably a nucleic acid selected from SEQ ID NOs 38 to 54 (see Table 3 in Example 2)

More preferably, the nucleic acid encoding the linker oligopeptide is preferably a nucleic acid selected from SEQ ID NOs 55 to 63 (see Table 4 in Example 2).

Most preferably, the nucleic acid encoding the a fusion protein of the invention is a nucleic acid comprising
i. nucleic acids with the sequence of SEQ ID NOs 34, 35 and 61; such as the nucleic acid of SEQ ID NO 64; or
ii. nucleic acids with the sequence of SEQ ID NOs 36, 37 and 61; such as the nucleic acid of SEQ ID NO 65; or
iii. nucleic acids with the sequence of SEQ ID NOs 34 and 35; or
iv. nucleic acids with the sequence of SEQ ID NOs 36 and 37; or
v. the nucleic acid of SEQ ID NO: 67.

Most preferably, the nucleic acid encoding a fusion protein of the invention is a nucleic acid comprises or consists of SEQ ID NO 64.

Even most preferably, the nucleic acid encoding a fusion protein of the invention is a nucleic acid comprises or consists of SEQ ID NO 65.

The present invention also includes vectors, which include such polynucleotides, host cells which are genetically engineered with such vectors and the production of the polypeptides of SEQ ID NOs: 30 to 33 and 66, preferably of SEQ ID NOs: 32 and 33 by recombinant techniques using the foregoing. Host cells are genetically engineered (transduced or transformed or transconjugated or transfected) with such vectors, which may be, for example, a cloning vector or an expression vector. The vector may be, for example, in the form of a plasmid, a conjugative plasmid, a viral particle, a phage, etc. The vector or the gene may be integrated into the chromosome at a specific or a not specified site. Methods for genome integration of recombinant DNA, such as homologous recombination or transposase-mediated integration, are well known in the art. The engineered host cells can be cultured in conventional nutrient media modified as appropriate for activating promoters, selecting transformants or amplifying the genes of the present invention. The culture conditions, such as temperature, pH and the like, are those commonly used with the host cell selected for expression, as well known to the ordinarily skilled artisan. The host cell can be a mammalian, insect or bacterial host cell, comprising a nucleic acid or a recombinant polynucleotide molecule or an expression vector described herein.

The polynucleotide sequence in the expression vector is operatively linked to an appropriate expression control sequence(s) (promoter) to direct mRNA synthesis. As representative examples of such promoters, there may be mentioned: LTR or SV40 promoter, the *E. coli* lac, ara, rha or trp, the phage lambda PL promoter and other promoters known to control expression of genes in prokaryotic or eukaryotic cells or their viruses.

One skilled in the art can select a vector based on desired properties, for example, for production of a vector in a particular cell such as a mammalian cell or a bacterial cell.

Any of a variety of inducible promoters or enhancers can be included in the vector for expression of an antibody of the invention or nucleic acid that can be regulated. Such inducible systems, include, for example, tetracycline inducible System; metallothionein promoter induced by heavy metals; insect steroid hormone responsive to ecdysone or related steroids such as muristerone; mouse mammary tumor virus (MMTV) induced by steroids such as glucocorticoid and estrogen; and heat shock promoters inducible by temperature changes; the rat neuron specific enolase gene promoter; the human β-actin gene promoter; the human platelet derived growth factor B (PDGF-B) chain gene promoter; the rat sodium channel gene promoter; the human copper-zinc superoxide dismutase gene promoter; and promoters for members of the mammalian POU-domain regulatory gene family.

Regulatory elements, including promoters or enhancers, can be constitutive or regulated, depending upon the nature of the regulation. The regulatory sequences or regulatory elements are operatively linked to one of the polynucleotide sequences of the invention such that the physical and functional relationship between the polynucleotide sequence and the regulatory sequence allows transcription of the polynucleotide sequence. Vectors useful for expression in eukaryotic cells can include, for example, regulatory elements including the CAG promoter, the SV40 early promoter, the cytomegalovirus (CMV) promoter, the mouse mammary tumor virus (MMTV) steroid-inducible promoter, Pgtf, Moloney marine leukemia virus (MMLV) promoter, thy-1 promoter and the like.

If desired, the vector can contain a selectable marker. As used herein, a "selectable marker" refers to a genetic element that provides a selectable phenotype to a cell in which the selectable marker has been introduced. A selectable marker is generally a gene whose gene product provides resistance to an agent that inhibits cell growth or kills a cell. A variety of selectable markers can be used in the DNA constructs of the invention, including, for example, Neo, Hyg, hisD, Gpt and Ble genes, as described, for example in Ausubel et al., 1999 and U.S. Patent No. 5,981,830. Drugs useful for selecting for the presence of a selectable marker include, for example, G418 for Neo, hygromycin for Hyg, histidinol for hisD, xanthine for Gpt, and bleomycin for Ble. DNA constructs of the invention can incorporate a positive selectable marker, a negative selectable marker, or both.

Various mammalian cell culture systems can also be employed to express a recombinant protein. Examples of mammalian expression systems include the COS-7 lines of monkey kidney fibroblasts. Other cell lines capable of expressing a compatible vector include, for example, the C127, 3T3, CHO, HeLa and BHK cell lines. Mammalian expression vectors will generally comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide required nontranscribed genetic elements.

The polypeptides can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Recovery can be facilitated if the polypeptide is expressed at the surface of the cells, but such is not a prerequisite. Recovery may also be desirable of cleavage products that are cleaved following expression of a longer form of the polypeptide. Protein refolding steps as known in this art can be used, as necessary, to complete configuration of the mature protein. High performance liquid chromatography (HPLC) can be employed for final purification steps.

In accordance with a further embodiment of the invention, there are provided are gene therapy vectors, e.g., for use in systemically or locally increasing the expression of the fusion proteins of the invention in a subject. The gene therapy vectors find use in preventing, mitigating, ameliorating, reducing, inhibiting, and/or treating a disease that can be treated by genome editing, in particular of hemophilia A. The gene therapy vectors typically comprise an expression cassette comprising a polynucleotide encoding a fusion protein of the invention. In one embodiment, the vector is a viral vector. In a preferred embodiment, the viral vector is from a virus selected from the group consisting of adenovirus, retrovirus, lentivirus, herpesvirus and adeno-associated virus (AAV). In a more preferred embodiment, the vector is from one or more of adeno-associated virus (AAV) serotypes 1-11, or any subgroups or any engineered forms thereof. In another embodiment, the viral vector is encapsulated in an anionic liposome.

In another embodiment, the vector is a non-viral vector. In a preferred embodiment, the non-viral vector is selected from the group consisting of naked DNA, a cationic liposome complex, a cationic polymer complex, a cationic liposome-polymer complex, and an exosome.

If the vector is a viral vector, the expression cassette suitably comprises operably linked in the 5' to 3' direction (from the perspective of the mRNA to be transcribed), a first inverse terminal repeat, an enhancer, a promoter, the polynucleotide encoding a fusion protein of the invention, a 3' untranslated region, polyadenylation (polyA) signal, and a second inverse terminal repeat. The promoter is e.g. selected from the group consisting of cytomegalovirus (CMV) promoter and chicken-beta actin (CAG) promoter. The polynucleotide comprises preferably DNA or cDNA or RNA or mRNA. In a preferred embodiment, the polynucleotide encoding a fusion protein of the invention comprises one or more of the polypeptides of SEQ ID NO: 30, SEQ ID NO: 31, SEQ ID NO: 32, SEQ ID NO: 33 and SEQ ID NO: 66. In a most preferred embodiment, the polynucleotide encoding a fusion protein of the invention has at least about 75%, 80%, 85% or 90% sequence identity, e.g. , at least about 91 %, 92%, 93%, 94%, 95%, 96%, 97%, 98%, 99% sequence identity, to one or more of SEQ ID NO: 64, SEQ ID NO: 65 and SEQ ID NO: 67.

The invention further relates to a fusion protein of the invention or a nucleic acid molecule, recombinant polynucleotide or expression vector of the invention for use as a medicament. In a more preferred embodiment, the invention further relates to a fusion protein of the invention or a nucleic acid molecule, recombinant polynucleotide or expression vector of the invention for use in the prevention or treatment of a disease that can be treated by genome editing, in particular hemophilia A.

In a further embodiment, the invention relates to use of a fusion protein of the invention or a nucleic acid molecule, recombinant polynucleotide or expression vector of the invention for the preparation of a medicament for the prevention or treatment of a disease that can be treated by genome editing, in particular hemophilia A.

In a further embodiment, the invention relates to a method of prevention or treatment of a disease that can be treated by genome editing, in particular hemophilia A, comprising the administering a therapeutically effective amount of a fusion protein of the invention or a nucleic acid molecule, recombinant polynucleotide or expression vector of the invention to a patient in need thereof.

The fusion protein of the invention or a nucleic acid molecule, recombinant polynucleotide or expression vector of the invention are particularly suitable for the treatment of severe forms of hemophilia A.

The fusion protein of the invention or a nucleic acid molecule, recombinant polynucleotide or expression vector of the invention can further be comprised in a pharmaceutical composition, which may optionally further contain one or more therapeutically acceptable diluents or carriers.

Provided are pharmaceutical compositions, e.g. , for use in preventing or treating a disorder that can be treated by genome editing, such us hemophilia A, which comprises a therapeutically effective amount of a vector which comprises a nucleic acid sequence of a polynucleotide that encodes one or more fusion proteins according to the invention, or which comprises a therapeutically active amount of a nucleic acid encoding a fusion protein of the invention, or which comprises a therapeutically active amount of a recombinant fusion protein of the invention (together named "therapeutically active agents").

It will be understood that the single dosage or the total daily dosage of the therapeutically active agents and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific nucleic acid or polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range per adult per day. The therapeutically effective amount of the therapeutically active agents, such as a vector according to the invention that should be administered, as well as the dosage for the treatment of a pathological condition with the number of viral or non-viral particles and/or pharmaceutical compositions described herein, will depend on numerous factors, including the age and condition of the patient, the severity of the disturbance or disorder, the method and frequency of administration and the particular peptide to be used.

The pharmaceutical compositions that contain a therapeutically active agent according to the invention may be in any form that is suitable for the selected mode of administration.

In one embodiment, a pharmaceutical composition of the present invention is administered parenterally.

The phrases "parenteral administration" and "administered parenterally" as used herein means modes of administration other than enteral and topical administration, usually by injection, and include epidermal, intravenous, intramuscular, intraarterial, intrathecal, intracapsular, intraorbital, intracardiac, intradermal, intraperitoneal, intratendinous, transtracheal, subcutaneous, subcuticular, intraarticular, subcapsular, subarachnoid, intraspinal, intracranial, intrathoracic, epidural and intrasternal injection and infusion.

The therapeutically active agents of the invention can be administered, as sole active agent, or in combination with other active agents, in a unit administration form, as a mixture with conventional pharmaceutical supports, to animals and human beings.

In further embodiments, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid. It must be stable under the conditions of manufacture and storage and must be preserved against the contaminating action of microorganisms, such as bacteria and fungi.

Solutions comprising the therapeutically active agents as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The therapeutically active agents can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be as solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions can be administered in a manner compatible with the dosage formulation and in such amount as is therapeutically effective. The formulations are easily administered in a variety of dosage forms, such as the type of injectable solutions described above, but drug release capsules and the like can also be employed. Multiple doses can also be administered. As appropriate, the therapeutically active agents described herein may be formulated in any suitable vehicle for delivery. For instance, they may be placed into a pharmaceutically acceptable suspension, solution or emulsion. Suitable mediums include saline and liposomal preparations. More specifically, pharmaceutically acceptable carriers may include sterile aqueous of non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include but are not limited to water, alcoholic/aqueous solutions, emulsions or suspensions, including saline and buffered media. Intravenous vehicles include fluid and nutrient replenishers, electrolyte replenishers (such as those based on Ringer's dextrose), and the like.

Preservatives and other additives may also be present such as, for example, antimicrobials, antioxidants, chelating agents, and inert gases and the like.

A colloidal dispersion system may also be used for targeted gene delivery. Colloidal dispersion systems include macromolecule complexes, nanocapsules, microspheres, beads, and lipid-based systems including oil-in-water emulsions, micelles, mixed micelles, and liposomes.

An appropriate therapeutic regimen can be determined by a physician, and will depend on the age, sex, weight, of the subject, and the stage of the disease. As an example, for delivery of a nucleic acid sequence encoding a fusion protein of the invention using a viral expression vector, each unit dosage of the fusion protein expressing vector may comprise 2.5 µl to 100 µl of a composition including a viral expression vector in a pharmaceutically acceptable fluid at a concentration ranging from 10¹¹ to 10¹⁶ viral genome per ml, for example.

The effective dosages and the dosage regimens for administering a fusion of the invention in the form of a recombinant polypeptide depend on the disease or condition to be treated and may be determined by the persons skilled in the art. An exemplary, non-limiting range for a therapeutically effective amount of a fusion protein of the present invention is about 0.1 -10 mg/kg/body weight, such as about 0.1-5 mg/kg/body weight, for example about 0.1-2 mg/kg/body weight, such as about 0.1-1 mg/kg/body weight, for instance about 0.15, about 0.2, about 0.5, about 1, about 1.5 or about 2 mg/kg/body weight.

A physician or veterinarian having ordinary skill in the art may readily determine and prescribe the effective amount of the pharmaceutical composition required. For example, the physician or veterinarian could start doses of the therapeutically active agents of the invention employed in the pharmaceutical composition at levels lower than that required in order to achieve the desired therapeutic effect and gradually increase the dosage until the desired effect is achieved. In general, a suitable daily dose of a composition of the present invention will be that amount of the delivery system which is the lowest dose effective to produce a therapeutic effect. Such an effective dose will generally depend upon the factors described above. Administration may e.g. be intravenous, intramuscular, intraperitoneal, or subcutaneous, and for instance administered proximal to the site of the target. If desired, the effective daily dose of a pharmaceutical composition may be administered as two, three, four, five, six or more sub-doses administered separately at appropriate intervals throughout the day, optionally, in unit dosage forms. While it is possible for a delivery system of the present invention to be administered alone, it is preferable to administer the delivery system as a pharmaceutical composition as described above.

Further provided are kits comprising a therapeutically active agent as described above and herein. In one embodiment, the kits provide the therapeutically active agents prepared in one or more unitary dosage forms ready for administration to a subject, for example in a preloaded syringe or in an ampoule. In another embodiment, the therapeutically active agents are provided in a lyophilized form.

In a further embodiment, the invention provides a method for determining recombination on genomic level in a host cell culture, comprising a fusion protein for efficient and specific genome editing according to the invention, wherein said method comprises the steps of:
i. providing a nucleic acid molecule encoding a first recombinase enzyme, wherein said first recombinase enzyme has been evolved by directed evolution or rational design to specifically recognize a first half-site of a recombinase target site;
ii. providing a nucleic acid molecule encoding a second recombinase enzyme, wherein said second recombinase enzyme has been evolved by directed evolution or rational design to specifically recognize a second half-site of a recombinase target site;
iii. providing a nucleic acid molecule encoding a linker peptide, wherein said nucleic acid encodes a linker oligopeptide comprising 4 to 60 amino acids;
iv. creating an expression vector by cloning the nucleic acid molecule encoding a first recombinase enzyme, the nucleic acid molecule encoding a second recombinase enzyme and the nucleic acid molecule encoding a linker peptide into an expression vector which further comprises a first reporter gene for expression of a first reporter protein;
v. transfecting a host cell with the expression vector of step iv) and transfecting the host cell with a reporter plasmid comprising a second reporter gene for expressing a second reporter protein;
vi. expressing the fusion protein comprising a first recombinase enzyme, a second recombinase enzyme and a linker peptide, wherein said fusion protein is fused to the first reporter gene; and expressing the second reporter protein;
vii. identifying cells, which show a double expression of the first reporter protein and the second reporter protein, which is indicative for a successful recombination.

A suitable first reporter gene according to step iv. is the gene encoding for EGFP. Consequently, a suitable first reporter protein is EGFP.

A suitable second reporter gene according to step v. is the gene encoding for mCherry. Consequently, a suitable second reporter protein is mCherry.

This system has the advantage that the transfection efficiency of the cells transfected with both expression and reporter plasmid can be measured based on the GFP fluorescence. GFP and mCherry double positive cells reflect recombination of the reporter in human cells. In order to calculate the recombination efficiency of the reporter plasmid in human cells, the double positive cells can be normalized to the transfection efficiency.

The fusion proteins described herein were developed to correct a large gene inversion of exon 1 in the F8 gene which is causing Hemophilia A. To study the inversion efficacy of the heterodimers on genomic level, an *in vitro* recombinase assay as described in example 9 was developed. The inversion efficacy on genomic level in recombinase expressing cells was found to be 20.3% for the non-fused heterodimer and 42.7% for the fused heterodimer. Thus, the fusion of the recombinase heterodimer by a linker of the invention results in two-fold higher inversion rate.

Accordingly, the invention provides in a further embodiment a method for inversion of DNA sequence on genomic level in a cell, comprising a fusion protein or DNA recombinase for efficient and specific genome editing according to the invention, wherein said method comprises the steps of:
i. providing a nucleic acid molecule encoding a first recombinase enzyme, wherein said first recombinase enzyme has been evolved by directed evolution or rational design to specifically recognize a first half-site of a recombinase target site;
ii. providing a nucleic acid molecule encoding a second recombinase enzyme, wherein said second recombinase enzyme has been evolved by directed evolution or rational design to specifically recognize a second half-site of a recombinase target site;
iii. providing a nucleic acid molecule encoding a linker peptide, wherein said nucleic acid encodes a linker oligopeptide comprising 6 to 30 amino acids;
iv. creating an expression vector by cloning the nucleic acid molecule encoding a first recombinase enzyme, the nucleic acid molecule encoding a second recombinase enzyme and the nucleic acid molecule encoding a linker peptide into an expression vector;
v. transfecting a cell, which comprises a DNA sequence, which is to be inverted, with the expression vector of step iv);
vi. expressing the fusion protein comprising a first recombinase enzyme, a second recombinase enzyme and a linker peptide;
vii. inverting of the DNA sequence on a human chromosome in said cell.

Preferably, the fusion protein of step 5 is a fusion protein of the invention as described herein, and more preferably recognizes the first half-site and the second half-site of an upstream target site and a downstream target site of a recombinase, most preferably the upstream target site of SQ ID NO: 17 and the downstream target site of SEQ ID NO: 18 of the *loxF8* recombinase.

In one embodiment, method for inversion of DNA sequence on genomic level is performed in genetically engineered host cell.

In a preferred embodiment, the method for inversion of DNA sequence on genomic level is performed *in vitro* in a human cell derived from a patient, more preferably from a patient suffering from hemophilia A.

In a further preferred embodiment, method for inversion of DNA sequence on genomic level is performed in patients, in particular in patient suffering from hemophilia A *in vivo.*

### Examples of the invention

The following examples are provided for the sole purpose of illustrating various embodiments of the present invention and are not meant to limit the present invention in any fashion.

### Example 1: Substrate-Linked Directed Evolution

Recombinases were evolved using the previously described substrate-linked protein evolution (SLiDE) (Buchholz and Stewart, 2001; Sakara et al., 2007; Karpinski et al., 2016; Lansing, et al., 2019). By varying selection of active and inactive recombinases on the *loxF8* and symmetric sites, respectively, a counterselection strategy was established.

### Example 2: Comparison of recombination activity of F8 recombinase monomer and F8 non-fused recombinase heterodimer

To compare whether the heterodimer recombinase, which consists of the monomers of SEQ ID NOs. 32 and 33, outperforms a single F8 monomer recombinase recognizing the *loxF8* sequence, SLiDE with a single recombinase was performed on the asymmetric loxF8 site, equivalent to the directed evolution of Tre and Brec1 on the asymmetric loxLTR and loxBRT sequences, respectively (Sarkar *et al.,* 2007; Hauber *et al.,* 2013). After 168 rounds of directed evolution, individual recombinases were tested in *E.coli* in the pEVO vector using different arabinose concentrations. The best clone identified (H7, SEQ ID NO: 66) did recombine the *loxF8* sequence, albeit at reduced efficiencies compared to the D7 heterodimer recombinase (SEQ ID NOs. 32 and 33) (Fig. 6).

To investigate the recombination activities of the monomer (H7) and heterodimer (D7) F8-recombinases in mammalian cells, HeLa cells were co-transfected with recombination reporter and recombinase expression plasmids. As in the *E.coli* assays, the H7 recombinase was less efficient than the heterodimer D7 recombinase (Fig. 7; 28% recombination efficiency versus 46% recombination efficiency).

To compare the abilities of the monomer F8-recombinase (H7) and the heterodimer F8 recombinase (D7) to induce the F8 inversion in human cells, the recombinase expression plasmids were transfected into HEK293 cells. 48 hours post transfection genomic DNA was isolated. A PCR reaction was performed to detect the genomic inversion. Again, the monomer H7 recombinase did not perform as well as the heterodimer D7 recombinase (Fig. 8).

To compare the specificity of the H7 monomer with the D7 heterodimer, the recombinases were tested on nine human sequences (SEQ ID NOs: 21 to 23 and 87 to 92) that display the highest similarity to the *loxF8* sequence (SEQ ID NO: 17) (Table 2). These nine off-target sites and the loxF8 on-target site were cloned into the pEVO vectors harboring the respective recombinases and the plasmids were grown for 24 hours in the presence of 10 ug/ml L-arabinose in *E.coli.* DNA extracted from these cultures revealed that the H7 monomer recombined the loxF8 site in addition to the off-target sites HS1, HS2, HS4 and HS8, which have the sequences of SEQ ID NOs: 21, 22, 87 and 91, respectively. In contrast, the D7 heterodimer was much more specific and only slightly recombined HS2, while showing robust activity on the *loxF8* on-target site (Fig. 9). Based on these cumulative results, the heterodimer D7 recombinase was selected for further experiments.

### Example 3: Recombination activity of the fused heterodimer on the symmetric target sites

Different linkers for fusing the F8 recombinase heterodimer were designed and tested. Flexible linkers with sequences (Gly-Gly-Ser)n and number of repeats (n) 2, 4, 6 and 8 were designed. The subsequent 5' and 3' oligonucleotides were synthesized with the addition of the sequences of "sticky ends" produced by XhoI and BsrGI-HF restriction enzymes, annealing of the sequences allowed obtaining the double-stranded DNA fragments that were directly used for cloning.

First, the non-fused heterodimer was tested on the symmetric sites. As expected, the test digest revealed strong recombination activity on the symmetric sites (Figure 10, Figure 12B), which can be explained by the activity of recombinase monomers on each of the symmetric sites. Importantly, however, the single recombinases do not show any activity on the final *loxF8* site by themselves (Figure 12A). On the other hand, the fused S2-(G₂S)₈-S1 heterodimer did not show any activity on the symmetric sites, even at high induction level (200 µg/ml L-arabinose) (Figure 12C).

In order to compare the recombination activity of the fused heterodimer comparing to the non-fused one, both of them were tested at different induction levels. Afterwards, calculations of the recombination efficacy based on the band intensities was performed. Test digests revealed that the fused heterodimer has comparable activity to the non-fused one.

As it was shown from the results of the fusion with (G₂S)₂₋₈ linkers, the length of the linker influences the recombination activity. To study whether an even longer linker can increase the efficiency of the recombination without showing the activity on the symmetric sites, the heterodimer was fused with the linkers of 10, 12, and 14 (Gly-Gly-Ser) repeats. The results showed that increasing the linker length does not have a significant effect on recombination efficiency, however, the heterodimer fused with longer linkers showed activity on the symmetric sites. Therefore, recombinases fused with linkers of 30 or more amino acids result in specificity loss.

### Example 4: Linker design and generation of a linker library

Three linker libraries using degenerate codon RVM, coding for Ala, Arg, Asn, Asp, Glu, Gly, Lys, Ser, Thr amino acids, were designed. Linker length of 24 amino acids was used. The whole sequence (24 amino acids), middle part (12 amino acids) or the edges (6 amino acids from each side) of the (G₂S)₈ linker were changed to the RVM codon. Because it is not known that changes to the linker flexibility can influence the activity of the fused proteins, different options were considered while designing the libraries. The (G2S) sequence with a known flexibility property was kept either further from the recombinases (in the middle of the linker) or closer to the recombinases (on the edges of the linker) to investigate its influence on the activity. The first library was designed in a way that the middle part of the linker sequence (12 amino acids) was kept the same as in the initially used flexible linker, containing four (G2S) repeats, and the right and left parts of the linker sequence (6 amino acids from both sides) were changed to (RVM)6. The second library was designed the other way around: the middle part of the 12 amino acids was changed to (RVM)₁₂, and the right and left parts of 6 amino acids were kept as two (G2S) repeats. In addition, as it was not known if the (G2S) repeats are needed in the linker, in the third library the whole 24 amino acids sequence of the linker was changed to (RVM)₂₄ (Fig. 13). Three oligonucleotides were designed and used as a template for three PCR reaction. The linker sequences were flanked by two adapters for primer annealing, and XhoI and BsrGI-HF restriction sites that allowed to use the linkers directly for cloning. The linker libraries were cloned in the fusion protein instead of (G2S)₈, resulting in three libraries: linker library 1, linker library 2 an linker library 3 (Figure 13) The fusion proteins contained linker libraries, but the sequences of the recombinases were not changed.

Oligonucleotides with degenerate base modification, containing XhoI and BsrGI-HF restriction sites, and an adapter from each end, were synthesized by Biomers.net GmbH. Oligonucleotides sequences are shown in Table 3. The oligonucleotides were used as a template for high-fidelity PCR, the PCR products were subsequently digested with XhoI and BsrGI-HF restriction enzymes, and cloned into pEVO_2rec_link between the S2- and S1-specific recombinases. Freshly prepared competent *E. coli XL-1 blue* were transformed with the plasmids, containing the heterodimer fused with the library of linkers. After the incubation in SOC medium for 1 h, 2 µl (1:500) of the electroporation mixture was plated on an agar plate with chloramphenicol, the rest was added to 100 ml of LB medium with 25 µg/ml of chloramphenicol and desired concentration of L-Arabinose. Both, solid and liquid cultures were incubated at 37°C for 12-16 h, the latter under constant shaking at 200 rpm. The library size was at least 25 000 linker variants that was assessed by the number of colonies on the plate.

**Table 3 - Sequences of the oligonucleotides used for linker generation**

| **Name** | **SEQ ID NO** | **Sequence (5'→3')** |
|---|---|---|
| (Gly₂Ser)₂-F | 38 | TCGAGGGTGGCAGCGGCGGTAGCAT |
| (Gly₂Ser)₂-R | 39 | GTACATGCTACCGCCGCTGCCACCC |
| (Gly₂Ser)₄-F | 40 | TCGAGGGCGGTAGCGGTGGCTCTGGCGGCAGCGGTGGTAG CAT |
| (Gly₂Ser)₄-R | 41 | GTACATGCTACCACCGCTGCCGCCAGAGCCACCGCTACCGC CC |
| (Gly₂Ser)₆-F | 42 | TCGAGGGCGGTAGCGGTGGCTCTGGCGGCAGCGGTGGTAG CGGTGGCTCT GGCGGTAGTAT |
| (Gly₂Ser)₆-R | 43 | TACATACTACCGCCAGAGCCACCGCTACCACCGCTGCCGCC AGAGCCACC GCTACCGCCC |
| (Gly₂Ser)₈-F | 44 | TCGAGGGCGGTAGCGGTGGCTCTGGCGGCAGCGGTGGTAG CGGTGGCTCT GGCGGTAGTGGTGGTAGTGGCGGCA |
| (Gly₂Ser)₈-R | 45 | GTACATGCTGCCGCCACTACCACCACTACCGCCAGAGCCAC CGCTACCAC CGCTGCCGCCAGAGCC |
| (Gly₂Ser)₁₀-F | 46 | TCGAGGGCGGTAGCGGTGGCTCTGGCGGCAGCGGTGGTAG CGGTGGCTCT GGCGGTAGTGGTGGTAGTGGCGGCAGCGGTGGCTCTGGCG GTAGTAT |
| (Gly₂Ser)₁₀-R | 47 | GTACATACTACCGCCAGAGCCACCGCTGCCGCCACTACCAC CACTACCGCCAGAGCCACCGCTACCACCGCTGCCGCCAGA GCCACCGCTACCGCCC |
| (Gly₂Ser)₁₂-F | 48 | TCGAGGGCGGTAGCGGTGGCTCTGGCGGCAGCGGTGGTAG CGGTGGCTCTGGCGGTAGTGGTGGTAGTGGCGGCAGCGGT GGCTCTGGCGGTAGTGGTGGTAGCGGTGGCTCTAT |
| (Gly₂Ser)₁₂-R | 49 | GTACATAGAGCCACCGCTACCACCACTACCGCCAGAGCCA CCGCTGCCGCCACTACCACCACTACCGCCAGAGCCACCGCT ACCACCGCTGCCGCCAGAGCCACCGCTACCGCCC |
| (Gly₂Ser)₁₄-F | 50 | TCGAGGGCGGTAGCGGTGGCTCTGGCGGCAGCGGTGGTAG CGGTGGCTCTGGCGGTAGTGGTGGTAGTGGCGGCAGCGGT GGCTCTGGCGGTAGTGGTGGTAGCGGTGGCTCTGGAGGTA GTGGCGGATCAAT |
| (Gly₂Ser)₁₄-R | 51 | GTACATTGATCCGCCACTACCTCCAGAGCCACCGCTACCAC CACTACCGCCAGAGCCACCGCTGCCGCCACTACCACCACTA CCGCCAGAGCCACCGCTACCACCGCTGCCGCCAGAGCCAC CGCTACCGCCC |
| Linker library 1 | 52 | GAAGTGCCATTCCGCCTGACCTCTCGAGRVMRVMRVMRV MRVMRVMGGCGGCAGCGGTGGTAGCGGTGGCTCTGGCGG TAGTRVMRVMRVMRVMRVMRVMATGTACAAGGCTAGGT GGAGGCTCAGTG |
| Linker library 2 | 53 | GAAGTGCCATTCCGCCTGACCTCTCGAGGGCGGTAGCGGT GGCTCTRVMRVMRVMRVMRVMRVMRVMRVMRVMRVMR VMRVMGGTGGTAGTGGCGGCAGCATGTACAAGGCTAGGT GGAGGCTCAGTG |
| Linker library 3 | 54 | GAAGTGCCATTCCGCCTGACCTCTCGAGRVMRVMRVMRV MRVMRVMRVMRVMRVMRVMRVMRVMRVMRVMRVMRVMRV MRVMRVMRVMRVMRVMRVMRVMRVMATGTACAAGGCT AGGTGGAGGCTCAGTG |

### Example 5: Linker selection

The fusion proteins with linker libraries were subcloned to the pEVO_2rec_loxF8 plasmid in order to test their activity on the *loxF8* target site. Test digest revealed that the heterodimers fused with all three linker libraries have activity on the *loxF8* site at high induction level of 200 µg/ml L-arabinose (Figure 14A).

In order to select for heterodimers fused with linkers that have recombination activity on the *loxF8* site, first, digestion with NdeI and AvrII restriction enzymes was performed. The restriction sites of these enzymes are located between the two *loxF8* sites on the pEVO_2rec_LoxF8 plasmid, therefore, only the plasmids that were not recombined, were linearized. Afterwards, high-fidelity PCR was performed with the forward primer (primer 4) annealing upstream the fusion protein, and the reverse primer (primer 19) annealing downstream the second *loxF8* site. As a result, only the fusion proteins that have recombined the plasmid were amplified by the PCR, because the non-recombined plasmids are digested and amplification is interrupted (Figure 14B).

The obtained PCR product was subsequently digested with SacI-HF and SbfI-HF restriction enzymes and subcloned to the pEVO_2rec_Sym1 plasmid in order to test the activity on the *Sym1* site. Test digest revealed that only one of the libraries, S2-link lib3-S1, had activity on the *Sym1* site at high induction level of 200 µg/ml L-arabinose (Figure 15A). The activity of the heterodimer fused with the linker library 1 and 2 were not detected by the test digest. In order to select the fused heterodimers that do not recombine the *Sym1* site, high-fidelity PCR was performed with the forward primer (primer 20) annealing upstream the fusion protein, and the reverse primer (primer 21) annealing between two *Sym1* sites (Figure 15B). Therefore, only fusion proteins that did not recombine the plasmid were amplified by the PCR. The obtained PCR product was digested with SacI-HF and SbfI-HF restriction enzymes and subcloned to the pEVO_2rec_Sym2 plasmid in order to test the activity on the *Sym2* site. No strong activity was detected by the test digest on the *Sym2* site at high induction level of 200 µg/ml L-arabinose, and the selection was performed the same way as on the *Sym1* site (Figure 15).

To select the linkers with the best properties, the same steps were repeated and the following selection rounds were performed: on *loxF8* at 10 µg/ml L-arabinose, *Sym1* at 200 µg/ml L-arabinose, *Sym2* at 200 µg/ml L-arabinose, *loxF8* at 1 µg/ml L-arabinose, *Sym1* at 200 µg/ml L-arabinose, *Sym2* at 200 µg/ml L-arabinose, *loxF8* at 1 µg/ml L-arabinose. Lower induction levels were used during the selection on the *loxF8* site to select fusion proteins with high activity on the final target site. During the selection on the symmetric sites, however, L-arabinose concentration was kept high at 200 µg/ml to select fusion protein without activity on the symmetric sites. After 10 selection rounds all three fusion proteins with the linker libraries showed activity on the *loxF8* site at low induction level of 1 µg/ml L-arabinose. However, the activity of the libraries on the *Sym1* and *Sym2* sites at high induction level of 200 µg/ml L-arabinose had also increased (Figure 16). The heterodimers fused with all three linker libraries had activity on the *Sym2* site, and the heterodimers fused with the linker library 1 and 3 had activity on the *Sym1* site. The increase of the activity on the symmetric sites is likely due to the accumulation of the active heterodimers in the libraries.

Because the libraries showed activity on the *loxF8* site at the very low induction level, at this point, single clones were analysed. Even though the libraries had some activity on the symmetric sites, it was expected that single linked recombinases from those libraries might only recombine *loxF8* but not *Sym1* or *Sym2.*

After testing the libraries on the *loxF8* site at 1 µg/ml L-arabinose, the digestion with NdeI and AvrII restriction enzymes was performed and the plasmids that were not digested were retransformed. That allowed to enrich the libraries for the fused proteins that are active at low induction level. Then the fusion proteins with linker libraries were again subcloned to the pEVO_2rec_LoxF8 plasmid and grown on a plate. 32 colonies of each library were picked, induced with 10 µg/ml L-arabinose, and tested for recombination using a three-primer PCR assay. 90 clones displayed recombination activity, out of them, 4 colonies of each library were further analyzed for their activity on the *loxF8* on-target (10 µg/ml L-arabinose) and on the *Sym1* and *Sym2* off-targets (200 µg/ml L-arabinose). All of the clones (L1-L12) showed recombination activity on the *loxF8* site, but the efficiency varied between the clones (Figure 17A). Two clones of library 1 and three clones of library 3 had activity on the *Sym1* site at high induction level (Figure 17B). Two clones of library 2 and all four clones of library 3 had measurable activity on the *Sym2* site at high induction level (Figure 17C). It is worth noting, that none of the clones from library 2 showed activity on the symmetric sites. Therefore, it can be assumed that the design of the library 2 was more beneficial to prevent activity on symmetric target sites. The picked clones were sequenced, and sequencing results demonstrated that the library strategy had worked (Table 4).

**Table 4: Sequences of the single clones from the linker libraries**

| **Name** | **Amino acid sequence (5'→3')** | **Nucleic acid sequence (5'→3')** |
|---|---|---|
| L1 (library 1) | AEATSE**GGSGGSGGSGGS**NGARRT SEQ ID NO: 8 | GCCGAAGCAACAAGCGAAGGCGGCAGC GGTGGTAGCGGTGGCTCTGGCGGTAG TAACGGCGCAAGAAGAACC SEQ ID NO: 55 |
| L3 (library 1) | AGTTAR**GGSGGSGGSGGS**GRRGAK SEQ ID NO: 9 | GCAGGAACAACCGCAAGAGGCGGCAGC GGTGGTAGCGGTGGCTCTGGCGGTAG TGGAAGAAGAGGAGCAAAA SEQ ID NO: 56 |
| L4 (library 1) | KNGRGR**GGSGGSGGSGGS**RTKRET SEQ ID NO: 10 | AAAAACGGAAGAGGAAGAGGCGGCAG CGGTGGTAGCGGTGGCTCTGGCGGTA GTAGAACAAAAAGAGAAACA SEQ ID NO: 57 |
| L5 (library 2) | **GGSGGS**TAAKEGAASSAS**GGSGGS** SEQ ID NO: 11 | GGCGGTAGCGGTGGCTCTACCGCCGCC AAAGAAGGAGCAGCAAGCAGCGCCAGC GGTGGTAGTGGCGGCAGC SEQ ID NO: 58 |
| L6 (library 2) | **GGSGGS**NSRSNTENSDKG**GGSGGS** SEQ ID NO: 12 | GGCGGTAGCGGTGGCTCTAACAGCAG AAGCAACACAGAAAACAGCGACAAAGG CGGTGGTAGTGGCGGCAGC SEQ ID NO: 59 |
| L7 (library 2) | **GGSGGS**NGEEGTERGKAT**GGSGGS** SEQ ID NO: 13 | GGCGGTAGCGGTGGCTCTAACGGCGA AGAAGGAACCGAAAGAGGCAAAGCAAC AGGTGGTAGTGGCGGCAGC SEQ ID NO: 60 |
| L8 (library 2) | **GGSGGS**TTKANRAKGGR**GGGSGGS** SEQ ID NO: 14 | GGCGGTAGCGGTGGCTCTACCACCAA AGCAAACAGAGCAAAAGGCGGCAGAGG AGGTGGTAGTGGCGGCAGC SEQ ID NO: 61 |
| L9 (library 3) | GANEDTNTEAAGSEGNEKTGTNSA SEQ ID NO: 15 | GGCGCAAACGAAGACACAAACACAGAA GCAGCCGGAAGCGAAGGCAACGAAAAA ACCGGAACAAACAGCGCT SEQ ID NO: 62 |
| L11 (library 3) | GESRAEDGAKGNGRGKGEATAGAA SEQ ID NO: 16 | GGCGAAAGCAGAGCAGAAGACGGCGCA AAAGGAAACGGCAGAGGCAAAGGAGAA GCAACAGCAGGCGCAGCA SEQ ID NO: 63 |

Based on the recombination efficiency on the *loxF8* site, and the absence of the activity on the symmetric sites, clone L8 (S2-linker L8-S1) was chosen for further analyses.

### Example 6: Recombination activity of recombinases fused with the L8-linker

The recombinase-fusion with the L8-linker was tested on the *loxF8* site at different induction levels in parallel with the (G₂S)₈ linker linker and the non-fused heterodimer. Test digest revealed that the L8-linker fusion recombined the plasmid more efficiently (Figure 18). Likewise, the L8-linker fusion heterodimer also demonstrated higher recombination efficacy in comparison to the non-fused heterodimer (Figure 19). Hence, fusion of the D7 monomers with the L8 linker sequence increases recombination activity.

### Example 7: Off-target activity of the fused heterodimer

A further goal was to find out whether fusion of the recombinase heterodimer increases the specificity of the recombination consequently minimizing potential off-target effects. Based on the remote similarity to the *loxF8* sequence, human sequences that display high similarity to the loxF8 sequence, resembling potential asymmetric and symmetric off-target sites in the for the F8 recombinase heterodimer were tested in *E.coli* excision assays (Table 2, SEQ ID NOs: 19 to 29).

The activity on potential off-target sites was tested at high induction levels in order to identify even weak recombination activity. To account for possible different efficiencies of on-target recombination on the *loxF8,* the concentration of L-arabinose was choosen to be five times higher for the fused L8 heterodimer. Accordingly, L-arabinose concentrations of 50 µg/ml (for the non-fused) and 250 µg/ml (for the L8) were used for testing the heterodimer activity on five asymmetric and four symmetric off-targets.

Test digests revealed that at high induction levels the non-fused heterodimer showed marked recombination activity on one of the five tested asymmetric off-targets (2LR site, SEQ ID NO: 22 site). In contrast, the fused L8-heterodimer did not show obvious recombination activity on any the asymmetric off-target sites (Figure 20).

On the symmetric off-target sites, the non-fused heterodimer showed noticeable activity on one of the tested sites (2L site, SEQ ID NO: 27 site). The fused heterodimer, on the contrary, did not display any activity on this, or any other of the tested sites (Figure 20). The results indicate that fusion of the heterodimer markedly increases recombination specificity of designer-SSRs.

### Example 8: Recombination activity in human cells

The fused L8-recombinase heterodimer has shown good activity in bacteria; therefore, the next step was to investigate the recombination efficacy in human cells and compare activities with the non-fused heterodimer. Transfection of HEK293T cells with an expression plasmid containing the fused or single recombinases transcriptionally fused with EGFP was performed (Figure 21). The cells transfected with the empty plasmids expressing only EGFP and non-transfected cells were used as positive and negative control of the transfection, respectively.

Together with the recombinase expression plasmids, the cells were also transfected with a reporter plasmid, coding for mCherry with an upstream stop codon located between two *loxF8* target sites. Upon recombination the reporter plasmid will excise the stop cassette, thereby allowing the expression of mCherry, which can be measured by flow cytometry analysis (Figure 22).

Flow cytometry analysis performed 48 h after transfection revealed that the transfection efficiency of the cells transfected with both expression and reporter plasmids was 28% for the non-fused heterodimer, and 21% for the fused heterodimer, respectively. GFP and mCherry double positive cells reflect recombination of the reporter in human cells. In order to calculate the recombination efficiency of the reporter plasmid in human cells, the double positive cells were normalized to the transfection efficiency (Figure 23). Accordingly, in 76.4% (non-fused) or 92.8% (fused) of the cells the reporters were recombined, indicating that the fused L8 recombinase has higher recombination efficiency in this assay.

### Example 9: Inversion efficacy on genomic level in human cells

The here described recombinases were developed to correct a large gene inversion of exon 1 in the F8 gene which is causing Hemophilia A. To study the inversion efficacy of the heterodimers on genomic level, recombinases were expressed utilizing the described expression constructs (Fig. 21) for 48 h in HEK293T cells and gDNA was extracted subsequently. HEK293T cells do not carry the inverted exon 1 of the F8 gene. However, recombinases can carry out the inversion reaction independent on the orientation of the genomic DNA fragment between the *loxF8* sites. Therefore, the ratio of the inverted to the non-inverted exon 1 can be used as a proxy for the inversion efficiency. A qPCR-based assay with TaqMan probes specific for the exon 1 inversion was developed to calculate the inversion frequency on genomic DNA. gDNA samples with defined amounts of exon 1 inversion were used to calculate a standard curve. The inversion efficiency of the recombinase transfected cells can be deduced afterwards using this standard curve. gDNA isolated from the samples transfected with the non-fused heterodimer showed that the inversion was induced in around 6,0% of cells. For samples transfected with the fused heterodimer the inversion frequency was around 9.0% (Figure 24). However, since the transfection efficiency is not 100% the inversion rate should be corrected for GFP+ cells because GFP expression is transcriptionally linked to recombinase expression. Flow cytometry data revealed transfection efficacies of 29.5% (non-fused) and 21,3% (fused), respectively. Hence, the inversion efficacy on genomic level in recombinase expressing cells was 20.3% for the non-fused heterodimer and 42.7% for the fused L8 heterodimer. Thus, fusion of the L8 recombinase heterodimer resulted in two-fold higher inversion rate, demonstrating that fusion of recombinase heterodimers increases recombination activity.

### References

Abi-Ghanem, J., Chusainow, J., Karimova, M., Spiegel, C., Hofmann-Sieber, H., Hauber, J., Buchholz, F., Pisabarro, M.T. (2013). Engineering of a target site-specific recombinase by a combined evolution- and structure-guided approach. Nucleic Acids Research, 41: 2394-2403.
Albanese, C., Hulit, J., Sakamaki, T., Pestell, R.G. (2002). Recent advances in inducible expression in transgenic mice. Seminars in Cell and Developmental Biology, 13: 129-141.
Ausubel et al., Current Protocols in Molecular Biology (Supplement 47), John Wiley & Sons, New York (1999).
Buchholz, F., Stewart, A.F. (2001). Alteration of Cre recombinase site specificity by substrate-linked protein evolution. Nature Biotechnology, 19: 1047-1052.
Carcao, M. (2014). Changing paradigm of prophylaxis with longer acting factor concentrates. Haemophilia, 20: 99-105.
Carroll, D. (2014). Genome engineering with targetable nucleases. Annual Review of Biochemistry, 83: 409-439.
Castaldo, G., D'Argenio, V., Nardiello, P., Zarrilli, F., Sanna, V., Rocino, A., Coppola, A., Di Minno, G., Salvatore, F. (2007). Haemophilia A: molecular insights. Clinical chemistry and laboratory medicine, 45: 450-461.
Chen, X., Zaro, J.L., Shen, W.C. (2013). Fusion protein linkers: property, design and functionality. Advanced Drug Delivery Reviews, 65: 1357-1369.
Cox, D.B., Platt, R.J., Zhang, F. (2015). Therapeutic Genome Editing: Prospects and Challenges. Nature Medicine 21: 121-131.
Cyranoski, D. (2016). CRISPR gene-editing tested in a person for the first time. Nature, 539: 479.
Dahlback, B. (2000). Blood coagulation. The Lancet, 355: 1627-1632.
Eroshenko, N., Church, G.M. (2013). Mutants of Cre recombinase with improved accuracy. Nature Communications, 4: 2509.
Evan, G.I., Lewis, G.K., Ramsay, G., Bishop, J.M. (1985). Molecular and Cell Biology, 5: 3610-3616.
Fischer, K., Bom, J.G., Mauser-Bunschoten, E.P., Roosendaal, G., Berg, H.M. (2005). Effects of haemophilic arthropathy on health-related quality of life and socio-economic parameters. Haemophilia, 11: 43-48.
Gelato, K.A., Martin, S.S., Liu, P.H., Saunders, A.A., Baldwin, E.P. (2008). Spatially-Directed Assembly of a Heterotetrameric Cre-Lox Synapse Restricts Recombination Specificity. Journal of Molecular Biology, 378: 653-665.
George, L.A., Ragni, M.V., Samelson-Jones, B.J., Cuker, A., Runoski, A.R., Cole, G., Wright, F., Chen, Y., Hui, D.J., Wachtel, K., Takefman, D., Couto, L.B., Reape, K.Z., Carr, M.E., Anguela, X.M., High, K.A. (2017). Spk-8011: preliminary results from a phase 1/2 dose escalation trial of an investigational AAV-mediated gene therapy for hemophilia A. Blood, 130: 604.
Gouw, S., van der Bom, J.G., Ljung, R., Escuriola, C., Cid, A.R., Claeyssens-Donadel, S., van Geet, C., Kenet, G., Mäkipernaa, A., Molinari, A.C., Muntean, W., Kobelt, R., Rivard, G., Santagostino, E., Thomas, A., van den Berg, H.M., PedNet and RODIN Study Group. (2013). Factor VIII Products and Inhibitor Development in Severe Hemophilia A. The New England Journal of Medicine, 368: 231-239.
Graw, J., Brackmann, H.H., Oldenburg, J., Schneppenheim, R., Spannagl, M., Schwaab, R. (2005). Haemophilia A: from mutation analysis to new therapies. Nature Reviews Genetics, 6: 488-501.
Hauber, I., Hofmann-Sieber, H., Chemnitz, J., Dubrau, D., Chusainow, J., Stucka, R., Hartjen, P., Schambach, A., Ziegler, P., Hackmann, K., Schröck, E., Schumacher, U., Lindner, C., Grundhoff, A., Baum, C., Manz, M.G., Buchholz, F., Hauber, J. (2013). Highly significant antiviral activity of HIV-1 LTR-specific tre-recombinase in humanized mice. PLoS Pathogens, 9: e1003587.
Justice, M.J., Siracusa, L.D., Stewart, A.F. (2011). Technical approaches for mouse models of human disease. Disease Model and Mechanisms, 4: 305-310.
Karimova, M., Splith, V., Karpinski, J., Pisabarro, M.T., Buchholz, F. (2016). Discovery of Nigri/nox and Panto/pox site-specific recombinase systems facilitates advanced genome engineering. Scientific Reports, 6: 30130.
Karpinski, J., Hauber, I., Chemnitz, J., Schäfer, C., Paszkowski-Rogacz, M., Chakraborty, D., Beschorner, N., Hofmann-Sieber, H., Lange, U.C., Grundhoff, A., Hackmann, K., Schrock, E., Abi-Ghanem, J., Pisabarro, M.T., Surendranath, V., Schambach, A., Lindner, C., van Lunzen, J., Hauber, J., Buchholz, F. (2016). Directed evolution of a recombinase that excises the provirus of most HIV-1 primary isolates with high specificity. Nature Biotechnology, 34: 401-409.
Konkle, B., Stasyshyn, O., Chowdary, P., Bevan, D.H., Mant, T., Shima, M., Engl, W., Dyck-Jones, J., Fuerlinger, M., Patrone, L., Ewenstein, B., Abbuehl, B. (2015). Pegylated, full-length, recombinant factor VIII for prophylactic and on-demand treatment of severe hemophilia A. Blood, 126: 1078-1085.
Kosicki, M., Tomberg, K., Bradley, A. (2018). Repair of double-strand breaks induced by CRISPR-Cas9 leads to large deletions and complex rearrangements. Nature Biotechnology, 36: 765-771.
Lansing, F., Paszkowski-Rogacz, M., Schmitt, L.T., Schneider M.P., Rojo Romanos, T., Sonntag, J., Buchholz, F. (2019). A heterodimer of evolved designer-recombinases precisely excises a human genomic DNA locus. Nucleic Acids Research*.* gkz1078
Lee, H.J., Kweon, J., Kim, E., Kim, S., Kim, J.S. (2012). Targeted chromosomal duplications and inversions in the human genome using zinc finger nucleases. Genome Research, 22: 539-548.
Lenting, P., Denis, C.V., Christophe, O.D. (2017). Emicizumab, a bispecific antibody recognizing coagulation factors IX and X: how does it actually compare to factor VIII? Blood, 130: 2463-2468.
Mahlangu, J., Powell, J.S., Ragni, M.V., Chowdary, P., Josephson, N.C., Pabinger, I., Hanabusa, H., Gupta, N., Kulkarni, R., Fogarty, P., Perry, D., Shapiro, A., Pasi, K.J., Apte, S., Nestorov, I., Jiang, H., Li, S., Neelakantan, S., Cristiano, L.M., Goyal, J., Sommer, J.M., Dumont, J.A., Dodd, N., Nugent, K., Vigliani, G., Luk, A., Brennan, A., Pierce, G.F., A-LONG Investigators. (2014). Phase 3 study of recombinant factor VIII Fc fusion protein in severe hemophilia A. Blood, 123: 317-325.
Meinke, G., Bohm, A., Hauber, J., Pisabarro, M.T., Buchholz, F. (2016). Cre recombinase and other tyrosine recombinases. Chemical reviews, 116: 12785-12820.
Melchiorre, D., Manetti, M., Matucci-Cerinic, M. (2017). Pathophysiology of Hemophilic Arthropathy. Journal of clinical medicine, 6: 63.
O'Hara, J., Hughes, D., Camp, C., Burke, T., Carroll, L., Diego, D.G. (2017). The cost of severe haemophilia in Europe: the CHESS study. Orphanet journal of rare diseases, 12: 106.
Pandey G., Mittal B. (2001). Molecular diagnosis in haemophilia A. Journal of Postgraduate Medicine, 47: 274.
Park, C.Y., Kim, J., Kweon, J., Son, J.S., Lee, J.S., Yoo, J.E., Cho, S.R., Kim, J.H., Kim, J.S., Kim, D.W. (2014). Targeted inversion and reversion of the blood coagulation factor 8 gene in human iPS cells using TALENs. roceedings of the National Academy of Sciences USA, 111: 9253-9258.
Park, C.Y., Kim, D.H., Son, J.S., Sung, J.J., Lee, J., Bae, S., Kim, J.H., Kim, D.W., Kim, J.S. (2015). Functional Correction of Large Factor VIII Gene Chromosomal Inversions in Hemophilia A Patient-Derived iPSCs Using CRISPR-Cas9. Cell Stem Cell, 17: 213-220.
Pasi, K.J., Rangarajan, S., Kim, B., Lester, W., Perry, D., Madan, B., Tavakkoli, F., Yang, K., Pierce, G.F., Wong, W.Y. (2017). Achievement of normal circulating factor VIII activity following Bmn 270 AAV5-FVIII gene transfer: interim, long-term efficacy and safety results from a phase 1/2 study in patients with severe hemophilia A. Blood, 130: 603.
Peters, R., Harris, T. (2018). Advances and innovations in haemophilia treatment. Nature Review Drug Discovery, 17: 493-508.
Peyvandi F., Garagiola, I., Young, G. (2016). The past and future of haemophilia: diagnosis, treatments, and its complications. Lancet, 388: 187-97.
Qasim, W., Amrolia, P.J., Samarasinghe, S., Ghorashian, S., Zhan, H., Stafford, S., Butler, K., Ahsan, G., Gilmour, K., Adams, S., Pinner, D., Chiesa, R., Chatters, S., Swift, S., Goulden, N., Peggs, K., Thrasher, A.J., Veys, P., & Pule, M. (2015). First Clinical Application of Talen Engineered Universal CAR19 T Cells in B-ALL. Blood, 126: 2046.
Santoro, S.W., Schultz, P.G. (2002). Directed evolution of the site specificity of Cre recombinase. Proceedings of the National Academy of Sciences, 99: 4185-4190.
Saraf-Levy, T., Santoro, S.W., Volpin, H., Kushnirsky, T., Eyal, Y., Schultz, P.G., Gidoni, D., Carmi, N. (2006). Site-specific recombination of asymmetric lox sites mediated by a heterotetrameric Cre recombinase complex. Bioorganic & Medicinal Chemistry, 14: 3081-3089.
Sarkar, I., Hauber, I., Hauber, J., Buchholz, F. (2007). HIV-1 proviral DNA excision using an evolved recombinase. Science, 316, 1912-1915.
Shahani, T., Covens, K., Lavend'homme, R., Jazouli, N., Sokal, E., Peerlinck, K., Jacquemin, M. (2014). Human liver sinusoidal endothelial cells but not hepatocytes contain factor VIII. Journal of Thrombosis and Haemostasis, 12: 36-42.
Tebas, P., Stein, D., Tang, W., Frank, I., Wang, S.Q., Lee, G., Spratt, S.K., Surosky, R.T., Giedlin, M.A., Nichol, G., Holmes, M.C., Gregory, P.D., Ando, D.G., Kalos, M., Collman, R.G., Binder-Scholl, G., Plesa, G., Hwang, W.T., Levine, B.L., June, C.H. (2014). Gene editing of CCR5 in autologous CD4 T cells of persons infected with HIV. The New England Journal of Medicine, 370: 901-910.
Tizzano E.F., Cornet, M., Baiget, M. (2003). Inversion of intron 1 of the factor VIII gene for direct molecular diagnosis hemophilia A. Haematologica, 88:118-20.
Turner, N., Moake, J. (2000). Factor VIII Is Synthesized in Human Endothelial Cells, Packaged in Weibel-Palade Bodies and Secreted Bound to ULVWF Strings. PLoS One, 10.
Vandamme, C., Adjali, O., Mingozzi, F. (2017). Unraveling the complex story of immune responses to AAV vectors trial after trial. Human Gene Therapy, 28: 1061-1074.
Wang, M., Glass, Z.A., Xu, Q. (2017). Non-viral delivery of genome-editing nucleases for gene therapy. Gene Therapy, 24: 144-150.
White, G., Rosendaal, F., Aledort, L.M., Lusher, J.M., Rothschild, C., Ingerslev, J., Factor VIII and Factor IX Subcommittee. (2001). Recommendation of the scientific subcommittee on factor VIII and factor IX of the scientific and standardization committee of the International Society on Thrombosis and Haemostasis. Thrombosis and Haemostasis, 85: 560.
Wilson, I.A., Niman, H.L., Houghten, R.A., Cherenson, A.R., Connolly, M.L., Lerner, R.A. (1984). The structure of an antigenic determinant in a protein. Cell, 37: 767-778.
Yu, Y., Bradley, A. (2002). Engineering chromosomal rearrangements in mice. Nature Review Genetics, 2: 780-790.
Zhang, C., Myers, C.A., Qi, Z., Mitra, R.D., Corbo, J.C., Havranek, J.J. (2015). Redesign of the monomer-monomer interface of Cre recombinase yields an obligate heterotetrameric complex. Nucleic Acids Research, 43: 9076-9085.

## Claims

1. A fusion protein for efficient and specific genome editing, comprising a complex of recombinases, wherein said complex comprises at least a first recombinase enzyme, a second recombinase enzyme and at least one linker, wherein said first recombinase enzyme and said second recombinase enzyme specifically recognize a first half-site and a second half-site of an upstream target site and/or a downstream target site of a recombinase; wherein said first recombinase enzyme and said second recombinase enzyme are interconnected via a linker; and wherein said linker comprises or consists of an oligopeptide comprising 4 to 50 amino acids.

2. The fusion protein of claim 1, wherein said linker
• is an oligopeptide selected from the group consisting of
| | |
|---|---|
| GGSGGS | (SEQ ID NO: 1); |
| GGSGGSGGSGGS | (SEQ ID NO: 2); |
| GGSGGSGGSGGSGGSGGS | (SEQ ID NO: 3); |
| GGSGGSGGSGGSGGSGGSGGSGGS | (SEQ ID NO: 4); |
| GGSGGSGGSGGSGGSGGSGGSGGSGGSGGS | (SEQ ID NO: 5); |
| GGSGGSGGSGGSGGSGGSGGSGGSGGSGGSGGSGGS | (SEQ ID NO: 6); |
| and | |
| GGSGGSGGSGGSGGSGGSGGSGGSGGSGGSGGSGGSGGSGGS | (SEQ ID NO: 7). |
• or consists of an oligopeptide of 24 amino acids;
• comprises or consists of an oligopeptide with an amino acid sequence selected from formula 1, formula 2 and formula 3:
X₁-X₂-X₃-X₄-X₅-X₆-(G₂S)₄-X₇-X₈-X₉-X₁₀-X₁₁-X₁₂ (formula 1);
(G₂S)₂-X₁-X₂-X₃-X₄-X₅-X₆-X₇-X₈-X₉-X₁₀-X₁₁-X₁₂-(G₂S)₂ (formula 2);
and 
X1– X2– X3– X4– X5–X6– X7– X8– X9– X10– X11–X12- X13– X14– X15– X16– X17–X18– X19–
X20– X21– X22– X23–X24 (formula 3);
 wherein
G is glycine;
S is serine; and
each of X₁ to X₂₄ is independently selected from the group consisting of alanine, arginine, asparagine, aspartic acid, glutamine, glycine, lysine, serine and threonine;
• or comprises or consists of an oligopeptide selected from the group consisting of
| | |
|---|---|
| AEATSEGGSGGSGGSGGSNGARRT | (SEQ ID NO: 8); |
| AGTTARGGSGGSGGSGGSGRRGAK | (SEQ ID NO: 9); |
| KNGRGRGGSGGSGGSGGSRTKRET | (SEQ ID NO: 10); |
| GGSGGSTAAKEGAASSASGGSGGS | (SEQ ID NO: 11); |
| GGSGGSNSRSNTENSDKGGGSGGS | (SEQ ID NO: 12); |
| GGSGGSNGEEGTERGKATGGSGGS | (SEQ ID NO: 13); |
| GGSGGSTTKANRAKGGRGGGSGGS | (SEQ ID NO: 14); |
| GANEDTNTEAAGSEGNEKTGTNSA | (SEQ ID NO: 15); and |
| GESRAEDGAKGNGRGKGEATAGAA | (SEQ ID NO: 16). |

3. The fusion protein according to any one of the preceding claims, wherein in said fusion protein, the first recombinase enzyme, the second recombinase enzyme and the linker are interconnected such that the C-terminus of the second recombinase enzyme, which specifically recognizes a second half-site of a recombinase target site, is fused with the linker to the N-terminus of the first recombinase enzyme, which specifically recognizes a first half-site of a recombinase target site.

4. The fusion protein according to any one of the preceding claims, wherein each recombinase enzyme of said heterodimer is a tyrosine site-specific recombinase, which have been evolved by directed evolution to independently recognize a first half-site and a second-half site of a recombinase target site of tyrosine site-specific recombinase.

5. The fusion protein according to claim 4, wherein said tyrosine site-specific recombinase is selected from the group consisting of Cre-, Dre-, VCre-, SCre-, Vika-, lambda-Int-, Flp-, R-, Kw-, Kd-, B2-, B3-, Nigri- and Panto-recombinase.

6. The fusion protein according to any one of the preceding claims, wherein said first recombinase enzyme is a protein, which has an amino acid sequence with at least 70 %, preferably 80 %, more preferably 90 %, sequence identity with a sequence according to SEQ ID NO: 30 or SEQ ID NO: 32 and/or wherein said second recombinase enzyme is a protein, which has an amino acid sequence with at least 70 %, preferably 80 %, more preferably 90 %, sequence identity with a sequence according to SEQ ID NO: 31 or SEQ ID NO: 33;
or wherein said fusion protein has an amino acid sequence with at least 70 %, preferably 80 %, more preferably 90 %, sequence identity with a sequence according to SEQ ID NO: 71 or SEQ ID NO: 72, wherein SEQ ID NO: 71 or SEQ ID NO: 72 include a linker with the amino acid sequence of SEQ ID NO: 14.

7. A DNA recombinase consisting of a heterodimer, wherein said heterodimer comprises or consists of a first recombinase enzyme and a second recombinase enzyme, wherein said first recombinase enzyme and said second recombinase enzyme specifically recognize a first half-site and a second half-site of an upstream target site and/or a downstream target site of a recombinase, wherein said first recombinase enzyme is a polypetide, which has an amino acid sequence with at least 70 %, preferably 80 %, more preferably 90 %, sequence identity with a sequence according to SEQ ID NO: 30 or SEQ ID NO: 32 and/or wherein said second recombinase enzyme is a polypeptide, which has an amino acid sequence with at least 70 %, preferably 80 %, more preferably 90 %, sequence identity with a sequence according to SEQ ID NO: 31 or SEQ ID NO: 33;
or a DNA recombinase monomer, which specifically recognizes a first half-site and a second half-site of an upstream target site and/or a downstream target site of a recombinase, wherein said DNA recombinase monomer has an amino acid sequence with at least 70 %, preferably 80 %, more preferably 90 %, sequence identity with a sequence according to SEQ ID NO: 66.

8. A fusion protein, DNA recombinase heterodimer or DNA recombinase monomer according to any one of the preceding claims, wherein said fusion protein, DNA recombinase heterodimer or DNA recombinase monomer specifically recognizes the upstream recombinase target sequence of the *loxF8* target site, which has the nucleic acid sequence
ATAAATCTGTGGAAACGCTGCCACACAATCTTAG (SEQ ID NO: 17);
and recognizes the downstream recombinase target sequence of the *loxF8* target site, which has the nucleic acid sequence
CTAAGATTGTGTGGCAGCGTTTCCACAGATTTAT (SEQ ID NO: 18);
and which catalyze the inversion of a gene sequence between the upstream recombinase target sequence of SEQ ID NO. 17 and the downstream recombinase target sequence of SEQ ID NO: 18 of the *loxF8* recombinase target site.

9. A nucleic acid molecule encoding a fusion protein or a DNA recombinase according to any one of claims 1 to 8.

10. The nucleic acid molecule of claim 9, which comprises or consists of
i. nucleic acids with the sequence of SEQ ID NOs 34, 35 and 61; such as the nucleic acid of SEQ ID NO 64; or
ii. nucleic acids with the sequence of SEQ ID NOs 36 37 and 61; such as the nucleic acid of SEQ ID NO 65; or
iii. nucleic acids with the sequence of SEQ ID NOs 34 and 36; or
iv. nucleic acids with the sequence of SEQ ID NOs 35 and 37; or
v. the nucleic acid of SEQ ID NO: 67.

11. A recombinant polynucleotide molecule comprising the nucleic acid molecule according to claim 10 or 11 plus expression-controlling elements operably linked with said nucleic acid to drive expression thereof.

12. A mammalian, insect or bacterial host cell comprising a nucleic acid molecule according to claim 9 or 10 or a recombinant polynucleotide molecule of claim 11 or an expression vector comprising the nucleic acid molecule of claim 9 or 10.

13. A pharmaceutical composition comprising a fusion protein or a DNA recombinase according to according to any one of claims 1 to 9 or a nucleic acid molecule or recombinant polynucleotide according to any one of claims 9 to 11 optionally in combination with one or more therapeutically acceptable diluents or carriers.

14. A fusion protein or a DNA recombinase according to any one of claims 1 to 8 or a nucleic acid molecule or recombinant polynucleotide according to any one of claims 9 to 11 or a pharmaceutical composition according to claim 13 for use in the treatment of hemophilia A, in particular severe hemophilia A.

15. A method for inversion of a DNA sequence on genomic level in a cell *in vitro,* comprising a fusion protein or DNA recombinase for efficient and specific genome editing according to the invention, wherein said method comprises the steps of:
i. providing a nucleic acid molecule encoding a first recombinase enzyme, wherein said first recombinase enzyme has been evolved by directed evolution or rational design to specifically recognize a first half-site of a recombinase target site;
ii. providing a nucleic acid molecule encoding a second recombinase enzyme, wherein said second recombinase enzyme has been evolved by directed evolution or rational design to specifically recognize a second half-site of a recombinase target site;
iii. providing a nucleic acid molecule encoding a linker peptide, wherein said nucleic acid encodes a linker oligopeptide comprising 6 to 30 amino acids;
iv. creating an expression vector by cloning the nucleic acid molecule encoding a first recombinase enzyme, the nucleic acid molecule encoding a second recombinase enzyme and the nucleic acid molecule encoding a linker peptide into an expression vector;
v. transfecting a cell, which comprises a DNA sequence, which is to be inverted, with the expression vector of step iv);
vi. expressing the fusion protein comprising a first recombinase enzyme, a second recombinase enzyme and a linker peptide;
vii. inversion of the DNA sequence on a human chromosome in said cell.

16. A *loxF8* recombinase target site comprising a 5' target sequence of SEQ ID NO: 17 and a 3' target sequence of SEQ ID NO: 18


SEQUENCE LISTING

<110>  Technische Universität Dresden

<120>  Fusion of site-specific recombinases for efficient and specific
       genome editing

<130>  TUD131EP

<160>  92

<170>  PatentIn version 3.5

<210>  1
<211>  6
<212>  PRT
<213>  Artificial sequence

<220>
<223>  Synthetic peptide

<400>  1

Gly Gly Ser Gly Gly Ser
1               5


<210>  2
<211>  12
<212>  PRT
<213>  Artificial sequence

<220>
<223>  Synthetic peptide

<400>  2

Gly Gly Ser Gly Gly Ser Gly Gly Ser Gly Gly Ser
1               5                   10


<210>  3
<211>  18
<212>  PRT
<213>  Artificial sequence

<220>
<223>  Synthetic peptide

<400>  3

Gly Gly Ser Gly Gly Ser Gly Gly Ser Gly Gly Ser Gly Gly Ser Gly
1               5                   10                  15


Gly Ser



<210>  4
<211>  24
<212>  PRT
<213>  Artificial sequence

<220>
<223>  Synthetic peptide

<400>  4

Gly Gly Ser Gly Gly Ser Gly Gly Ser Gly Gly Ser Gly Gly Ser Gly
1               5                   10                  15

Gly Ser Gly Gly Ser Gly Gly Ser
            20

<210>  5
<211>  30
<212>  PRT
<213>  Artificial Sequence

<220>
<223>  Synthetic peptide

<400>  5

Gly Gly Ser Gly Gly Ser Gly Gly Ser Gly Gly Ser Gly Gly Ser Gly
1               5                   10                  15

Gly Ser Gly Gly Ser Gly Gly Ser Gly Gly Ser Gly Gly Ser
            20                  25                  30

<210>  6
<211>  36
<212>  PRT
<213>  Artificial Sequence

<220>
<223>  Synthetic peptide

<400>  6

Gly Gly Ser Gly Gly Ser Gly Gly Ser Gly Gly Ser Gly Gly Ser Gly
1               5                   10                  15

Gly Ser Gly Gly Ser Gly Gly Ser Gly Gly Ser Gly Gly Ser Gly Gly
            20                  25                  30

Ser Gly Gly Ser
        35

<210>  7
<211>  42
<212>  PRT
<213>  Artificial Sequence

<220>
<223>  Synthetic peptide

<400>  7

Gly Gly Ser Gly Gly Ser Gly Gly Ser Gly Gly Ser Gly Gly Ser Gly
1               5                   10                  15

Gly Ser Gly Gly Ser Gly Gly Ser Gly Gly Ser Gly Gly Ser Gly Gly
            20                  25                  30

Ser Gly Gly Ser Gly Gly Ser Gly Gly Ser
        35                  40

<210>  8
<211>  24
<212>  PRT
<213>  Artificial Sequence

<220>
<223>  Synthetic peptide

<400>  8

Ala Glu Ala Thr Ser Glu Gly Gly Ser Gly Gly Ser Gly Gly Ser Gly
1               5                   10                  15

Gly Ser Asn Gly Ala Arg Arg Thr
            20

<210>  9
<211>  24
<212>  PRT
<213>  Artificial Sequence

<220>
<223>  Snthetic peptide

<400>  9

Ala Gly Thr Thr Ala Arg Gly Gly Ser Gly Gly Ser Gly Gly Ser Gly
1               5                   10                  15

Gly Ser Gly Arg Arg Gly Ala Lys
            20

<210>  10
<211>  24
<212>  PRT
<213>  Artificial Sequence

<220>
<223>  Synthetic peptide

<400>  10

Lys Asn Gly Arg Gly Arg Gly Gly Ser Gly Gly Ser Gly Gly Ser Gly
1               5                   10                  15

Gly Ser Arg Thr Lys Arg Glu Thr
            20

<210>  11
<211>  24
<212>  PRT
<213>  Artificial Sequence

<220>
<223>  Synthetic peptide

<400>  11

Gly Gly Ser Gly Gly Ser Thr Ala Ala Lys Glu Gly Ala Ala Ser Ser
1               5                   10                  15

Ala Ser Gly Gly Ser Gly Gly Ser
            20

<210>  12
<211>  24
<212>  PRT
<213>  Artificial Sequence

<220>
<223>  Synthetic peptide

<400>  12

Gly Gly Ser Gly Gly Ser Asn Ser Arg Ser Asn Thr Glu Asn Ser Asp
1               5                   10                  15

Lys Gly Gly Gly Ser Gly Gly Ser
            20

<210>  13
<211>  24
<212>  PRT
<213>  Artificial Sequence

<220>
<223>  Synthetic peptide

<400>  13

Gly Gly Ser Gly Gly Ser Asn Gly Glu Glu Gly Thr Glu Arg Gly Lys
1               5                   10                  15

Ala Thr Gly Gly Ser Gly Gly Ser
            20

<210>  14
<211>  24
<212>  PRT
<213>  Artificial Sequence

<220>
<223>  Synthetic peptide

<400>  14

Gly Gly Ser Gly Gly Ser Thr Thr Lys Ala Asn Arg Ala Lys Gly Gly
1               5                   10                  15

Arg Gly Gly Gly Ser Gly Gly Ser
            20

<210>  15
<211>  24
<212>  PRT
<213>  Artificial Sequence

<220>
<223>  Synthetic peptide

<400>  15

Gly Ala Asn Glu Asp Thr Asn Thr Glu Ala Ala Gly Ser Glu Gly Asn
1               5                   10                  15

Glu Lys Thr Gly Thr Asn Ser Ala
            20

<210>  16
<211>  24
<212>  PRT
<213>  Artificial Sequence

<220>
<223>  Synthetic peptide

<400>  16

Gly Glu Ser Arg Ala Glu Asp Gly Ala Lys Gly Asn Gly Arg Gly Lys
1               5                   10                  15

Gly Glu Ala Thr Ala Gly Ala Ala
            20

<210>  17
<211>  34
<212>  DNA
<213>  Homo sapiens

<400>  17
ataaatctgt ggaaacgctg ccacacaatc ttag                                34

<210>  18
<211>  34
<212>  DNA
<213>  Homo sapiens

<400>  18
ctaagattgt gtggcagcgt ttccacagat ttat                                    34


<210>  19
<211>  34
<212>  DNA
<213>  Homo sapiens

<400>  19
ataaatctgt ggaaacgctg ctccacagat ttat                                    34


<210>  20
<211>  34
<212>  DNA
<213>  Homo sapiens

<400>  20
ctaagattgt gtgaacgctg ccacacaatc ttag                                    34


<210>  21
<211>  34
<212>  DNA
<213>  Homo sapiens

<400>  21
ataaatttgt ggaaattaaa caacacactc ttaa                                    34


<210>  22
<211>  34
<212>  DNA
<213>  Homo sapiens

<400>  22
acaaaattgt ggaaattaaa caacacaatc ttaa                                    34


<210>  23
<211>  34
<212>  DNA
<213>  Homo sapiens

<400>  23
acaaatatgt ggaaattaaa caacacactc ttga                                    34


<210>  24
<211>  34
<212>  DNA
<213>  Homo sapiens

<400>  24
ataaatttgt ggaaattaaa caacacactc ttaa                                    34


<210>  25
<211>  34
<212>  DNA
<213>  Homo sapiens

<400>  25
ttaagagtgt gttttttaat ttccacatat ttgt                                    34


<210>  26
<211>  34
<212>  DNA
<213>  Homo sapiens

<400>  26
ataaatatgt gaatatacat attcacatat ttat                                   34


<210>  27
<211>  34
<212>  DNA
<213>  Homo sapiens

<400>  27
atatatctat agatatagat atccacagat atat                                   34


<210>  28
<211>  34
<212>  DNA
<213>  Homo sapiens

<400>  28
ctatgttttt gaggtcttat tcacaaaatc tttt                                   34


<210>  29
<211>  34
<212>  DNA
<213>  Homo sapiens

<400>  29
ctattattgt gtaacaaatt acccccaaac ttag                                   34


<210>  30
<211>  343
<212>  PRT
<213>  Artificial Sequence

<220>
<223>  Recombinant peptide

<400>  30

Met Ser Asp Leu Gln Thr Leu His Gln Asn Leu Ser Ala Leu Leu Val
1               5                   10                  15


Asp Ala Thr Ser Asp Glu Ala Arg Lys Asn Leu Met Asp Val Phe Arg
            20                  25                  30


Asp Arg Gln Ala Phe Ser Glu Ala Thr Trp Asn Thr Leu Leu Ser Val
        35                  40                  45


Cys Arg Ser Trp Ala Ala Trp Cys Lys Ser Asn Asn Arg Glu Trp Phe

50                  55                  60

Pro Ala Glu Pro Glu Asp Val Arg Asp Tyr Leu Leu His Leu Gln Val
65                  70                  75                  80

Arg Gly Leu Ala Val Asn Thr Ile Gln Arg His Leu Ala Leu Leu Asn
                85                  90                  95

Met Leu His Arg Arg Ser Gly Leu Pro Arg Pro Gly Asp Ser Asn Ala
            100                 105                 110

Val Ser Leu Val Met Arg Arg Ile Arg Lys Glu Asn Val Asp Ala Gly
        115                 120                 125

Glu Arg Val Arg Gln Ala Leu Ala Phe Glu Arg Thr Asp Phe Asp Gln
    130                 135                 140

Val Arg Ser Leu Met Gly Ser Ser Asp Arg Arg Asn Asp Ile Arg Asn
145                 150                 155                 160

Leu Ala Phe Leu Gly Val Ala Tyr Asn Thr Leu Leu Arg Ile Ser Glu
                165                 170                 175

Ile Ala Arg Ile Arg Ile Lys Asp Ile Ser Arg Thr Asp Gly Gly Arg
            180                 185                 190

Met Leu Val His Ile Gly Arg Thr Lys Thr Leu Val Ser Thr Ala Gly
        195                 200                 205

Val Glu Lys Ala Leu Ser Leu Gly Val Thr Arg Leu Val Glu Arg Trp
    210                 215                 220

Ile Ser Val Ser Gly Val Ala Gly Asp Pro Asn Asn Tyr Leu Phe Cys
225                 230                 235                 240

Arg Val Arg Lys Asn Gly Val Ala Ala Pro Ser Ala Ser Ser Gln Leu
                245                 250                 255

Thr Thr Asp Val Leu Arg Gly Ile Phe Val Ala Ala His Arg Leu Val
            260                 265                 270

His Gly Ala Arg Asp Asp Ser Gly Gln Arg Tyr Leu Thr Trp Ser Gly
        275                 280                 285

His Ser Ala Arg Val Gly Ala Ala Arg Asp Met Ala Arg Ala Gly Val
    290                 295                 300

Ser Ile Ala Glu Ile Met Gln Ala Gly Gly Trp Thr Thr Val Glu Ser
305                 310                 315                 320

Val Met Ser Tyr Leu Arg Asn Leu Asp Ser Glu Thr Gly Ala Met Val
                325                 330                 335

Arg Leu Leu Glu Asp Gly Asp
            340

<210>  31
<211>  343
<212>  PRT
<213>  Artificial Sequence

<220>
<223>  Recombinant peptide

<400>  31

Met Ser Asn Leu Gln Ala Leu His Gln Asn Pro Ser Thr Leu Leu Ala
1               5                   10                  15

Asp Ala Thr Cys Asp Glu Val Arg Lys Asn Leu Met Asp Met Phe Arg
            20                  25                  30

Asp Ser Gln Ala Phe Ser Glu His Thr Trp Lys Ala Leu Gln Ser Val
        35                  40                  45

Cys Arg Ser Trp Val Ala Trp Cys Lys Leu Asn Ser Arg Arg Trp Phe
    50                  55                  60

Pro Ala Glu Pro Glu Asp Val Arg Asp Tyr Leu Leu His Leu Gln Thr
65                  70                  75                  80

Arg Gly Leu Ala Val Lys Thr Ile Gln His His Leu Gly Ser Leu Asn
                85                  90                  95

Met Leu His Arg Gln Ser Gly Leu Pro Arg Pro Gly Asp Ser Asn Ala
            100                 105                 110

Val Ser Leu Val Met Arg Arg Ile Arg Arg Glu Asn Val Asp Ala Gly
        115                 120                 125

Glu Arg Ala Lys Gln Ala Leu Ala Phe Glu Arg Ala Asp Leu Asp Gln
    130                 135                 140

Val Arg Ala Leu Met Glu Asn Ser Asp Arg Cys Gln Asp Ile Arg Asn
145                 150                 155                 160

Leu Ala Phe Leu Gly Val Ala Tyr Asn Thr Leu Leu Arg Ile Gly Glu

                165                 170                 175

Ile Ala Arg Ile Arg Val Arg Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Lys Leu Val Glu Arg Trp
    210                 215                 220

Ile Ser Val Ser Gly Val Ala Asp Asp Pro Asp Asn Tyr Leu Phe Cys
225                 230                 235                 240

Arg Val Arg Arg Tyr Gly Val Ala Lys Pro Ser Ala Thr Ser Gln Leu
                245                 250                 255

Ser Thr Tyr Ala Leu Gln Gly Ile Phe Gly Ala Ala His Arg Leu Val
            260                 265                 270

Tyr Gly Ala Lys Asp Asp Ser Gly Gln Lys Tyr Leu Ala Trp Ser Gly
        275                 280                 285

His Ser Ala Arg Val Gly Ala Ala Arg Asp Met Ala Arg Ala Gly Val
    290                 295                 300

Ser Leu Pro Glu Ile Met Gln Ala Gly Gly Trp Thr Thr Val Asn Ser
305                 310                 315                 320

Val Met Asn Tyr Ile Arg Asn Leu Asp Ser Glu Thr Gly Ala Met Val
                325                 330                 335

Arg Leu Leu Glu Asp Asp Asp
            340

<210> 32
<211> 343
<212> PRT
<213> Artificial Sequence

<220>
<223> Recombinant protein

<400> 32

Met Ser Asn Leu Gln Thr Leu His Gln Asn Leu Ser Ala Leu Leu Ala
1               5                   10                  15

Asn Ala Thr Ser Asp Glu Ala Arg Lys Asn Leu Met Asp Val Phe Arg
            20                  25                  30

Asp Arg Arg Ala Phe Ser Glu Ala Thr Trp Lys Thr Leu Leu Ser Val
        35                  40                  45

Cys Arg Thr Trp Ala Ala Trp Cys Lys Leu Asn Asn Arg Lys Trp Phe
    50                  55                  60

Pro Ala Glu Pro Glu Asp Val Arg Asp Tyr Leu Leu His Leu Gln Val
65                  70                  75                  80

Arg Gly Leu Ala Val Asn Thr Ile Gln Arg His Leu Ala Leu Leu Asn
                85                  90                  95

Met Leu His Arg Arg Ser Gly Leu Pro Arg Pro Gly Asp Ser Ser Ala
            100                 105                 110

Val Ser Leu Val Met Arg Arg Ile Arg Lys Glu Asn Val Asp Ala Gly
        115                 120                 125

Glu Arg Val Arg Gln Ala Leu Ala Phe Glu Arg Thr Asp Phe Asp Lys
    130                 135                 140

Val Arg Ser Leu Met Gly Asn Ser Asp Arg Cys Gln Asp Ile Arg Asn
145                 150                 155                 160

Leu Ala Phe Leu Gly Val Ala Tyr Asn Thr Leu Leu Arg Ile Ser Glu
                165                 170                 175

Ile Ala Arg Ile Arg Ile Lys Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Arg Leu Val Gly Arg Trp
    210                 215                 220

Ile Ser Val Ser Gly Val Ala Gly Asp Pro Asn Asn Tyr Leu Phe Cys
225                 230                 235                 240

Arg Val Arg Lys Asn Gly Val Ala Ala Pro Ser Ala Thr Ser Gln Leu
                245                 250                 255

Ser Thr Asp Val Leu Arg Gly Val Phe Ala Ala Ala His Arg Leu Val
            260                 265                 270

Tyr Gly Thr Lys Asp Asp Ser Gly Gln Gly Tyr Leu Thr Trp Ser Gly

275                 280                 285

His Ser Ala Arg Val Gly Ala Ala Arg Asp Met Ala Arg Ala Gly Val
    290                 295                 300

Ser Ile Ala Glu Ile Met Gln Ala Gly Gly Trp Thr Thr Val Glu Ser
305                 310                 315                 320

Val Met Ser Tyr Leu Arg Asn Leu Asp Ser Glu Thr Gly Ala Met Val
                325                 330                 335

Arg Leu Leu Glu Asp Gly Asp
            340

<210>  33
<211>  343
<212>  PRT
<213>  Artificial Sequence

<220>
<223>  Recombinant protein

<400>  33

Met Ser Asn Ile Gln Thr Pro His Gln Ser Leu Ser Ala Leu Leu Thr
1               5                   10                  15

Asp Ala Thr Ser Asp Val Thr Arg Lys Asn Leu Ala Asp Met Phe Arg
            20                  25                  30

Asp Ser Gln Ala Phe Ser Glu His Thr Trp Lys Met Leu Leu Ser Val
        35                  40                  45

Cys Arg Ser Trp Ala Ala Trp Cys Glu Leu Asn Asn Arg Lys Trp Leu
    50                  55                  60

Pro Val Glu Pro Glu Asp Val Arg Asp Tyr Leu Leu His Leu Gln Thr
65                  70                  75                  80

Arg Gly Leu Ala Val Lys Thr Ile Gln His His Leu Gly Ser Leu Asn
                85                  90                  95

Met Leu His Arg Arg Ala Gly Leu Pro Arg Pro Gly Asp Ser Asn Ala
            100                 105                 110

Val Ser Leu Val Met Arg Arg Ile Arg Arg Glu Asn Val Asp Ala Gly
        115                 120                 125

Glu Arg Ala Gln Gln Ala Leu Ala Phe Glu Arg Thr Asp Phe Asp Gln
    130                 135                 140

Val Arg Ser Leu Val Glu Asn Ser Asp Arg Cys Gln Asp Ile Arg Asn
145                 150                 155                 160

Leu Ala Phe Leu Gly Val Ala Tyr Asn Thr Leu Leu Arg Ile Ser Glu
                165                 170                 175

Ile Ala Arg Ile Arg Val Lys Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Lys Leu Val Glu Arg Trp
    210                 215                 220

Ile Ser Val Ser Gly Val Ala Asp Asp Pro Asn Asn Tyr Leu Phe Cys
225                 230                 235                 240

Arg Val Arg Arg Tyr Gly Val Ala Lys Pro Ser Ala Thr Ser Gln Leu
                245                 250                 255

Ser Thr Tyr Ala Leu Gln Gly Ile Phe Gly Ala Ala His Arg Leu Val
            260                 265                 270

Tyr Gly Ala Lys Gly Asp Ser Gly Gln Lys Tyr Leu Ala Trp Ser Gly
        275                 280                 285

His Ser Ala Arg Val Gly Ala Ala Arg Asp Met Ala Arg Ala Gly Val
    290                 295                 300

Pro Ile Pro Glu Ile Met Gln Ala Gly Gly Trp Thr Thr Val Asn Ser
305                 310                 315                 320

Val Met Asn Tyr Ile Arg Asn Leu Asp Ser Glu Thr Gly Ala Met Val
                325                 330                 335

Arg Leu Leu Glu Asp Ser Asp
            340

<210>  34
<211>  1032
<212>  DNA
<213>  Artificial Sequence

<220>
<223>  Recombinant DNA

<400>  34

atgtccgatt tacagacctt acaccaaaat ttgtctgcat tactggtcga tgcaacgagc 60

gatgaggctc gcaagaacct gatggacgtg ttcagggacc gccaggcgtt ttctgaggca 120

acctggaata cgctcctgtc cgtttgccgg tcgtgggcag catggtgcaa gtcgaataac 180

cgggaatggt ttcccgcaga acctgaagat gttcgcgatt atcttctaca cctccaggta 240

cgcggtctgg cagtaaacac tatccagcga catttggcgc tgctgaacat gcttcaccgt 300

cggtccgggc tgccacgacc aggcgacagc aatgctgttt cactggttat gcggcgtatc 360

cggaaagaaa acgttgatgc cggtgagcgt gtaagacagg ctctggcgtt cgaacgcacc 420

gatttcgacc aggttcgttc actcatgggg agtagcgatc ggcgcaatga tatacgtaat 480

ctggcatttc ttggggttgc atataacacc ctgttacgta tatccgaaat cgccaggatt 540

cggataaaag atatctcgcg tactgacggt gggaggatgc tagtccatat tggcagaacg 600

aaaacgctgg tcagcaccgc aggcgtagag aaagcactta gcctgggggt aaccagactg 660

gtcgagcgat ggatttccgt ctctggcgta gctggtgatc cgaataacta cctgttttgc 720

cgggtcagaa aaaacggtgt tgccgcgcca tctgcctcca gccagctgac aaccgacgtc 780

ctgcgaggga tttttgtagc cgcccaccgc ctggtccacg gtgccaggga tgactctggg 840

cagagatacc tgacctggtc tggacacagt gcccgcgtcg gagccgcgcg ggacatggcc 900

cgcgctggag tctcaatagc ggagattatg caggctggtg gctggaccac cgtagagagc 960

gtcatgagct atctccgtaa cctggatagt gaaacggggg caatggtgcg cctgctggaa 1020

gacggcgatt ag 1032

<210> 35
<211> 1032
<212> DNA
<213> Artificial Sequence

<220>
<223> Recombinant DNA

<400> 35
atgtccaatt tacaggcctt acaccaaaat ccgtccacat tactggctga cgcgacgtgt 60

gacgaggttc gcaagaacct gatggacatg ttcagggata gccaggcgtt ttcagagcat 120

acctggaaag cgcttcaatc cgtttgccgg tcgtgggtgg catggtgcaa actgaacagc 180

cggaggtggt ttcccgcaga gcccgaagat gttcgcgatt atcttctgca tcttcagacg 240

cgcggtctgg cagtaaaaac catccagcac catctgggct cactaaacat gcttcatcgt 300

cagtccgggc tgccacgacc aggtgacagc aatgccgttt cactggttat gcggcgaatc 360

cgaagagaaa acgttgatgc cggtgaacgt gcaaaacagg ctctggcgtt cgagcgcgct 420

gacctcgacc aggtccgtgc actcatggaa aacagcgatc gctgccagga tatacgtaat 480

ctggcatttc tgggcgttgc ttacaacacc ctgttacgaa taggcgaaat tgccagaatc 540

cgggttagag atatctcacg tactgacggt gggagaatgt taatccacat tggcagaacg 600

aaaacactgg tcagcaccgc aggtgtggag aaggcactta gcctgggggt aactaaactg 660

gtcgagcgat ggatttccgt ctccggtgta gctgatgacc cagataacta cctgttttgc 720

cgggtcagaa gatatggcgt tgccaagcca tctgccacca gccagctgtc aacctacgcc 780

ctgcaaggga tttttggagc cgctcaccgc ctggtctacg gtgctaagga tgactctggt 840

cagaaatacc tggcctggtc tggacacagt gcccgtgtcg gagccgcgcg ggatatggcc 900

cgcgctggag tttcgttacc ggagattatg caggctggtg gctggaccac cgtaaatagt 960

gtcatgaact atatccgtaa cctggatagt gagacagggg ccatggtgcg cctgctggaa 1020

gatgacgact ag 1032

<210> 36
<211> 1032
<212> DNA
<213> Artificial Sequence

<220>
<223> Recombinant DNA

<400> 36
atgtccaatc tacagaccct acaccagaat ttgtccgcac tactggccaa tgcaacaagt 60

gatgaggctc gcaagaacct gatggacgtc ttcaggaccc gccgggcgtt ttctgaagct 120

acctggaaaa cgcttctgtc cgtttgccgg acgtgggcgg catggtgcaa gctgaataac 180

cggaaatggt ttcccgcaga acctgaagat gtacgcgatt atcttctaca tctccaggta 240

cgcggtctgg cggtgaacac aatccagcga cacttggccc tgctaaacat gcttcaccgt 300

cggtccgggc tgccgcgacc aggtgacagc agcgctgttt cactggttat gcggcgtatc 360

cgaaaggaaa acgttgatgc cggtgaacgc gtaaggcagg ccctggcgtt cgagcgcact 420

gatttcgaca aggttcgttc actcatggga aacagcgatc gctgccagga tatacgtaat 480

ctggcatttc tgggtgttgc atataacacc ctgctacgca tatccgaaat tgccaggatt 540

cggataaaag atatctcacg taccgacggt gggaggatgc taatccatat cggcagaacg 600

aaaacgctgg ttagcaccgc aggcgtagag aaggcactta gccttggggt aaccagactg 660

gtcgggcggt ggatttctgt ctctggtgta gctggcgacc cgaataacta cctgttctgc 720

cgggtcagaa aaaatggtgt tgccgcgcca tctgccacca gccagctatc gaccgacgtc 780

ctgagagggg tttttgcagc cgcccaccgt ctggtctacg gtaccaagga tgactctggt 840

caggggtacc tgacctggtc tggacacagt gcccgcgtcg gagccgcgcg ggatatggcc 900

cgcgctgggg tttcaatagc ggagattatg caggctggtg gctggaccac cgtagagagc 960

gttatgagct atctccgtaa cctggatagt gagacaggtg caatggtgcg cctgctggag 1020

gatggcgatt ag                                                          1032

<210>  37
<211>  1032
<212>  DNA
<213>  Artificial Sequence

<220>
<223>  Recombinant DNA

<400>  37
atgtccaata tacagacccc acaccaaagt ttgtctgcat tactgactga tgcaacgagt        60

gatgtgactc gcaagaacct ggcggacatg ttcagggata gccaggcgtt ttcagagcat       120

acctggaaaa tgctcctgtc cgtttgccgg tcgtgggcgg catggtgcga gctgaataac       180

cggaaatggc ttcccgtaga acctgaggat gttcgcgatt acctcctgca ccttcagacg       240

cgtggtctgg cggtaaaaac tatccagcat catctgggct cgctaaacat gcttcaccgt       300

cgggctgggc tgccacgacc gggtgacagc aatgctgttt cactggttat gcggcgtatc       360

cgaagagaaa acgttgatgc cggtgaacgc gcacaacagg ccctggcgtt cgaacgcacc       420

gatttcgacc aggttcggtc actcgtggaa aacagcgacc gctgccagga tatacgtaat       480

ctggcatttc tgggggtcgc ttataacacc ctgttgcgta tatccgaaat tgccaggatt       540

cgggttaagg atatctcacg tactgacggt gggaggatgt taatccatat tggcagaacg       600

aaaacgctgg ttagtaccgc aggtgtagag aaggcactca gcctgggggt gaccaaactg       660

gtcgagcgat ggatttccgt ctccggtgta gccgatgacc caaataacta cctgttttgc       720

cgggtcagaa gatatggcgt tgccaagccg tctgccacca gccagctgtc aacttacgcc       780

ctgcaaggga ttttcggagc cgcccaccgc ctggtctacg gtgccaaggg tgactctggc       840

cagaagtacc tggcctggtc gggacacagt gcccgtgtcg gagccgcacg agacatggcc       900

cgcgctggag ttccaatacc ggagattatg caggctggtg gctggaccac cgtgaatagt       960

gtcatgaact atatccgtaa cctggatagc gagacaggag caatggtgcg cctgctggaa      1020

gacagcgatt ag                                                          1032

<210>  38
<211>  25
<212>  DNA
<213>  Artificial Sequence

<220>
<223>  Artificial oligonucleotide

<400>  38
   tcgagggtgg cagcggcggt agcat                                               25

<210>  39
<211>  25

<212>  DNA
<213>  Artificial Sequence

<220>
<223>  Synthetic oligonucleotide

<400>  39
gtacatgcta ccgccgctgc caccc                                          25


<210>  40
<211>  43
<212>  DNA
<213>  Artificial Sequence

<220>
<223>  Synthetic oligonucleotide

<400>  40
tcgagggcgg tagcggtggc tctggcggca gcggtggtag cat                      43


<210>  41
<211>  43
<212>  DNA
<213>  Artificial Sequence

<220>
<223>  Synthetic oligonucleotide

<400>  41
gtacatgcta ccaccgctgc cgccagagcc accgctaccg ccc                      43


<210>  42
<211>  61
<212>  DNA
<213>  Artificial Sequence

<220>
<223>  Syntheti oligonucleotide

<400>  42
tcgagggcgg tagcggtggc tctggcggca gcggtggtag cggtggctct ggcggtagta    60

t                                                                    61


<210>  43
<211>  60
<212>  DNA
<213>  Artificial Sequence

<220>
<223>  Synthetic oligonucleotide

<400>  43
tacatactac cgccagagcc accgctacca ccgctgccgc cagagccacc gctaccgccc    60


<210>  44
<211>  75
<212>  DNA

<213>  Artificial Sequence

<220>
<223>  Synthetic oligonucleotide

<400>  44
tcgagggcgg tagcggtggc tctggcggca gcggtggtag cggtggctct ggcggtagtg      60

gtggtagtgg cggca                                                        75


<210>  45
<211>  66
<212>  DNA
<213>  Artificial Sequence

<220>
<223>  Synthetic oligonucleotide

<400>  45
gtacatgctg ccgccactac caccactacc gccagagcca ccgctaccac cgctgccgcc      60

agagcc                                                                  66


<210>  46
<211>  97
<212>  DNA
<213>  Artificial Sequence

<220>
<223>  Synthetic oligonucleotide

<400>  46
tcgagggcgg tagcggtggc tctggcggca gcggtggtag cggtggctct ggcggtagtg      60

gtggtagtgg cggcagcggt ggctctggcg gtagtat                                97


<210>  47
<211>  97
<212>  DNA
<213>  Artificial Sequence

<220>
<223>  Synthetic oligonucleotide

<400>  47
gtacatacta ccgccagagc caccgctgcc gccactacca ccactaccgc cagagccacc      60

gctaccaccg ctgccgccag agccaccgct accgccc                                97


<210>  48
<211>  115
<212>  DNA
<213>  Artificial Sequence

<220>
<223>  Synthetic oligonucleotide

<400>  48
tcgagggcgg tagcggtggc tctggcggca gcggtggtag cggtggctct ggcggtagtg      60

gtggtagtgg cggcagcggt ggctctggcg gtagtggtgg tagcggtggc tctat          115

<210>  49
<211>  115
<212>  DNA
<213>  Artificial Sequence

<220>
<223>  Synthetic oligonucleotide

<400>  49
gtacatagag ccaccgctac caccactacc gccagagcca ccgctgccgc cactaccacc      60

actaccgcca gagccaccgc taccaccgct gccgccagag ccaccgctac cgccc          115

<210>  50
<211>  133
<212>  DNA
<213>  Artificial Sequence

<220>
<223>  Synthetic oligonucleotide

<400>  50
tcgagggcgg tagcggtggc tctggcggca gcggtggtag cggtggctct ggcggtagtg      60

gtggtagtgg cggcagcggt ggctctggcg gtagtggtgg tagcggtggc tctggaggta     120

gtggcggatc aat                                                        133

<210>  51
<211>  133
<212>  DNA
<213>  Artificial Sequence

<220>
<223>  Synthetic oligonucleotide

<400>  51
gtacattgat ccgccactac ctccagagcc accgctacca ccactaccgc cagagccacc      60

gctgccgcca ctaccaccac taccgccaga gccaccgcta ccaccgctgc cgccagagcc     120

accgctaccg ccc                                                        133

<210>  52
<211>  128
<212>  DNA
<213>  Artificial Sequence

<220>
<223>  Synthetic oligonucleotide

<220>
<221>  variation
<222>  (29)..(46)
<223>  r is a or g

v is a or c or g
m is a or c

<220>
<221>  variation
<222>  (83)..(100)
<223>  r is a or g
v is a or c or g
m is a or c

<400>  52
gaagtgccat tccgcctgac ctctcgagrv mrvmrvmrvm rvmrvmggcg gcagcggtgg      60

tagcggtggc tctggcggta gtrvmrvmrv mrvmrvmrvm atgtacaagg ctaggtggag     120

gctcagtg                                                              128


<210>  53
<211>  128
<212>  DNA
<213>  Artificial Sequence

<220>
<223>  Synthetic oligonucleotide


<220>
<221>  variation
<222>  (47)..(82)
<223>  r is a or g
v is a or c or g
m is a or c

<400>  53
gaagtgccat tccgcctgac ctctcgaggg cggtagcggt ggctctrvmr vmrvmrvmrv      60

mrvmrvmrvm rvmrvmrvmr vmggtggtag tggcggcagc atgtacaagg ctaggtggag     120

gctcagtg                                                              128


<210>  54
<211>  128
<212>  DNA
<213>  Artificial Sequence

<220>
<223>  Synthetic oligonucleotide


<220>
<221>  variation
<222>  (29)..(100)
<223>  r is a or g
v is a or c or g
m is a or c

<400>  54
gaagtgccat tccgcctgac ctctcgagrv mrvmrvmrvm rvmrvmrvmr vmrvmrvmrv      60

mrvmrvmrvm rvmrvmrvmr vmrvmrvmrv mrvmrvmrvm atgtacaagg ctaggtggag     120

gctcagtg                                                          128

<210>  55
<211>  72
<212>  DNA
<213>  Artificial Sequence

<220>
<223>  Recombinant oligonucleotide

<400>  55
gccgaagcaa caagcgaagg cggcagcggt ggtagcggtg gctctggcgg tagtaacggc      60

gcaagaagaa cc                                                          72

<210>  56
<211>  72
<212>  DNA
<213>  Artificial Sequence

<220>
<223>  Recombinant oligonucleotide

<400>  56
gcaggaacaa ccgcaagagg cggcagcggt ggtagcggtg gctctggcgg tagtggaaga      60

agaggagcaa aa                                                          72

<210>  57
<211>  72
<212>  DNA
<213>  Artificial Sequence

<220>
<223>  Recombinant oligonucleotide

<400>  57
aaaaacggaa gaggaagagg cggcagcggt ggtagcggtg gctctggcgg tagtagaaca      60

aaaagagaaa ca                                                          72

<210>  58
<211>  72
<212>  DNA
<213>  Artificial Sequence

<220>
<223>  recombinant oligonucleotide

<400>  58
ggcggtagcg gtggctctac cgccgccaaa gaaggagcag caagcagcgc cagcggtggt      60

agtggcggca gc                                                          72

<210>  59
<211>  72
<212>  DNA
<213>  Artificial Sequence

<220>
<223>  Recombinant oligonucleotide

<400>  59
ggcggtagcg gtggctctaa cagcagaagc aacacagaaa acagcgacaa aggcggtggt      60

agtggcggca gc                                                          72


<210>  60
<211>  72
<212>  DNA
<213>  Artificial Sequence

<220>
<223>  Recombinant oligonucleotide

<400>  60
ggcggtagcg gtggctctaa cggcgaagaa ggaaccgaaa gaggcaaagc aacaggtggt      60

agtggcggca gc                                                          72


<210>  61
<211>  72
<212>  DNA
<213>  Artificial Sequence

<220>
<223>  Synthetic oligonucleotide

<400>  61
ggcggtagcg gtggctctac caccaaagca aacagagcaa aaggcggcag aggaggtggt      60

agtggcggca gc                                                          72


<210>  62
<211>  72
<212>  DNA
<213>  Artificial Sequence

<220>
<223>  Recombinant oligonucleotide

<400>  62
ggcgcaaacg aagacacaaa cacagaagca gccggaagcg aaggcaacga aaaaaccgga      60

acaaacagcg ct                                                          72


<210>  63
<211>  72
<212>  DNA
<213>  Artificial Sequence

<220>
<223>  Recombinant oligonucleotide

<400>  63
ggcgaaagca gagcagaaga cggcgcaaaa ggaaacggca gaggcaaagg agaagcaaca      60

gcaggcgcag ca                                                          72

<210>  64
<211>  2148
<212>  DNA
<213>  Artificial Sequence

<220>
<223>  Recombinant DNA

<400>  64
atgtccaatt tacaggcctt acaccaaaat ccgtccacat tactggctga cgcgacgtgt      60

gacgaggttc gcaagaacct gatggacatg ttcagggata gccaggcgtt ttcagagcat     120

acctggaaag cgcttcaatc cgtttgccgg tcgtgggtgg catggtgcaa actgaacagc     180

cggaggtggt ttcccgcaga gcccgaagat gttcgcgatt atcttctgca tcttcagacg     240

cgcggtctgg cagtaaaaac catccagcac catctgggct cactaaacat gcttcatcgt     300

cagtccgggc tgccacgacc aggtgacagc aatgccgttt cactggttat gcggcgaatc     360

cgaagagaaa acgttgatgc cggtgaacgt gcaaaacagg ctctggcgtt cgagcgcgct     420

gacctcgacc aggtccgtgc actcatggaa aacagcgatc gctgccagga tatacgtaat     480

ctggcatttc tgggcgttgc ttacaacacc ctgttacgaa taggcgaaat tgccagaatc     540

cgggttagag atatctcacg tactgacggt gggagaatgt taatccacat tggcagaacg     600

aaaacactgg tcagcaccgc aggtgtggag aaggcactta gcctgggggt aactaaactg     660

gtcgagcgat ggatttccgt ctccggtgta gctgatgacc cagataacta cctgttttgc     720

cgggtcagaa gatatggcgt tgccaagcca tctgccacca gccagctgtc aacctacgcc     780

ctgcaaggga tttttggagc cgctcaccgc ctggtctacg gtgctaagga tgactctggt     840

cagaaatacc tggcctggtc tggacacagt gcccgtgtcg gagccgcgcg ggatatggcc     900

cgcgctggag tttcgttacc ggagattatg caggctggtg gctggaccac cgtaaatagt     960

gtcatgaact atatccgtaa cctggatagt gagacagggg ccatggtgcg cctgctggaa    1020

gatgacgact cgctcgaggg cggtagcggt ggctctacca ccaaagcaaa cagagcaaaa    1080

ggcggcagag gaggtggtag tggcggcagc atgtacatgt ccgatttaca gaccttacac    1140

caaaatttgt ctgcattact ggtcgatgca acgagcgatg aggctcgcaa gaacctgatg    1200

gacgtgttca gggaccgcca ggcgttttct gaggcaacct ggaatacgct cctgtccgtt    1260

tgccggtcgt gggcagcatg gtgcaagtcg aataaccggg aatggtttcc cgcagaacct    1320

gaagatgttc gcgattatct tctacacctc caggtacgcg gtctggcagt aaacactatc    1380

cagcgacatt tggcgctgct gaacatgctt caccgtcggt ccgggctgcc acgaccaggc    1440

gacagcaatg ctgtttcact ggttatgcgg cgtatccgga aagaaaacgt tgatgccggt    1500

gagcgtgtaa gacaggctct ggcgttcgaa cgcaccgatt tcgaccaggt tcgttcactc    1560

atggggagta gcgatcggcg caatgatata cgtaatctgg catttcttgg ggttgcatat 1620

aacaccctgt tacgtatatc cgaaatcgcc aggattcgga taaaagatat ctcgcgtact 1680

gacggtggga ggatgctagt ccatattggc agaacgaaaa cgctggtcag caccgcaggc 1740

gtagagaaag cacttagcct gggggtaacc agactggtcg agcgatggat ttccgtctct 1800

ggcgtagctg gtgatccgaa taactacctg ttttgccggg tcagaaaaaa cggtgttgcc 1860

gcgccatctg cctccagcca gctgacaacc gacgtcctgc gagggatttt tgtagccgcc 1920

caccgcctgg tccacggtgc cagggatgac tctgggcaga gatacctgac ctggtctgga 1980

cacagtgccc gcgtcggagc cgcgcgggac atggcccgcg ctggagtctc aatagcggag 2040

attatgcagg ctggtggctg gaccaccgta gagagcgtca tgagctatct ccgtaacctg 2100

gatagtgaaa cgggggcaat ggtgcgcctg ctggaagacg gcgattag 2148

<210> 65
<211> 2148
<212> DNA
<213> Artificial Sequence

<220>
<223> Recombinant DNA

<400> 65
atgtccaata tacagacccc acaccaaagt ttgtctgcat tactgactga tgcaacgagt 60

gatgtgactc gcaagaacct ggcggacatg ttcagggata gccaggcgtt ttcagagcat 120

acctggaaaa tgctcctgtc cgtttgccgg tcgtgggcgg catggtgcga gctgaataac 180

cggaaatggc ttcccgtaga acctgaggat gttcgcgatt acctcctgca ccttcagacg 240

cgtggtctgg cggtaaaaac tatccagcat catctgggct cgctaaacat gcttcaccgt 300

cgggctgggc tgccacgacc gggtgacagc aatgctgttt cactggttat gcggcgtatc 360

cgaagagaaa acgttgatgc cggtgaacgc gcacaacagg ccctggcgtt cgaacgcacc 420

gatttcgacc aggttcggtc actcgtggaa aacagcgacc gctgccagga tatacgtaat 480

ctggcatttc tgggggtcgc ttataacacc ctgttgcgta tatccgaaat tgccaggatt 540

cgggttaagg atatctcacg tactgacggt gggaggatgt taatccatat tggcagaacg 600

aaaacgctgg ttagtaccgc aggtgtagag aaggcactca gcctgggggt gaccaaactg 660

gtcgagcgat ggatttccgt ctccggtgta gccgatgacc caaataacta cctgttttgc 720

cgggtcagaa gatatggcgt tgccaagccg tctgccacca gccagctgtc aacttacgcc 780

ctgcaaggga ttttcggagc cgcccaccgc ctggtctacg gtgccaaggg tgactctggc 840

cagaagtacc tggcctggtc gggacacagt gcccgtgtcg gagccgcacg agacatggcc 900

cgcgctggag ttccaatacc ggagattatg caggctggtg gctggaccac cgtgaatagt 960

gtcatgaact atatccgtaa cctggatagc gagacaggag caatggtgcg cctgctggaa 1020

gacagcgatt cgctcgaggg cggtagcggt ggctctacca ccaaagcaaa cagagcaaaa 1080

ggcggcagag gaggtggtag tggcggcagc atgtacatgt ccaatctaca gaccctacac 1140

cagaatttgt ccgcactact ggccaatgca acaagtgatg aggctcgcaa gaacctgatg 1200

gacgtcttca gggaccgccg ggcgttttct gaagctacct ggaaaacgct tctgtccgtt 1260

tgccggacgt gggcggcatg gtgcaagctg aataaccgga aatggtttcc cgcagaacct 1320

gaagatgtac gcgattatct tctacatctc caggtacgcg gtctggcggt gaacacaatc 1380

cagcgacact tggccctgct aaacatgctt caccgtcggt ccgggctgcc gcgaccaggt 1440

gacagcagcg ctgtttcact ggttatgcgg cgtatccgaa aggaaaacgt tgatgccggt 1500

gaacgcgtaa ggcaggccct ggcgttcgag cgcactgatt tcgacaaggt tcgttcactc 1560

atgggaaaca gcgatcgctg ccaggatata cgtaatctgg catttctggg tgttgcatat 1620

aacaccctgc tacgcatatc cgaaattgcc aggattcgga taaaagatat ctcacgtacc 1680

gacggtggga ggatgctaat ccatatcggc agaacgaaaa cgctggttag caccgcaggc 1740

gtagagaagg cacttagcct tggggtaacc agactggtcg ggcggtggat ttctgtctct 1800

ggtgtagctg gcgacccgaa taactacctg ttctgccggg tcagaaaaaa tggtgttgcc 1860

gcgccatctg ccaccagcca gctatcgacc gacgtcctga gaggggtttt tgcagccgcc 1920

caccgtctgg tctacggtac caaggatgac tctggtcagg ggtacctgac ctggtctgga 1980

cacagtgccc gcgtcggagc cgcgcgggat atggcccgcg ctggggtttc aatagcggag 2040

attatgcagg ctggtggctg gaccaccgta gagagcgtta tgagctatct ccgtaacctg 2100

gatagtgaga caggtgcaat ggtgcgcctg ctggaggatg gcgattag 2148

<210> 66
<211> 343
<212> PRT
<213> Artificial Sequence

<220>
<223> Recombinant protein

<400> 66

Met Ser Asn Leu Gln Thr Leu His Gln Asn Leu Ser Ala Leu Leu Ala
1               5                   10                  15

Asp Ala Thr Ser Asp Glu Thr Arg Lys Asn Leu Met Asp Val Leu Arg
            20                  25                  30

Asp Ser Gln Ala Phe Ser Glu Gln Thr Trp Lys Ser Leu Leu Phe Val
        35                  40                  45

Cys Arg Ser Trp Ala Ala Trp Cys Glu Leu Asn Asn Arg Glu Trp Phe
    50                  55                  60

Pro Ala Glu Pro Glu Asp Val Arg Asp Tyr Leu Leu His Leu Gln Ala
65                  70                  75                  80

Arg Gly Leu Ala Val Lys Thr Ile Gln Asn His Leu Gly Ser Leu Asn
                85                  90                  95

Met Leu His Arg Arg Ser Gly Leu Pro Arg Pro Gly Asp Ser Asn Ala
            100                 105                 110

Val Ser Leu Val Met Arg Arg Ile Arg Lys Glu Asn Val Asp Ala Gly
        115                 120                 125

Glu Arg Ala Lys Gln Ala Leu Ala Phe Glu Arg Ala Asp Phe Ser Arg
    130                 135                 140

Val Arg Ala Leu Met Glu Asn Ser Asp Arg Pro Gln Asp Ile Arg Asn
145                 150                 155                 160

Leu Ala Phe Leu Gly Val Ala Tyr Asn Thr Leu Leu Arg Ile Ser Glu
                165                 170                 175

Ile Ala Arg Ile Arg Val Lys Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Lys Leu Val Glu Arg Trp
    210                 215                 220

Ile Ser Val Ser Gly Val Val Asp Asp Pro Asn Asn Tyr Leu Phe Cys
225                 230                 235                 240

Arg Val Arg Arg Gly Gly Val Ala Lys Pro Ser Ala Thr Ser Gln Leu
                245                 250                 255

Ser Thr Cys Ala Leu Gln Gly Ile Phe Ala Ala Ala His Arg Leu Leu
            260                 265                 270

Tyr Gly Ala Lys Asp Asp Ser Gly Gln Arg Tyr Leu Ala Trp Ser Gly
        275                 280                 285

His Ser Ala Arg Val Gly Ala Ala Arg Asp Met Ala Arg Ala Gly Val
    290                 295                 300

Pro Ile Val Gln Ile Met Gln Ala Gly Gly Trp Thr Thr Val Glu Ser
305                 310                 315                 320

Val Met Asn Tyr Ile Arg Asn Leu Asp Ser Glu Thr Gly Ala Met Val
                325                 330                 335

Arg Leu Leu Glu Asp Gly Asp
            340

<210>  67
<211>  1032
<212>  DNA
<213>  Artificial Sequence

<220>
<223>  Recombinant DNA

<400>  67
atgtcaaatt tacagacctt acaccagaac ttgtccgcac tactggccga tgcaacgagc      60

gatgagactc gtaagaacct gatggatgtg ctcagggata gccaggcgtt ttccgagcaa     120

acctggaaat cactcctgtt cgtttgccgg tcgtgggcgg catggtgcga gctgaataac     180

cgggaatggt tccctgcgga acctgaagat gtccgcgatt atcttctgca ccttcaggcg     240

cgcggtctgg cagtaaaaac tatccagaac catttgggct cgctaaacat gctccaccgt     300

cggtccgggc tgccacggcc aggcgacagc aatgccgttt cactggttat gcgacgaatc     360

cgaaaagaaa acgttgatgc cggtgaacgt gcaaagcagg ccctggcgtt cgaacgcgcc     420

gacttcagcc gagttcgtgc gctcatggaa aatagcgatc gcccccagga tatacgtaat     480

ctggcctttc tgggggttgc ttataacacc ctgttacgca tatccgagat tgccaggatt     540

cgggttaagg acatctcacg tactgacggt gggagaatgt taatccacat cggcaggacg     600

aaaacgctgg tcagcaccgc aggcgtagag aaggcgctta gcctgggggt aaccaagctg     660

gtcgagcggt ggatctccgt ctctggtgta gtcgatgacc cgaataacta cctgttctgc     720

agggtcagac gaggtggtgt tgccaagcca tctgccacca gccagctatc aacttgcgcc     780

ctgcaaggga tttttgcagc cgcccaccgt ctgctctacg gtgccaagga tgactctggt     840

cagagatacc tggcctggtc tggacacagc gcccgcgtcg gggccgcgcg ggacatggcc     900

cgcgccgggg ttccaatagt gcagattatg caggctggtg gctggaccac cgtagagagt     960

gtcatgaact atatccgtaa cctggatagt gaaacaggag caatggtacg tctgctggag    1020

gacggcgatt ag                                                        1032

<210>  68
<211>  343
<212>  PRT
<213>  Homo sapiens

<400>  68

Met Ser Asn Leu Leu Thr Val His Gln Asn Leu Pro Ala Leu Pro Val
1               5                   10                  15

Asp Ala Thr Ser Asp Glu Val Arg Lys Asn Leu Met Asp Met Phe Arg
            20                  25                  30

Asp Arg Gln Ala Phe Ser Glu His Thr Trp Lys Met Leu Leu Ser Val
        35                  40                  45

Cys Arg Ser Trp Ala Ala Trp Cys Lys Leu Asn Asn Arg Lys Trp Phe
    50                  55                  60

Pro Ala Glu Pro Glu Asp Val Arg Asp Tyr Leu Leu Tyr Leu Gln Ala
65                  70                  75                  80

Arg Gly Leu Ala Val Lys Thr Ile Gln Gln His Leu Gly Gln Leu Asn
                85                  90                  95

Met Leu His Arg Arg Ser Gly Leu Pro Arg Pro Ser Asp Ser Asn Ala
            100                 105                 110

Val Ser Leu Val Met Arg Arg Ile Arg Lys Glu Asn Val Asp Ala Gly
        115                 120                 125

Glu Arg Ala Lys Gln Ala Leu Ala Phe Glu Arg Thr Asp Phe Asp Gln
    130                 135                 140

Val Arg Ser Leu Met Glu Asn Ser Asp Arg Cys Gln Asp Ile Arg Asn
145                 150                 155                 160

Leu Ala Phe Leu Gly Ile Ala Tyr Asn Thr Leu Leu Arg Ile Ala Glu
                165                 170                 175

Ile Ala Arg Ile Arg Val Lys Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Lys Leu Val Glu Arg Trp
    210                 215                 220

Ile Ser Val Ser Gly Val Ala Asp Asp Pro Asn Asn Tyr Leu Phe Cys
225                 230                 235                 240

Arg Val Arg Lys Asn Gly Val Ala Ala Pro Ser Ala Thr Ser Gln Leu
                245                 250                 255

Ser Thr Arg Ala Leu Glu Gly Ile Phe Glu Ala Thr His Arg Leu Ile
            260                 265                 270

Tyr Gly Ala Lys Asp Asp Ser Gly Gln Arg Tyr Leu Ala Trp Ser Gly
        275                 280                 285

His Ser Ala Arg Val Gly Ala Ala Arg Asp Met Ala Arg Ala Gly Val
    290                 295                 300

Ser Ile Pro Glu Ile Met Gln Ala Gly Gly Trp Thr Asn Val Asn Ile
305                 310                 315                 320

Val Met Asn Tyr Ile Arg Asn Leu Asp Ser Glu Thr Gly Ala Met Val
                325                 330                 335

Arg Leu Leu Glu Asp Gly Asp
            340

<210>  69
<211>  343
<212>  PRT
<213>  Artificial Sequence

<220>
<223>  Recombinant protein

<400>  69

Met Ser Asn Leu Leu Thr Leu His His Ser Leu Pro Ala Leu Pro Ala
1               5                   10                  15

Asp Ala Thr Ser Asp Glu Val Arg Lys Asn Leu Met Asp Val Phe Arg
            20                  25                  30

Asp Arg Pro Ala Phe Ser Glu His Thr Trp Glu Met Leu Leu Ser Val
        35                  40                  45

Cys Arg Ser Trp Ala Ala Trp Cys Lys Leu Asn Asn Arg Lys Trp Phe
    50                  55                  60

Pro Ala Glu Pro Glu Asp Val Arg Asp Tyr Leu Leu His Leu Gln Ala
65                  70                  75                  80

Arg Gly Leu Ala Val Lys Thr Ile Gln Gln His Leu Cys Arg Leu Asn
                85                  90                  95

Met Leu His Arg Arg Ser Gly Leu Pro Arg Pro Ser Asp Ser Asn Ala

            100                 105                 110

Val Ser Leu Val Met Arg Arg Ile Arg Lys Glu Asn Val Asp Ala Gly
        115                 120                 125

Glu Arg Thr Lys Gln Ala Leu Ala Phe Glu Arg Thr Asp Phe Asp Gln
    130                 135                 140

Val Arg Ser Leu Met Glu Asn Ser Asp Arg Cys Gln Asp Ile Arg Asn
145                 150                 155                 160

Leu Ala Phe Leu Gly Val Ala Tyr Asn Thr Leu Leu Arg Ile Ala Glu
                165                 170                 175

Ile Ala Arg Ile Arg Val Lys Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Lys Leu Val Glu Arg Trp
    210                 215                 220

Ile Ser Val Ser Gly Val Ala Asp Asp Pro Asn Asn Tyr Leu Phe Cys
225                 230                 235                 240

Arg Val Arg Arg Tyr Gly Val Ala Ala Pro Ser Ala Thr Ser Gln Leu
                245                 250                 255

Ser Thr Tyr Ala Leu Gln Arg Ile Phe Glu Ala Thr His Arg Leu Ile
            260                 265                 270

Tyr Gly Ala Lys Asp Asp Ser Gly Gln Arg Tyr Leu Ala Trp Ser Gly
        275                 280                 285

His Ser Ala Arg Val Gly Ala Ala Arg Asp Met Ala Arg Ala Gly Val
    290                 295                 300

Ser Ile Pro Glu Ile Met Gln Ala Gly Gly Trp Thr Thr Val Asn Ser
305                 310                 315                 320

Val Met Asn Tyr Ile Arg Asn Leu Asp Ser Glu Thr Gly Ala Met Val
                325                 330                 335

Arg Leu Leu Glu Asp Gly Asp
            340

<210>  70
<211>  343
<212>  PRT
<213>  Artificial Sequence

<220>
<223>  Recombinant protein

<400>  70

Met Ser Ile Leu Leu Thr Leu His Gln Ser Leu Ser Ala Leu Leu Val
1               5                   10                  15

Asp Ala Thr Ser Asp Glu Ala Arg Lys Asn Leu Met Asp Val Leu Arg
            20                  25                  30

Asp Arg Gln Ala Phe Ser Glu Arg Thr Trp Lys Val Leu Leu Ser Val
        35                  40                  45

Cys Arg Thr Trp Ala Ala Trp Cys Lys Leu Asn Asn Arg Lys Trp Phe
    50                  55                  60

Pro Ala Glu Pro Glu Asp Val Arg Asp Tyr Leu Leu His Leu Gln Ala
65                  70                  75                  80

Arg Gly Leu Ala Val Asn Thr Ile Leu Gln His Leu Ala Gln Leu Asn
                85                  90                  95

Met Leu His Arg Arg Phe Gly Leu Pro Arg Pro Gly Asp Ser Asp Ala
            100                 105                 110

Val Ser Leu Val Met Arg Arg Ile Arg Arg Glu Asn Val Asp Ala Gly
        115                 120                 125

Glu Arg Thr Lys Gln Ala Leu Ala Phe Glu Arg Thr Asp Phe Asp Gln
    130                 135                 140

Val Arg Ala Leu Met Glu Asn Ser Glu Arg Gly Gln Asp Ile Arg Thr
145                 150                 155                 160

Leu Ala Leu Leu Gly Val Ala Tyr Asn Thr Leu Leu Arg Val Ser Glu
                165                 170                 175

Ile Ala Arg Ile Arg Ile Lys Asp Ile Ser Arg Thr Asp Gly Gly Arg
            180                 185                 190

Met Leu Ile His Ile Ser Arg Thr Lys Thr Leu Val Ser Thr Ala Gly
        195                 200                 205

Val Glu Lys Ala Leu Ser Leu Gly Val Thr Lys Leu Val Glu Arg Trp

210                 215                 220

Ile Ser Val Ser Gly Val Ala Ser Asp Pro Asn Asn Tyr Leu Phe Cys
225                 230                 235                 240

Gln Val Arg Ile Asn Gly Val Ala Val Pro Ser Ala Thr Ser Arg Leu
                245                 250                 255

Ser Thr Asp Val Leu Arg Lys Ile Phe Glu Ala Ala His Arg Leu Ile
            260                 265                 270

Tyr Gly Ala Lys Asp Gly Ser Gly Gln Arg Tyr Leu Ala Trp Ser Gly
        275                 280                 285

His Ser Ala Arg Val Gly Ala Ala Arg Asp Met Ala Arg Ala Gly Val
    290                 295                 300

Ser Ile Ala Glu Ile Met Gln Ala Gly Gly Trp Thr Thr Val Glu Ser
305                 310                 315                 320

Val Met Asn Tyr Ile Arg Asn Leu Asp Ser Glu Thr Gly Ala Met Val
                325                 330                 335

Arg Leu Leu Glu Asp Gly Asp
            340

<210>  71
<211>  715
<212>  PRT
<213>  Artificial Sequence

<220>
<223>  Rcombinant protein

<400>  71

Met Ser Asn Leu Gln Ala Leu His Gln Asn Pro Ser Thr Leu Leu Ala
1               5                   10                  15

Asp Ala Thr Cys Asp Glu Val Arg Lys Asn Leu Met Asp Met Phe Arg
            20                  25                  30

Asp Ser Gln Ala Phe Ser Glu His Thr Trp Lys Ala Leu Gln Ser Val
        35                  40                  45

Cys Arg Ser Trp Val Ala Trp Cys Lys Leu Asn Ser Arg Arg Trp Phe
    50                  55                  60

Pro Ala Glu Pro Glu Asp Val Arg Asp Tyr Leu Leu His Leu Gln Thr
65                  70                  75                  80

Arg Gly Leu Ala Val Lys Thr Ile Gln His His Leu Gly Ser Leu Asn
                85                  90                  95

Met Leu His Arg Gln Ser Gly Leu Pro Arg Pro Gly Asp Ser Asn Ala
            100                 105                 110

Val Ser Leu Val Met Arg Arg Ile Arg Arg Glu Asn Val Asp Ala Gly
        115                 120                 125

Glu Arg Ala Lys Gln Ala Leu Ala Phe Glu Arg Ala Asp Leu Asp Gln
    130                 135                 140

Val Arg Ala Leu Met Glu Asn Ser Asp Arg Cys Gln Asp Ile Arg Asn
145                 150                 155                 160

Leu Ala Phe Leu Gly Val Ala Tyr Asn Thr Leu Leu Arg Ile Gly Glu
                165                 170                 175

Ile Ala Arg Ile Arg Val Arg Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Lys Leu Val Glu Arg Trp
    210                 215                 220

Ile Ser Val Ser Gly Val Ala Asp Asp Pro Asp Asn Tyr Leu Phe Cys
225                 230                 235                 240

Arg Val Arg Arg Tyr Gly Val Ala Lys Pro Ser Ala Thr Ser Gln Leu
                245                 250                 255

Ser Thr Tyr Ala Leu Gln Gly Ile Phe Gly Ala Ala His Arg Leu Val
            260                 265                 270

Tyr Gly Ala Lys Asp Asp Ser Gly Gln Lys Tyr Leu Ala Trp Ser Gly
        275                 280                 285

His Ser Ala Arg Val Gly Ala Ala Arg Asp Met Ala Arg Ala Gly Val
    290                 295                 300

Ser Leu Pro Glu Ile Met Gln Ala Gly Gly Trp Thr Thr Val Asn Ser
305                 310                 315                 320

Val Met Asn Tyr Ile Arg Asn Leu Asp Ser Glu Thr Gly Ala Met Val

                325                 330                 335

Arg Leu Leu Glu Asp Asp Asp Ser Leu Glu Gly Gly Ser Gly Gly Ser
            340                 345                 350

Thr Thr Lys Ala Asn Arg Ala Lys Gly Gly Arg Gly Gly Gly Ser Gly
        355                 360                 365

Gly Ser Met Tyr Met Ser Asp Leu Gln Thr Leu His Gln Asn Leu Ser
    370                 375                 380

Ala Leu Leu Val Asp Ala Thr Ser Asp Glu Ala Arg Lys Asn Leu Met
385                 390                 395                 400

Asp Val Phe Arg Asp Arg Gln Ala Phe Ser Glu Ala Thr Trp Asn Thr
                405                 410                 415

Leu Leu Ser Val Cys Arg Ser Trp Ala Ala Trp Cys Lys Ser Asn Asn
            420                 425                 430

Arg Glu Trp Phe Pro Ala Glu Pro Glu Asp Val Arg Asp Tyr Leu Leu
        435                 440                 445

His Leu Gln Val Arg Gly Leu Ala Val Asn Thr Ile Gln Arg His Leu
    450                 455                 460

Ala Leu Leu Asn Met Leu His Arg Arg Ser Gly Leu Pro Arg Pro Gly
465                 470                 475                 480

Asp Ser Asn Ala Val Ser Leu Val Met Arg Arg Ile Arg Lys Glu Asn
                485                 490                 495

Val Asp Ala Gly Glu Arg Val Arg Gln Ala Leu Ala Phe Glu Arg Thr
            500                 505                 510

Asp Phe Asp Gln Val Arg Ser Leu Met Gly Ser Ser Asp Arg Arg Asn
        515                 520                 525

Asp Ile Arg Asn Leu Ala Phe Leu Gly Val Ala Tyr Asn Thr Leu Leu
    530                 535                 540

Arg Ile Ser Glu Ile Ala Arg Ile Arg Ile Lys Asp Ile Ser Arg Thr
545                 550                 555                 560

Asp Gly Gly Arg Met Leu Val His Ile Gly Arg Thr Lys Thr Leu Val
                565                 570                 575

Ser Thr Ala Gly Val Glu Lys Ala Leu Ser Leu Gly Val Thr Arg Leu
            580                 585                 590

Val Glu Arg Trp Ile Ser Val Ser Gly Val Ala Gly Asp Pro Asn Asn
        595                 600                 605

Tyr Leu Phe Cys Arg Val Arg Lys Asn Gly Val Ala Ala Pro Ser Ala
    610                 615                 620

Ser Ser Gln Leu Thr Thr Asp Val Leu Arg Gly Ile Phe Val Ala Ala
625                 630                 635                 640

His Arg Leu Val His Gly Ala Arg Asp Asp Ser Gly Gln Arg Tyr Leu
                645                 650                 655

Thr Trp Ser Gly His Ser Ala Arg Val Gly Ala Ala Arg Asp Met Ala
            660                 665                 670

Arg Ala Gly Val Ser Ile Ala Glu Ile Met Gln Ala Gly Gly Trp Thr
        675                 680                 685

Thr Val Glu Ser Val Met Ser Tyr Leu Arg Asn Leu Asp Ser Glu Thr
    690                 695                 700

Gly Ala Met Val Arg Leu Leu Glu Asp Gly Asp
705                 710                 715

<210>  72
<211>  715
<212>  PRT
<213>  Artificial Sequence

<220>
<223>  Recombinant protein

<400>  72

Met Ser Asn Ile Gln Thr Pro His Gln Ser Leu Ser Ala Leu Leu Thr
1               5                   10                  15

Asp Ala Thr Ser Asp Val Thr Arg Lys Asn Leu Ala Asp Met Phe Arg
            20                  25                  30

Asp Ser Gln Ala Phe Ser Glu His Thr Trp Lys Met Leu Leu Ser Val
        35                  40                  45

Cys Arg Ser Trp Ala Ala Trp Cys Glu Leu Asn Asn Arg Lys Trp Leu
    50                  55                  60

Pro Val Glu Pro Glu Asp Val Arg Asp Tyr Leu Leu His Leu Gln Thr

65                  70                  75                  80

Arg Gly Leu Ala Val Lys Thr Ile Gln His His Leu Gly Ser Leu Asn
                85                  90                  95

Met Leu His Arg Arg Ala Gly Leu Pro Arg Pro Gly Asp Ser Asn Ala
            100                 105                 110

Val Ser Leu Val Met Arg Arg Ile Arg Arg Glu Asn Val Asp Ala Gly
        115                 120                 125

Glu Arg Ala Gln Gln Ala Leu Ala Phe Glu Arg Thr Asp Phe Asp Gln
    130                 135                 140

Val Arg Ser Leu Val Glu Asn Ser Asp Arg Cys Gln Asp Ile Arg Asn
145                 150                 155                 160

Leu Ala Phe Leu Gly Val Ala Tyr Asn Thr Leu Leu Arg Ile Ser Glu
                165                 170                 175

Ile Ala Arg Ile Arg Val Lys Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Lys Leu Val Glu Arg Trp
    210                 215                 220

Ile Ser Val Ser Gly Val Ala Asp Asp Pro Asn Asn Tyr Leu Phe Cys
225                 230                 235                 240

Arg Val Arg Arg Tyr Gly Val Ala Lys Pro Ser Ala Thr Ser Gln Leu
                245                 250                 255

Ser Thr Tyr Ala Leu Gln Gly Ile Phe Gly Ala Ala His Arg Leu Val
            260                 265                 270

Tyr Gly Ala Lys Gly Asp Ser Gly Gln Lys Tyr Leu Ala Trp Ser Gly
        275                 280                 285

His Ser Ala Arg Val Gly Ala Ala Arg Asp Met Ala Arg Ala Gly Val
    290                 295                 300

Pro Ile Pro Glu Ile Met Gln Ala Gly Gly Trp Thr Thr Val Asn Ser
305                 310                 315                 320

Val Met Asn Tyr Ile Arg Asn Leu Asp Ser Glu Thr Gly Ala Met Val
                325                 330                 335

Arg Leu Leu Glu Asp Ser Asp Ser Leu Glu Gly Gly Ser Gly Gly Ser
            340                 345                 350

Thr Thr Lys Ala Asn Arg Ala Lys Gly Gly Arg Gly Gly Gly Ser Gly
        355                 360                 365

Gly Ser Met Tyr Met Ser Asn Leu Gln Thr Leu His Gln Asn Leu Ser
    370                 375                 380

Ala Leu Leu Ala Asn Ala Thr Ser Asp Glu Ala Arg Lys Asn Leu Met
385                 390                 395                 400

Asp Val Phe Arg Asp Arg Arg Ala Phe Ser Glu Ala Thr Trp Lys Thr
                405                 410                 415

Leu Leu Ser Val Cys Arg Thr Trp Ala Ala Trp Cys Lys Leu Asn Asn
            420                 425                 430

Arg Lys Trp Phe Pro Ala Glu Pro Glu Asp Val Arg Asp Tyr Leu Leu
        435                 440                 445

His Leu Gln Val Arg Gly Leu Ala Val Asn Thr Ile Gln Arg His Leu
    450                 455                 460

Ala Leu Leu Asn Met Leu His Arg Arg Ser Gly Leu Pro Arg Pro Gly
465                 470                 475                 480

Asp Ser Ser Ala Val Ser Leu Val Met Arg Arg Ile Arg Lys Glu Asn
                485                 490                 495

Val Asp Ala Gly Glu Arg Val Arg Gln Ala Leu Ala Phe Glu Arg Thr
            500                 505                 510

Asp Phe Asp Lys Val Arg Ser Leu Met Gly Asn Ser Asp Arg Cys Gln
        515                 520                 525

Asp Ile Arg Asn Leu Ala Phe Leu Gly Val Ala Tyr Asn Thr Leu Leu
    530                 535                 540

Arg Ile Ser Glu Ile Ala Arg Ile Arg Ile Lys Asp Ile Ser Arg Thr
545                 550                 555                 560

Asp Gly Gly Arg Met Leu Ile His Ile Gly Arg Thr Lys Thr Leu Val
                565                 570                 575

Ser Thr Ala Gly Val Glu Lys Ala Leu Ser Leu Gly Val Thr Arg Leu
            580                 585                 590

Val Gly Arg Trp Ile Ser Val Ser Gly Val Ala Gly Asp Pro Asn Asn
        595                 600                 605

Tyr Leu Phe Cys Arg Val Arg Lys Asn Gly Val Ala Ala Pro Ser Ala
    610                 615                 620

Thr Ser Gln Leu Ser Thr Asp Val Leu Arg Gly Val Phe Ala Ala Ala
625                 630                 635                 640

His Arg Leu Val Tyr Gly Thr Lys Asp Asp Ser Gly Gln Gly Tyr Leu
                645                 650                 655

Thr Trp Ser Gly His Ser Ala Arg Val Gly Ala Ala Arg Asp Met Ala
            660                 665                 670

Arg Ala Gly Val Ser Ile Ala Glu Ile Met Gln Ala Gly Gly Trp Thr
        675                 680                 685

Thr Val Glu Ser Val Met Ser Tyr Leu Arg Asn Leu Asp Ser Glu Thr
    690                 695                 700

Gly Ala Met Val Arg Leu Leu Glu Asp Gly Asp
705                 710                 715

<210>  73
<211>  34
<212>  DNA
<213>  Homo sapiens

<400>  73
ataacttcgt ataatgtatg ctatacgaag ttat                                34

<210>  74
<211>  32
<212>  DNA
<213>  Homo sapiens

<400>  74
taactttaaa taatgccaat tatttaaagt ta                                  32

<210>  75
<211>  34
<212>  DNA
<213>  Homo sapiens

<400>  75
tcaatttctg agaatgacag ttctcggaaa ttga                                34

<210>  76
<211>  34
<212>  DNA
<213>  Homo sapiens

<400>  76
ctcgtgtccg ataactgtaa ttatcggaca tgat                                    34


<210>  77
<211>  34
<212>  DNA
<213>  Homo sapiens

<400>  77
aataggtctg agaatgggcg ttctcagacg tatt                                    34


<210>  78
<211>  21
<212>  DNA
<213>  Homo sapiens

<400>  78
cagctttttt atactaagtt g                                                  21


<210>  79
<211>  34
<212>  DNA
<213>  Homo sapiens

<400>  79
gaagttccta tactttctag agaataggaa cttc                                    34


<210>  80
<211>  31
<212>  DNA
<213>  Homo sapiens

<400>  80
ttgatgaaag aataacgtat tctttcatca a                                       31


<210>  81
<211>  43
<212>  DNA
<213>  Homo sapiens

<400>  81
acgaaaaatg gtaaggaata gaccattcct taccattttt cgt                          43


<210>  82
<211>  61
<212>  DNA
<213>  Homo sapiens

<400>  82
aaacgatatc agacatttgt ctgataatgc ttcattatca gacaaatgtc tgatatcgtt        60

t                                                                   61

<210>  83
<211>  39
<212>  DNA
<213>  Homo sapiens

<400>  83
gagtttcatt aaggaataac taattcccta atgaaactc                          39

<210>  84
<211>  33
<212>  DNA
<213>  Homo sapiens

<400>  84
ggttgcttaa gaataagtaa ttcttaagca acc                                33

<210>  85
<211>  34
<212>  DNA
<213>  Homo sapiens

<400>  85
tgaatgtcct ataattacac ttataggaca ttca                               34

<210>  86
<211>  34
<212>  DNA
<213>  Homo sapiens

<400>  86
gaaactttaa ataataagtc ttatttaaag tttc                               34

<210>  87
<211>  34
<212>  DNA
<213>  Homo sapiens

<400>  87
ataaatatgt gtgtatatat acacacaaac atat                               34

<210>  88
<211>  34
<212>  DNA
<213>  Homo sapiens

<400>  88
atatatctgt gtatatatat acacacacac atat                               34

<210>  89
<211>  34
<212>  DNA
<213>  Homo sapiens

<400>  89

atatatatgt gtatatatat acacacatac atat                                34

<210>  90
<211>  34
<212>  DNA
<213>  Homo sapiens

<400>  90
atatatgtgt gtatatatat acacacacac atac                                34

<210>  91
<211>  34
<212>  DNA
<213>  Homo sapiens

<400>  91
ataaatatgt gtaaactaaa caacacactc ttaa                                34

<210>  92
<211>  34
<212>  DNA
<213>  Homo sapiens

<400>  92
acaaatatgt ggaaactaaa caacacattc ttga                                34